# EUROPEAN PATENT APPLICATION

(11) **EP 4 059 938 A1**
(43) Date of publication of application: **21.09.2022**
(21) Application number: 20888307.4
(22) Date of filing: 11.11.2020
(51) Int. Cl.: C07F 9/6561, C07F 9/6558, A61K 31/4745, A61K 39/39, A61P 31/14, A61P 35/00, A61P 31/20, A61P 17/12

(54) **IMIDAZOQUINOLINE SUBSTITUTED PHOSPHORIC ESTER AGONIST, AND PREPARATION THEREFOR AND APPLICATION THEREOF**

(30) Priority: 11.11.2019 CN 201911097066
(71) Applicant: Suzhou Zelgen Biopharmaceutical Co., Ltd., Jiangsu 215300 (CN); Shanghai Zelgen Pharma.Tech Co., Ltd., Shanghai 201203 (CN)
(72) Inventor: LV, Binhua, Suzhou, Jiangsu 215300 (CN); CUI, Dawei, Suzhou, Jiangsu 215300 (CN); CHAI, Chuanke, Suzhou, Jiangsu 215300 (CN); LIU, Ruifeng, Suzhou, Jiangsu 215300 (CN); SHENG, Zelin, Suzhou, Jiangsu 215300 (CN)
(74) Representative: dompatent von Kreisler Selting Werner - Partnerschaft von Patent- und Rechtsanwälten mbB
(86) International application number: PCT/CN2020/128168
(87) International publication number: WO 2021/093779

(57) **Abstract**

The present invention relates to an imidazoquinoline substituted phosphoric ester agonist, and a preparation therefor and an application thereof. Specifically, the compounds of the present invention have the structure shown in formula (I), wherein the definition of each group and substituent is as described in the description. Also disclosed in the present invention are a preparation method for the compound and use thereof as a TLR agonist.

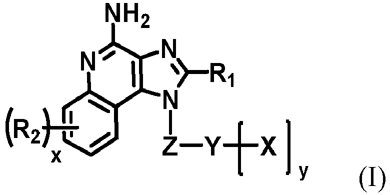

## Description

### TECHNICAL FIELD

The invention belongs to the field of pharmaceuticals, and specifically relates to an imidazoquinoline substituted phosphoric ester agonist, and a preparation therefor and an application thereof.

### BACKGROUND ART

Toll-like receptors (TLRs) are important protein molecules involved in non-specific immunity (innate immunity). When microorganisms break through the body's physical barriers (such as skin or mucous membranes), TLRs can be stimulated to generate immune cell responses by identifying molecules with conservative structures derived from microorganisms. So far, the TLR that has been determined contains at least 13 members, wherein TLR1/TLR2 heterodimers recognize triacetyl ester peptides; TLR2/TLR6 heterodimers recognize triacetyl ester peptides; TLR2 and TLR4 can recognize the surface structure of bacteria (such as lipoproteins, lipoteichoic acid, peptidoglycans and lipopolysaccharides, etc.); TLR3 recognizes double-stranded RNA; TLR5 recognizes flagellin in bacterial flagella; TLR7 and TLR8 recognize single-stranded RNA; TLR9 recognizes CpG-ODN from bacteria and viruses; TLR11 recognizes inhibitory protein-like molecules from Toxoplasma gondii (Huck, BR. et al., Angew. Chem. Int. Ed. 2018, 57, 4412-4428; Isogawa, M. et al., J Virology, 2005, 79 (11), 7269-7272). TLR1, 2, 4, 5 and 6 are mainly expressed on the cell surface, while TLR3, 7, 8 and 9 are mainly expressed in the inner body.

TLR7 is mainly expressed by plasmacytoid dendritic cells (pDC) and recognized by ligand thereby inducing interferon α□(INF-α) secretion. TLR8 is mainly expressed by bone marrow immune cells and recognized by ligand thereby stimulating and inducing cytokine production, such as tumor necrosis factor α (TNFα), interleukin 18(IL18), interleukin 12(IL12) and interferon γ (INF-γ). In addition to stimulating the secretion of pro-inflammatory cytokines and chemokines, TLR8 agonists can also promote the expression of co-stimulatory molecules such as CD8⁺cells, major histocompatibility complex molecules and chemokine receptors (McGowan, D. et al., J. Med. Chem. 2016, 59, 7936-7949).

Existing studies have shown that activating innate and adaptive immune responses can induce immune responses, thereby providing a treatment for diseases such as autoimmune, inflammation, allergies, asthma, transplant rejection, graft versus host disease (GvHD), infection, cancer, and immunodeficiency. For example, for hepatitis B (HBV), activating TLR8 on professional antigen presenting cells (pAPCs) and other immune cells in the liver will lead to the secretion of pro-inflammatory cytokines, thus increasing HBV-specific T cell response, activating NK cells in the liver and promoting the reconstruction of antiviral immune system in the body (Wille-Reece, U. et al., J Exp Med 2006, 203, 1249-1258; Peng, G. et al., science 2005, 309, 1380-1384; Jo, J. et al., PLoS Pathogens 2014, 10, e1004210; Watashi, K. et al., J Bioi Chern 2013, 288, 31715-31727).

Because Toll-like receptors are pathologically associated with a variety of diseases, new Toll-like receptor modulators such as TLR8 agonists are currently needed for clinical treatment. TLR8 agonists with high selectivity and high activity have the potential to reduce off-target effects, thus having more urgent clinical needs.

### SUMMARY OF THE INVENTION

The object of the present invention is to provide novel compounds with selective agonistic effects and/or better pharmacodynamic properties on Toll-like receptors 7 and/or 8 (TLR7 and/or TLR8) and use thereof.

In the first aspect of the present invention, it provides an
imidazoquinoline-substituted phosphoric ester compound having a structure of general formula (I), or a stereoisomer, a tautomer, a crystal form, or a pharmaceutically acceptable salt, a hydrate, a solvate or a prodrug thereof: 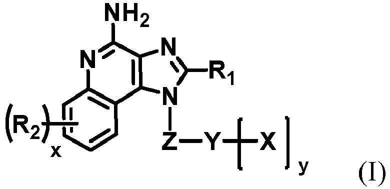 wherein:
R₁ is selected from the substituted or unsubstituted group consisting of hydrogen, C1-C18 alkyl, deuterated C1-C18 alkyl, halogenated C1-C18 alkyl, C1-C18 alkoxy, deuterated C1-C18 alkoxy, halogenated C1-C18 alkoxy, halogen, amino, nitro, hydroxyl, cyano, C3-C18 cycloalkyl, heterocyclyl, C6-C10 aryl, heteroaryl, -(CH₂)ₙOR₅, -(CH₂)ₙO(CH₂)ₘR₅, -(CH₂)ₙSR₅, -(CH₂)ₙCOR₅, -(CH₂)ₙC(O)OR₅, -(CH₂)ₙS(O)ₘR₅, -(CH₂)ₙNR₆R₇, -(CH₂)ₙC(O)NR₆R₇, -(CH₂)ₙNR₆C(O)R₅, and -(CH₂)ₙNR₆S(O)ₘR₅; wherein the substituted means to be substituted by one or more groups selected from the group consisting of hydrogen, deuterium, C1-C18 alkyl, deuterated C1-C18 alkyl, halogenated C1-C18 alkyl, C1-C18 alkoxy, deuterated C1-C18 alkoxy, halogenated C1-C18 alkoxy, halogen, amino, nitro, hydroxyl, cyano, C3-C18 cycloalkyl, heterocyclyl, C6-C10 aryl, heteroaryl, -(CH₂)ₙOR₅, -(CH₂)ₙSR₅, -(CH₂)ₙCOR₅, -(CH₂)ₙC(O)OR₅,-(CH₂)ₙS(O)ₘR₅, -(CH₂)ₙNR₆R₇, -(CH₂)ₙC(O)NR₆R₇, -(CH₂)ₙC(O)NHR₆, -(CH₂)ₙNR₆C(O)R₅, and -(CH₂)ₙNR₆S(O)ₘR₅;
R₂ is each the same or different, and is independently selected from the group consisting of hydrogen, deuterium, C1-C18 alkyl, deuterated C1-C18 alkyl, halogenated C1-C18 alkyl, C1-C18 alkoxy, deuterated C1-C18 alkoxy, halogenated C1-C18 alkoxy, halogen, amino, nitro, hydroxyl, cyano, C3-C18 cycloalkyl, heterocyclyl, C6-C10 aryl, heteroaryl, -(CH₂)ₙOR₅, -(CH₂)ₙSR₅, -(CH₂)ₙCOR₅, -(CH₂)ₙC(O)OR₅, -(CH₂)ₙS(O)ₘR₅, -(CH₂)ₙNR₆R₇, -(CH₂)ₙC(O)NR₆R₇, -(CH₂)ₙC(O)NHR₆, -(CH₂)ₙNR₆C(O)R₅, and -(CH₂)ₙNR₆S(O)ₘR₅;
Z is selected from the substituted or unsubstituted group consisting of C1-C18 alkylene, deuterated C1-C18 alkylene, halogenated C1-C18 alkylene, C1-C18 alkyleneoxy, halogenated C1-C18 alkyleneoxy, C3-C18 cycloalkylene, C1-C18 alkylene C3-C18 cycloalkylene, C3-C18 cycloalkylene C1-C18 alkylene, C1-C18 alkylene C3-C18 cycloalkylene C1-C18 alkylene, heterocyclylene, C6-C10 arylene, and heteroarylene, wherein the substituted means to be substituted by one or more groups selected from the group consisting of hydrogen, deuterium, C1-C18 alkyl, deuterated C1-C18 alkyl, halogenated C1-C18 alkyl, C1-C18 alkoxy, deuterated C1-C18 alkoxy, halogenated C1-C18 alkoxy, halogen, amino, nitro, hydroxyl, cyano, C3-C18 cycloalkyl, heterocyclyl, C6-C10 aryl, heteroaryl, -(CH₂)ₙOR₅, -(CH₂)ₙSR₅, -(CH₂)ₙCOR₅, -(CH₂)ₙC(O)OR₅,-(CH₂)ₙS(O)ₘR₅, -(CH₂)ₙNR₆R₇, -(CH₂)ₙC(O)NR₆R₇, -(CH₂)ₙC(O)NHR₆, -(CH₂)ₙNR₆C(O)R₅, and -(CH₂)ₙNR₆S(O)ₘR₅;
Y is selected from O, N or NR₄;
R₄ is selected from the substituted or unsubstituted group consisting of hydrogen, C1-C18 alkyl, deuterated C1-C18 alkyl, halogenated C1-C18 alkyl, C1-C18 alkoxy, deuterated C1-C18 alkoxy, halogenated C1-C18 alkoxy, C3-C18 cycloalkyl, heterocyclyl, C6-C10 aryl, and heteroaryl, wherein the substituted means to be substituted by one or more groups selected from the group consisting of hydrogen, deuterium, C1-C18 alkyl, deuterated C1-C18 alkyl, halogenated C1-C18 alkyl, C1-C18 alkoxy, deuterated C1-C18 alkoxy, halogenated C1-C18 alkoxy, halogen, amino, nitro, hydroxyl, cyano, C3-C18 cycloalkyl, heterocyclyl, C6-C10 aryl, heteroaryl, -(CH₂)ₙOR₅, -(CH₂)ₙSR₅, -(CH₂)ₙCOR₅, -(CH₂)ₙC(O)OR₅, -(CH₂)ₙS(O)ₘR₅, -(CH₂)ₙNR₆R₇, -(CH₂)ₙC(O)NR₆R₇, -(CH₂)ₙC(O)NHR₆, -(CH₂)ₙNR₆C(O)R₅, and -(CH₂)ₙNR₆S(O)ₘR₅;
R₅ is selected from the substituted or unsubstituted group consisting of hydrogen, deuterium, C1-C18 alkyl, deuterated C1-C18 alkyl, halogenated C1-C18 alkyl, C1-C18 alkoxy, deuterated C1-C18 alkoxy, halogenated C1-C18 alkoxy, amino, hydroxyl, cyano, C3-C18 cycloalkyl, heterocyclyl, C6-C10 aryl, and heteroaryl, wherein the substituted means to be substituted by one or more groups selected from the group consisting of hydrogen, deuterium, C1-C18 alkyl, deuterated C1-C18 alkyl, halogenated C1-C18 alkyl, C1-C18 alkoxy, deuterated C1-C18 alkoxy, halogenated C1-C18 alkoxy, halogen, amino, nitro, hydroxyl, cyano, C3-C18 cycloalkyl, heterocyclyl, C6-C10 aryl, heteroaryl, -(CH₂)ₙOR₈, -(CH₂)ₙSR₈, -(CH₂)ₙCOR₈, -(CH₂)ₙC(O)OR₈, -(CH₂)ₙS(O)ₘR₈, -(CH₂)ₙNR₈R₉, -(CH₂)ₙC(O)NR₈R₉, -(CH₂)ₙC(O)NHR₉, -(CH₂)ₙNR₉C(O)R₈, and -(CH₂)ₙNR₉S(O)ₘR₈;
R₆ and R₇ are the same or different, and are each independently selected from the substituted or unsubstituted group consisting of hydrogen, deuterium, C1-C18 alkyl, deuterated C1-C18 alkyl, halogenated C1-C18 alkyl, C1-C18 alkoxy, deuterated C1-C18 alkoxy, halogenated C1-C18 alkoxy, amino, hydroxyl, C3-C18 cycloalkyl, heterocyclyl, C6-C10 aryl, and heteroaryl; wherein the substituted means to be substituted by one or more groups selected from the group consisting of hydrogen, deuterium, C1-C18 alkyl, deuterated C1-C18 alkyl, halogenated C1-C18 alkyl, C1-C18 alkoxy, deuterated C1-C18 alkoxy, halogenated C1-C18 alkoxy, halogen, amino, nitro, hydroxyl, cyano, C3-C18 cycloalkyl, heterocyclyl, C6-C10 aryl, heteroaryl, -(CH₂)ₙOR₈, -(CH₂)ₙSR₈, -(CH₂)COR₈, -(CH₂)ₙC(O)OR₈, -(CH₂)ₙS(O)ₘR₈, -(CH₂)ₙNR₈R₉, -(CH₂)ₙC(O)NR₈R₉, -(CH₂)ₙC(O)NHR₉, -(CH₂)ₙNR₉C(O)R₈, and -(CH₂)ₙNR₉S(O)ₘR₈;
R₈ and R₉ are the same or different, and are each independently selected from the substituted or unsubstituted group consisting of hydrogen, deuterium, C1-C18 alkyl, deuterated C1-C18 alkyl, halogenated C1-C18 alkyl, C1-C18 alkoxy, deuterated C1-C18 alkoxy, halogenated C1-C18 alkoxy, amino, hydroxyl, -COOR₁₆, C3-C18 cycloalkyl, heterocyclyl, C6-C10 aryl, and heteroaryl, wherein the substituted means to be substituted by one or more groups selected from the group consisting of deuterium, C1-C20 alkyl, C1-C6 alkoxy, C3-C10 cycloalkyl, C4-C10 heterocyclyl, C6-C10 aryl, C5-C10 heteroaryl, halogen, amino, nitro, -COOR₁₆, cyano, hydroxyl, amido, and sulfonamido;
X is selected from the group consisting of -P(=O)(OH)₂, -P(=O)(OH)OP(=O)(OH)₂, -P(=O)(OH)OP(=O)(OH)OP(=O)(OH)₂, -P(=O)(X₁R₁₁)(X₂R₁₂), -P(=O)(X₁R₁₁)(X₃R₁₄R₁₅), -P(=O)(X₃R₁₄R₁₈)(X₃R₁₄R₁₅), -CH₂P(=O)(X₁R₁₁)(X₂R₁₂), -CH₂P(=O)(X₁R₁₁)(X₃R₁₄R₁₅), -CH₂P(=O)(X₃R₁₄R₁₅)(X₃R₁₄R₁₅), -P(=S)(X₁R₁₁)(X₂R₁₂), -P(=S)(X₁R₁₁)(X₃R₁₄R₁₅), -P(=S)(X₃R₁₄R₁₅)(X₃R₁₄R₁₅), -CH₂P(=S)(X₁R₁₁)(X₂R₁₂), -CH₂P(=S)(X₁R₁₁)(X₃R₁₄R₁₅), -CH₂P(=S)(X₃R₁₄R₁₅)(X₃R₁₄R₁₅), -P(=NR₁₃)(X₁R₁₁)(X₂R₁₂), -P(=NR₁₃)(X₁R₁₁)(X₃R₁₄R₁₅), -P(=NR₁₃)(X₃R₁₄R₁₅)(X₃R₁₄R₁₅), -CH₂P(=NR₁₃)(X₁R₁₁)(X₂R₁₂), -CH₂P(=NR₁₃)(X₁R₁₁)(X₃R₁₄R₁₅) , and -CH₂P(=NR₁₃)(X₃R₁₄R₁₅)(X₃R₁₄R₁₅);
X₁ and X₂ are independently selected from the group consisting of oxygen, sulfur, and -OCH₂O-;
X3 is nitrogen;
R₁₁, R₁₂, R₁₃, R₁₄ and R₁₅ are independently selected from the substituted or unsubstituted group consisting of hydrogen, C1-C20 alkyl, deuterated C1-C20 alkyl, C3-C10 cycloalkyl, C4-C10 heterocyclyl, C6-C10 aryl, and C5-C10 heteroaryl, or R₁₁ and R₁₂ combine with adjacent X₁, X₂ and P to form substituted 5-7-membered heterocyclyl, and the substituted means to be substituted by one or more substituents selected from the group consisting of deuterium, C1-C20 alkyl, halogenated C1-C20 alkyl, C1-C6 alkoxy, C3-C10 cycloalkyl, C4-C10 heterocyclyl, C6-C10 aryl, halogenated C6-C10 aryl, C5-C10 heteroaryl, halogen, amino, nitro, -COR₁₆, -COOR₁₆, -OCOOR₁₆, cyano, hydroxyl, amido, and sulfonamido;
R₁₆ is selected from the substituted or unsubstituted group consisting of hydrogen, C1-C18 alkyl, deuterated C1-C20 alkyl, C3-C10 cycloalkyl, C3-C10 cycloalkenyl, C6-C10 aryl, amino, and heterocyclyl, wherein the substituted means to be substituted by one or more C6-C10 aryl;
x is an integer of 0, 1, 2, 3 or 4;
y is an integer of 1 or 2;
m is an integer of 0, 1 or 2;
and n is an integer of 0, 1, 2, 3, 4 or 5;
and each heterocyclyl is independently 5-15-membered heterocycloalkyl containing 1-3 heteroatoms selected from N, O or S;
each heteroaryl is independently 5-15-membered heteroaryl containing 1-3 heteroatoms selected from N, O or S.

In another preferred embodiment, it is a compound of general formula (II-1) or (II-2), or a stereoisomer, a tautomer, a crystal form, or a pharmaceutically acceptable salt, a hydrate, a solvate or a prodrug thereof: 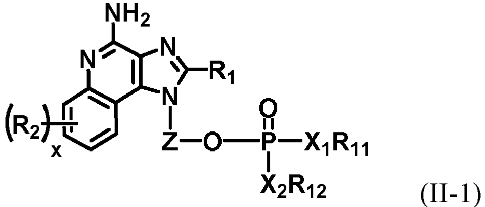 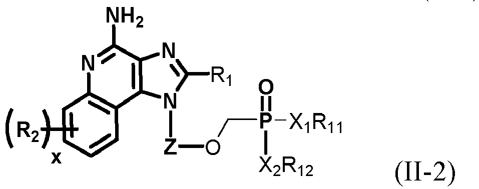 wherein:
R₁, R₂, x, Z, X₁, X₂, R₁₁ and R₁₂ are as described in general formula (I).

In another preferred embodiment, the compound is a compound represented by general formula (IV-1) or (IV-2): 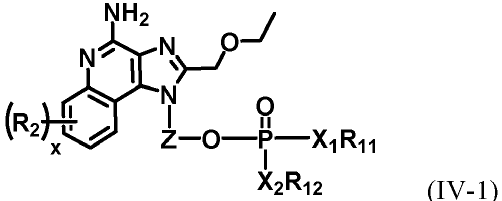 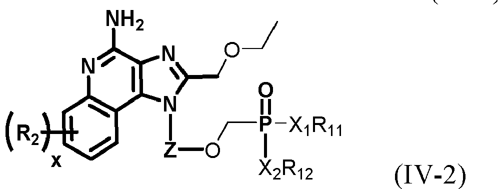 wherein:
R₂, x, Z, X₁, X₂, R₁₁ and R₁₂ are as described in general formula (I).

In another preferred embodiment, it is a compound of general formula (III-1) or (III-2), or a stereoisomer, a tautomer, a crystal form, or a pharmaceutically acceptable salt, a hydrate, a solvate or a prodrug thereof: 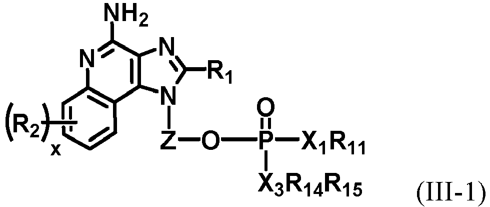 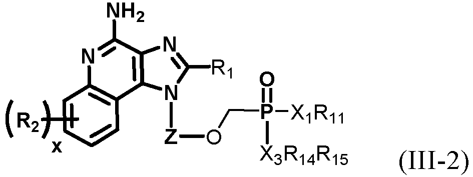 R₁, R₂, x, Z, X₁, X₃, R₁₁, R₁₄ and R₁₅ are as described in general formula (I).

In another preferred embodiment, the compound is a compound represented by general formula (IV-3) or (IV-4): 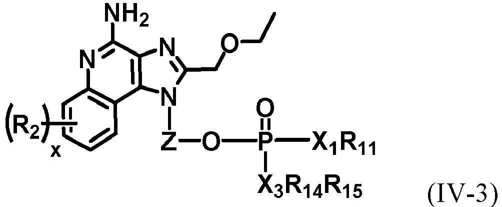 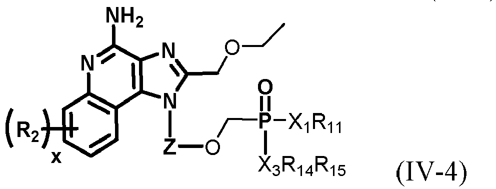 R₂, x, Z, X₁, X₃, R₁₁, R₁₄ and R₁₅ are as described in general formula (I).

In another preferred embodiment, Z has a configuration of S or R.

In another preferred embodiment, Z has a configuration of S.

In another preferred embodiment, structure P has a configuration of S or R.

In another preferred embodiment, structure P has a configuration of S.

In another preferred embodiment, at least one of R₁₄ and R₁₅ has a configuration of S.

In the second aspect of the present invention, it provides a method for preparing the imidazoquinoline-substituted phosphoric ester compound having a structure of general formula (I), or a stereoisomer, a tautomer, a crystal form, or a pharmaceutically acceptable salt, a hydrate, a solvate or a prodrug thereof according to the first aspect of the present invention comprising the steps:
1) protecting one of the H in -NH₂ of 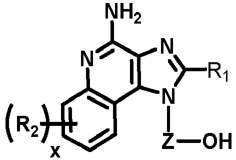 to obtain 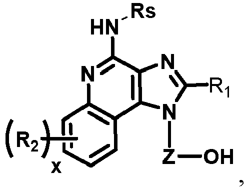 wherein Rs is a protecting group;
2) reacting 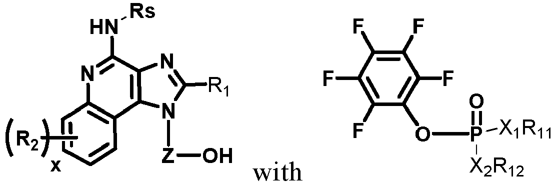 or 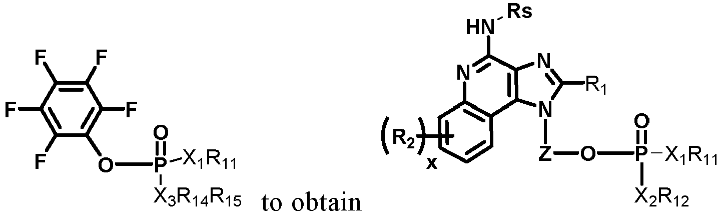 or 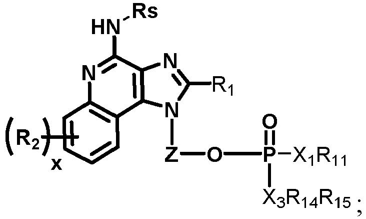
3) deprotecting 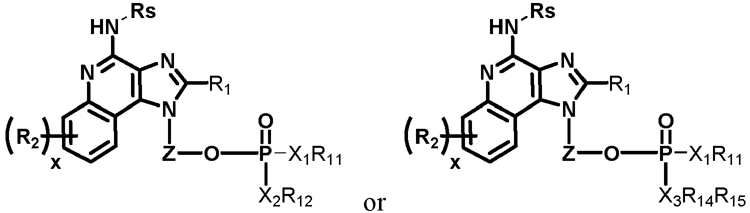 to obtain 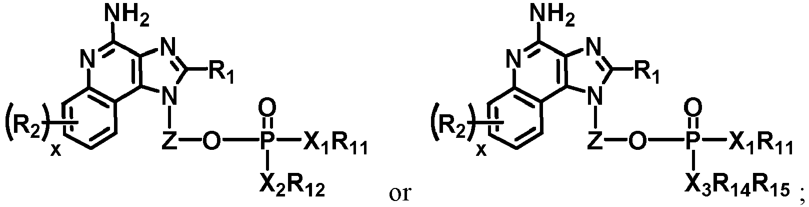 wherein, X is selected from the group consisting of -P(=O)(X₁R₁₁)(X₂R₁₂) and -P(=O)(X₁R₁₁)(X₃R₁₄R₁₅);
   y is 1;
   R₁, R₂, x, Z, X₁, X₂, X₃, R₁₁, R₁₂, R₁₄ and R₁₅ are as described in the first aspect of the present invention.

In another preferred embodiment, Rs is triphenylmethyl or 4,4'-bis-methoxytrityl.

In the third aspect of the present invention, it provides a pharmaceutical composition comprising a pharmaceutically acceptable carrier and one or more imidazoquinoline-substituted phosphoric ester compounds having a structure of general formula (I), or a stereoisomer, a tautomer, a crystal form, or a pharmaceutically acceptable salt, a hydrate, a solvate or a prodrug thereof according to the first aspect of the present invention.

In another preferred embodiment, it further comprises other drugs or combinations for preventing and/or treating diseases selected from the group consisting of inflammation, cancer, cardiovascular disease, infection, immune disease, and metabolic disease.

In another preferred embodiment, the infection is a viral infection.

In another preferred embodiment, the infection is cancer.

In another preferred embodiment, the pharmaceutical composition also comprises a drug selected from the group consisting of
PD-1 inhibitors (nivolumab, pembrolizumab, pidilizumab, cemiplimab, JS-001, SHR-120, BGB-A317, IBI-308, GLS-010, GB-226, STW204, HX008, HLX10, BAT 1306, AK105, LZM 009 or biosimilars of the above drugs), PD-L1 inhibitors (durvalumab, atezolizumab, avelumab, CS1001, KN035, HLX20, SHR-1316, BGB-A333, JS003, CS1003, KL-A167, F 520, GR1405, MSB2311 or biosimilars of the above drugs), CD20 antibodies (e. g., rituximab, obinutuzumab, ofatumumab, veltuzumab, tositumomab, ¹³¹I- tositumomab, ibritumomab, ⁹⁰Y- ibritumomab, ⁹⁰In-ibritumomab, ibritumomab tiuxetan), CD47 antibodies (Hu5F9-G4, CC-90002, TTI-621, TTI-622, OSE-172, SRF-231, ALX-148, NI-1701, SHR-1603, IBI188, IMM01), or combinations thereof.

In another preferred embodiment, the pharmaceutical composition also comprises a drug selected from the group consisting of
interferon α (standard INFα and polyethanolated INFα), nucleoside drugs (such as Telbivudine, Lamivudine, Clevudine, Adefovir, Tenofovir, Besifovir, Tenofovir Disoproxil Fumarate (TDF), Tenofovir Alafenamide Fumarate (TAF) and HDP-PMPA (CMX157), etc.), shell protein allosteric regulators (such as BAY41-4109, RG-7907, NVR 3-778, ABI-H0731, ABI-H2158, JNJ-56136379, GLS 4JHS, etc.), cccDNA inhibitors, TLR3/7/8/9 agonists (such as RG-7854, GS9620, etc.), hepatitis B virus entry inhibitors (such as Myrcludex B, etc.), interfering nucleotides (such as ARB 1467, ARB 1740, etc.), HBV surface antigen inhibitors (such as RG7834, REP2139, REP2165,etc.), CRISPER/Cas9, or combinations thereof.

In the fourth aspect of the present invention, it provides a use of the imidazoquinoline-substituted phosphoric ester compound having a structure of general formula (I), or a stereoisomer, a tautomer, a crystal form, or a pharmaceutically acceptable salt, a hydrate, a solvate or a prodrug thereof according to the first aspect of the present invention for a use selected from the group consisting of
1) for the preparation of TLR7 agonists;
2) for the preparation of TLR8 agonists;
3) for the preparation of TLR7 and TLR8 dual agonists;
4) for the preparation of drugs for the prevention and/or treatment of viral infectious diseases;
5) for the preparation of drugs for the prevention and/or treatment of cancer.

In another preferred embodiment, the use is selected from the group consisting of
1) for the preparation of TLR7 agonists;
2) for the preparation of TLR8 agonists.

In another preferred embodiment, the use is selected from the group consisting of
3) for the preparation of drugs for the prevention and/or treatment of viral infectious diseases;
4) for the preparation of drugs for the prevention and/or treatment of cancer.

In another preferred embodiment, the viral infectious disease is selected from the group consisting of dengue virus, yellow fever virus, West Nile virus, Japanese encephalitis virus, tick-borne encephalitis virus, Kunjin virus, Murray Valley encephalitis virus, St. Louis encephalitis virus, Omsk hemorrhagic fever virus, bovine viral diarrhea virus, Zika virus, viral hepatitis, viral skin disease.

In another preferred embodiment, the virus infection hepatitis is selected from the group consisting of hepatitis B, and hepatitis C.

In another preferred embodiment, the viral skin disease is selected from the group consisting of condyloma acuminatum, molluscum contagiosum, genital herpes, and nevus flammeus.

In another preferred embodiment, the cancer is selected from the group consisting of lung cancer, breast cancer, prostate cancer, esophageal cancer, colorectal cancer, bone cancer, kidney cancer, gastric cancer, liver cancer, large intestine cancer, melanoma, lymphoma, blood cancer, brain tumor, myeloma, soft tissue sarcoma, pancreatic cancer, and skin cancer.

It should be understood that in the present invention, any of the technical features specifically described above and below (such as in the Examples) can be combined with each other, so as to constitute new or preferred technical solutions which will not redundantly be described one by one herein.

### DETAILED DESCRIPTION OF THE INVENTION

After long and in-depth research, the present inventors have unexpectedly prepared a new class of compounds with selective agonistic effects and/or better pharmacodynamic properties on Toll-like receptors 7 and/or 8 (TLR7 and/or TLR8). On this basis, the inventors have completed the present invention.

### TERMS

In the present invention, unless specifically indicated, the terms used have the general meaning well known to those skilled in the art.

The term "alkyl" refers to a linear, branched, or cyclic alkane group containing 1-20 carbon atoms such as 1-18 carbon atoms especially 1-8 carbon atoms. Typical "alkyl" includes methyl, ethyl, propyl, isopropyl, n-butyl, tert-butyl, isobutyl, 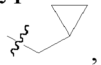 pentyl, isoamyl, heptyl,4,4-dimethylpentyl, octyl, 2,2,4-trimethylpentyl, nonyl, decyl, undecyl, dodecyl, etc.

The term "C1-C8 alkyl" refers to a linear or branched or cyclic alkyl group that includes 1 to 8 carbon atoms, such as methyl, ethyl, propyl, isopropyl 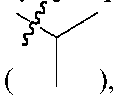 n-butyl, tert-butyl, isobutyl (such as 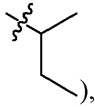 n-pentyl, isoamyl, n-hexyl, iso-hexyl, n-heptyl, isoheptyl. "Substituted alkyl" means that one or more positions in the alkyl are substituted, especially 1 to 4 substituents, which can be substituted at any position. Typical substituents include, but are not limited to, one or more of the following groups: such as hydrogen, deuterium, halogen (e. g., monohalogen substituents or polyhalogen substituents, the latter such as trifluoromethyl or an alkyl containing Cl₃), cyano, nitro, oxo (e. g. =O), trifluoromethyl, trifluoromethoxy, cycloalkyl, alkenyl, cycloalkenyl, alkynyl, heterocyclyl, aromatic ring, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein Rₐ appearing here may independently represent hydrogen, deuterium, alkyl, cycloalkyl, alkenyl, cycloalkenyl, alkynyl, heterocyclyl or aromatic ring, and R_{b}, R_{c} and R_{d} may independently represent hydrogen, deuterium, alkyl, cycloalkyl, heterocyclyl or aromatic ring, or R_{b} and R_{c} together with N atom can form a heterocyclyl; Rₑ can independently represent hydrogen, alkyl, cycloalkyl, alkenyl, cycloalkenyl, alkynyl, heterocyclyl or aromatic ring. The above typical substituents, such as alkyl, cycloalkyl, alkenyl, cycloalkenyl, alkynyl, heterocyclyl or aromatic ring, may be optionally substituted.

The term "alkylene" refers to a group formed by "alkyl" removing a hydrogen atom, such as methylene, ethylene, propylene, isopropylene (such as 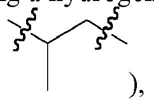 butylene (such as 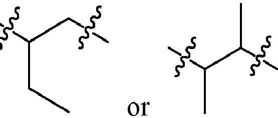 ), pentylene (such as 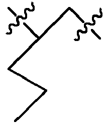 ), hexylene (such as 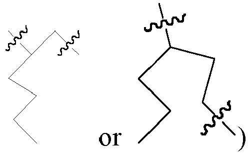 , heptylene (such as 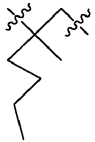 ), etc.

The term "C1-C18 alkylene C3-C18 cycloalkylene" or "C3-C18 cycloalkylene C1-C18 alkylene" has the same meaning and refers to a group formed by cycloalkylalkyl or alkylcycloalkyl removing two hydrogen atoms such as 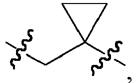 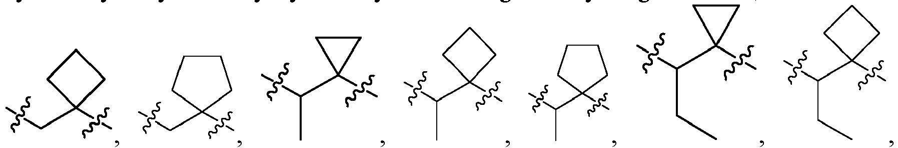 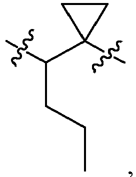 etc.

The term "alkenyl" refers to a linear or branched hydrocarbon group containing 2 to 18 carbon atoms and at least one carbon-carbon double bond. Typical groups include vinyl or allyl. The term"(C₂-C₆) alkenyl" refers to a linear or branched group containing 2-6 carbon atoms and at least one carbon-carbon double bond, such as vinyl, propenyl,2-propenyl, (E)-2-butenyl, (Z)-2-butenyl, (E)-2-methyl-2-butenyl, (Z)-2-methyl-2-butenyl, 2,3-dimethyl-2-butenyl, (Z)-2-pentenyl, (E)-1-pentenyl, (Z)-1-hexenyl, (E)-2- pentenyl, (Z)-2-hexenyl, (E)-1-hexenyl, (Z)-1-hexenyl, (E)-2-hexenyl, (Z)-3- hexenyl, (E)-3-hexenyl and (E)-1,3-hexadienyl. "Substituted alkenyl" means that one or more positions in the alkenyl are substituted, especially1-4 substituents, which can be substituted at any position. Typical substituents include, but are not limited to, one or more of the following groups: such as hydrogen, deuterium, halogen (e. g., monohalogen substituents or polyhalogen substituents, the latter such as trifluoromethyl or an alkyl containing Cl₃), cyano, nitro, oxo (e. g. =O), trifluoromethyl, trifluoromethoxy, cycloalkyl, alkenyl, cycloalkenyl, alkynyl, heterocyclyl, aromatic ring, ORa, SRa, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein Rₐ appearing here may independently represent hydrogen, deuterium, alkyl, cycloalkyl, alkenyl, cycloalkenyl, alkynyl, heterocyclyl or aromatic ring, and R_{b}, R_{c} and R_{d} may independently represent hydrogen, deuterium, alkyl, cycloalkyl, heterocyclyl or aromatic ring, or R_{b} and R_{c} together with N atom can form a heterocyclyl; Rₑ can independently represent hydrogen, deuterium, alkyl, cycloalkyl, alkenyl, cycloalkenyl, alkynyl, heterocyclyl or aromatic ring. The above typical substituents, such as alkyl, cycloalkyl, alkenyl, cycloalkenyl, alkynyl, heterocyclyl or aromatic ring, may be optionally substituted.

The term "alkynyl" refers to a linear or branched hydrocarbon group containing 2 to 18 carbon atoms and at least one carbon-carbon triple bond. Typical groups include ethynyl. The term "(C₂-C₆) alkynyl" refers to a linear or branched group containing 2-6 carbon atoms and at least one carbon-carbon triple bond, such as ethynyl, 1-propynyl, 2-propynyl, 1-butynyl, 2-butynyl, 1-pentynyl, 2-pentynyl,1-hexynyl, 2-hexynyl, 3-hexynyl. "Substituted alkynyl" means that one or more positions in the alkynyl are substituted, especially1-4 substituents, which can be substituted at any position. Typical substituents include, but are not limited to, one or more of the following groups: such as hydrogen, deuterium, halogen (e. g., monohalogen substituents or polyhalogen substituents, the latter such as trifluoromethyl or an alkyl containing Cl₃), cyano, nitro, oxo (e. g. =O), trifluoromethyl, trifluoromethoxy, cycloalkyl, alkenyl, cycloalkenyl, alkynyl, heterocyclyl, aromatic ring, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein Rₐ appearing here may independently represent hydrogen, deuterium, alkyl, cycloalkyl, alkenyl, cycloalkenyl, alkynyl, heterocyclyl or aromatic ring, and R_{b}, R_{c} and R_{d} may independently represent hydrogen, deuterium, alkyl, cycloalkyl, heterocyclyl or aromatic ring, or R_{b} and R_{c} together with N atom can form a heterocyclyl; Rₑ can independently represent hydrogen, deuterium, alkyl, cycloalkyl, alkenyl, cycloalkenyl, alkynyl, heterocyclyl or aromatic ring. Typical substituents can be optionally substituted.

The term "cycloalkyl" refers to a fully saturated cyclic hydrocarbon compound group, including 1 to 4 rings, each contains 3 to 8 carbon atoms. "Substituted cycloalkyl" means that one or more positions in a cycloalkyl are substituted, especially 1 to 4 substituents, which can be substituted at any position. Typical substituents include, but are not limited to, one or more of the following groups: such as hydrogen, deuterium, halogen (e. g., monohalogen substituents or polyhalogen substituents, the latter such as trifluoromethyl or an alkyl containing Cl₃), cyano, nitro, oxo (e. g. =O), trifluoromethyl, trifluoromethoxy, cycloalkyl, alkenyl, cycloalkenyl, alkynyl, heterocyclyl, aromatic ring, ORa, SRa, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein Rₐ appearing here may independently represent hydrogen, deuterium, alkyl, cycloalkyl, alkenyl, cycloalkenyl, alkynyl, heterocyclyl or aromatic ring, and R_{b}, R_{c} and R_{d} may independently represent hydrogen, deuterium, alkyl, cycloalkyl, heterocyclyl or aromatic ring, or R_{b} and R_{c} together with N atom can form a heterocyclyl; Rₑ can independently represent hydrogen, deuterium, alkyl, cycloalkyl, alkenyl, cycloalkenyl, alkynyl, heterocyclyl or aromatic ring. The above typical substituents can be optionally substituted. Typical substitutions also include spiro, bridged or fused ring substituent, especially spiro alkyl, spiro alkenyl, spiro heterocyclyl (excluding heteroaromatic ring), bridged cycloalkyl, bridged cycloalkenyl, bridged heterocyclyl (excluding heteroaromatic ring), fused cycloalkyl, fused cycloalkenyl, fused heterocyclyl or fused aromatic ring, the above cycloalkyl, cycloalkenyl, heterocyclyl and heteroaryl may be optionally substituted.

The term "cycloalkenyl" refers to a partially unsaturated cyclic hydrocarbon compound group, including 1 to 4 rings, each contains 3 to 8 carbon atoms. Typical cycloalkenyl such as cyclobutenyl, cyclopentenyl, cyclohexenyl, etc. "Substituted cycloalkenyl" means that one or more positions in a cycloalkyl are substituted, especially 1 to 4 substituents, which can be substituted at any position. Typical substituents include, but are not limited to, one or more of the following groups: such as hydrogen, deuterium, halogen (e. g., monohalogen substituents or polyhalogen substituents, the latter such as trifluoromethyl or an alkyl containing Cl₃), cyano, nitro, oxo (e. g. =O), trifluoromethyl, trifluoromethoxy, cycloalkyl, alkenyl, cycloalkenyl, alkynyl, heterocyclyl, aromatic ring, ORa, SRa, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein Rₐ appearing here may independently represent hydrogen, deuterium, alkyl, cycloalkyl, alkenyl, cycloalkenyl, alkynyl, heterocyclyl or aromatic ring, and R_{b}, R_{c} and R_{d} may independently represent hydrogen, deuterium, alkyl, cycloalkyl, heterocyclyl or aromatic ring, or R_{b} and R_{c} together with N atom can form a heterocyclyl; Rₑ can independently represent hydrogen, deuterium, alkyl, cycloalkyl, alkenyl, cycloalkenyl, alkynyl, heterocyclyl or aromatic ring. The above typical substituents can be optionally substituted. Typical substitutions also include spiro or fused ring substituent, in particular spiro cycloalkyl, spiro cycloalkenyl, spiro heterocyclyl (excluding heteroaryl), fused cycloalkyl, fused cycloalkenyl, fused heterocyclyl or fused aromatic ring, the above cycloalkyl, cycloalkenyl, heterocyclyl and heteroaryl may be optionally substituted.

The term "heterocyclyl" refers to a fully saturated or partially unsaturated cyclic group (including, but not limited to, such as a 4-7-membered monocyclic ring, 7-11membered bicyclic ring, or 8-16 membered tricyclic system) in which at least one heteroatom is present in a ring having at least one carbon atom. Each heterocyclyl containing heteroatoms can have 1, 2, 3 or 4 heteroatoms selected from nitrogen atom, oxygen atom or sulfur atom, wherein nitrogen atom or sulfur atom can be oxidized and nitrogen atom can also be quaternized. Heterocyclyl can be attached to a residue of any heteroatom or carbon atom of a ring or ring system molecule. Typical monocyclic heterocyclyls include, but are not limited to, azetidinyl, pyrrolidinyl, oxetanyl, pyrazolinyl, imidazolinyl, imidazolidinyl, oxazolidinyl, isoxazolidinyl, thiazolidinyl, isothiazolidinyl, tetrahydrofuranyl, piperidinyl, piperazinyl,2-oxopiperazinyl, 2-oxopiperidinyl, 2-oxopyrrolidinyl, hexahydroazepinyl, 4-oxopiperidinyl, tetrahydropyranyl, morpholinyl, thiomorpholinyl, thiomorpholinyl sulfoxide group, thiomorpholine sulfone group, 1,3-dioxanyl and tetrahydro-1,1-dioxothiophene, etc. Polycyclic heterocyclyls include spiro, fused and bridged heterocyclyl; the spiro, fused and bridged heterocyclyl involved are optionally connected with other groups through single bonds, or further fused with other cycloalkyl, heterocyclyl, aryl and heteroaryl through any two or more atoms on the ring; heterocyclyl can be substituted or unsubstituted, when being substituted, the substituent is preferably one or more of the following groups, which is independently selected from the group consisting of alkyl, deuterated alkyl, haloalkyl, alkoxy, haloalkoxy, alkenyl, alkynyl, alkylthio, alkylamino, halogen, amino, nitro, hydroxyl, mercapto, cyano, cycloalkyl, heterocyclyl, aryl, heteroaryl, cycloalkylthio, oxo, carboxyl, and carboxylate.

The term "aryl" refers to an aromatic cyclic hydrocarbon compound group having 1 to 5 rings, especially monocyclic and bicyclic groups, such as phenyl, biphenyl, or naphthyl. Aromatic rings where there are two or more aromatic rings (bicyclic, etc.), aryl groups can be linked by single bonds (such as biphenyl) or fused (such as naphthyl, anthryl, etc.). "Substituted aryl" means that one or more positions in the aryl are substituted, especially 1 to 3 substituents, which can be substituted at any position. Typical substituents include, but are not limited to, one or more of the following groups: such as hydrogen, deuterium, halogen (e. g., monohalogen substituents or polyhalogen substituents, the latter such as trifluoromethyl or an alkyl containing Cl₃), cyano, nitro, oxo (e. g. =O), trifluoromethyl, trifluoromethoxy, cycloalkyl, alkenyl, cycloalkenyl, alkynyl, heterocyclyl, aromatic ring, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein Rₐ appearing here may independently represent hydrogen, deuterium, alkyl, cycloalkyl, alkenyl, cycloalkenyl, alkynyl, heterocyclyl or aromatic ring, and R_{b}, R_{c} and R_{d} may independently represent hydrogen, deuterium, alkyl, cycloalkyl, heterocyclyl or aromatic ring, or R_{b} and R_{c} together with N atom can form a heterocyclyl; Rₑ can independently represent hydrogen, deuterium, alkyl, cycloalkyl, alkenyl, cycloalkenyl, alkynyl, heterocyclyl or aromatic ring. The above typical substituents can be optionally substituted. Typical substituents also include fused ring substituents, especially fused cycloalkyl, fused cycloalkenyl, fused heterocyclyl or fused aromatic ring, the above cycloalkyl, cycloalkenyl, heterocyclyl and heteroaryl may be optionally substituted.

The term "heteroaryl" refers to a heteroaromatic system containing 1-4 heteroatoms and 5-14 ring atoms, wherein the heteroatoms are selected from oxygen, nitrogen, or sulfur. The heteroaryl is preferably a 5- to 10-membered ring, more preferably 5- or 6-membered, for example, pyrrolyl, pyrazolyl, imidazolyl, oxazolyl, isoxazolyl, thiazolyl, thiadiazolyl, isothiazolyl, furanyl, pyridyl, pyrazinyl, pyrimidinyl, pyridazinyl, triazinyl, triazolyl, and tetrazolyl. The "heteroaryl" may be substituted or unsubstituted, and when being substituted, the substituent is preferably one or more of the following groups independently selected from the group consisting of alkyl, deuterated alkyl, haloalkyl, alkoxy, haloalkoxy, alkenyl, alkynyl, alkylthio, alkylamino, halogen, amino, nitro, hydroxyl, mercapto, cyano, cycloalkyl, heterocyclyl, aryl, heteroaryl, cycloalkylthio, oxo, carboxyl, and carboxylate.

The term "amido" refers to a group having a structure of -CONRR", wherein R and R" may independently represent hydrogen, alkyl or substituted alkyl, cycloalkyl or substituted cycloalkyl, cycloalkenyl or substituted cycloalkenyl, aryl or substituted aryl, heterocyclyl or substituted heterocyclyl, as defined above. R and R" can be the same or different in the dialkylamino fragment.

The term "sulfonamido" refers to a group having a structure of -SO₂NRR", wherein R and R" may independently represent hydrogen, alkyl or substituted alkyl, cycloalkyl or substituted cycloalkyl, cycloalkenyl or substituted cycloalkenyl, aryl or substituted aryl, heterocyclyl or substituted heterocyclyl, as defined above. R and R" can be the same or different in the dialkylamino fragment.

The term "halogen" or "halo" refers to chlorine, bromine, fluorine, and iodine.

The term "halogenated" refers to being substituted by halogen.

The term "deuterated" refers to being substituted by deuterium.

The term "C1-C18 alkoxy" refers to a linear or branched or cyclic alkoxy having 1 to 18 carbon atoms, including, without limitation, methoxy, ethoxy, propoxy, isopropoxy, butoxy, and the like. Preferably C1-C8 alkoxy, more preferably C1-C6 alkoxy. The term "C1-C18 alkoxyene" refers to a group obtained by removing a hydrogen atom from "C1-C18 alkoxy". The terms "C1-C8 alkoxyene" and "C1-C6 alkoxyene" have similar meanings.

In the present invention, the term "substituted" means that one or more hydrogen atoms on a specific group are replaced with a specific substituent. The specific substituents are the substituents described correspondingly in the foregoing, or the substituents appearing in the respective examples. Unless otherwise specified, a substituted group may have a substituent selected from a specific group at any substitutable position of the group, and the substituent may be the same or different at each position. Those skilled in the art will understand that the combinations of substituents contemplated by the present invention are those that are stable or chemically achievable. The substituents are, for example (but not limited to): halogen, hydroxyl, carboxyl (-COOH), C1-C6 alkyl, C2-C6 alkenyl, C2-C6 alkynyl, C3-C8 cycloalkyl, 3-12 membered heterocyclyl, aryl, heteroaryl, C1-C8 aldehyde group, C2-C10 acyl, C2-C10 ester group, amino, C1-C6 alkoxy, C1-C10 sulfonyl and the like.

Unless otherwise stated, it is assumed that any heteroatom that is not in its valence state has enough hydrogen atoms to supplement its valence state.

### Compound

The invention provides an imidazoquinoline-substituted phosphoric ester compound having a structure of general formula (I), or a stereoisomer, a tautomer, a crystal form, or a pharmaceutically acceptable salt, a hydrate, a solvate or a prodrug thereof: 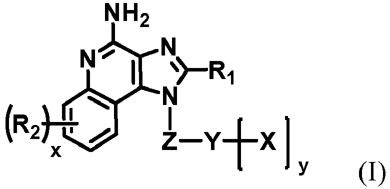 wherein, each group is as defined above.

In another preferred embodiment, in the compound, any one of R₁, R₂, x, Z, Y, X and y is a corresponding group in the specific compound described below.

In another preferred embodiment, the compound is preferably a compound prepared in the examples.

In another preferred embodiment, the compound is selected from the group consisting of:

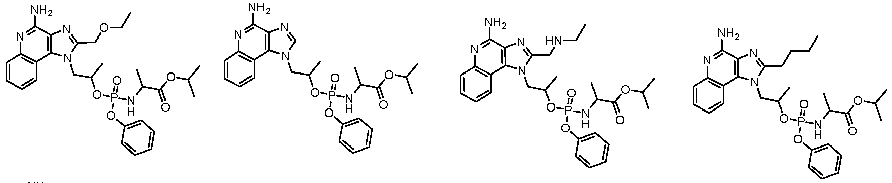

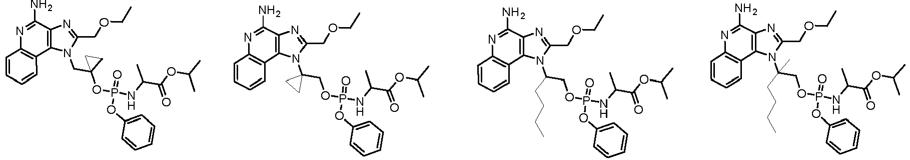

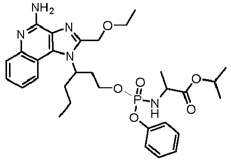

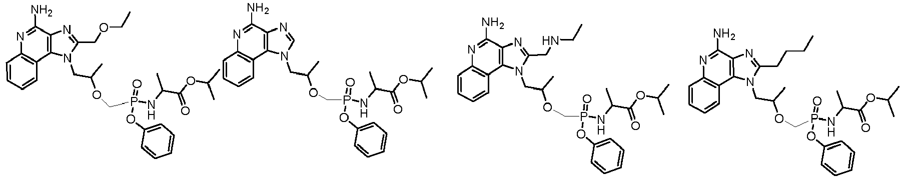

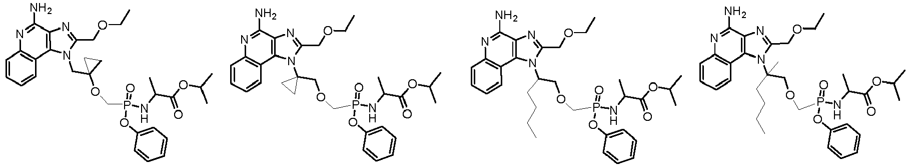

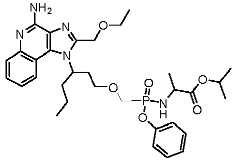

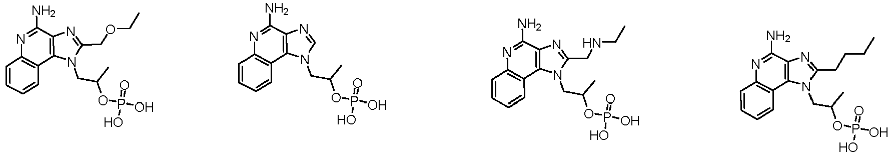

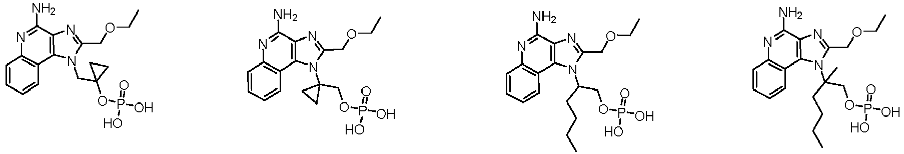

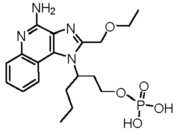

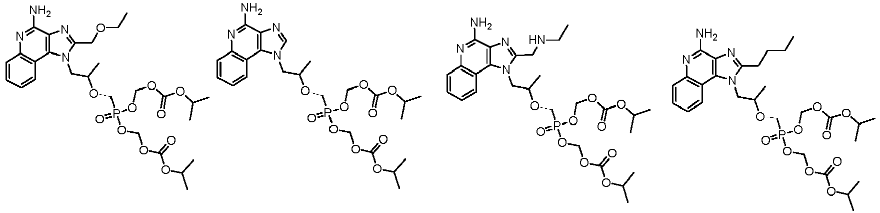

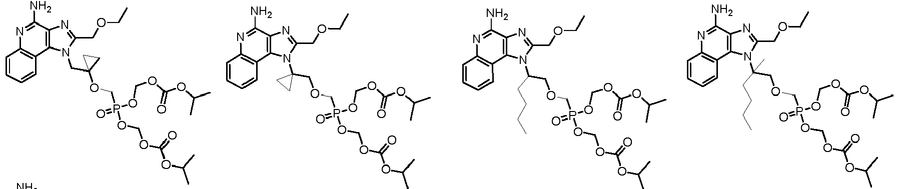

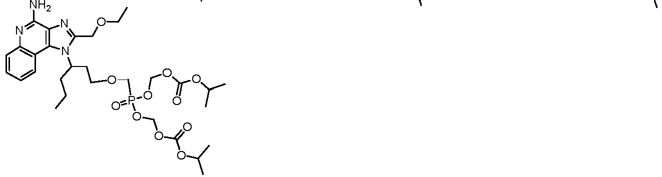

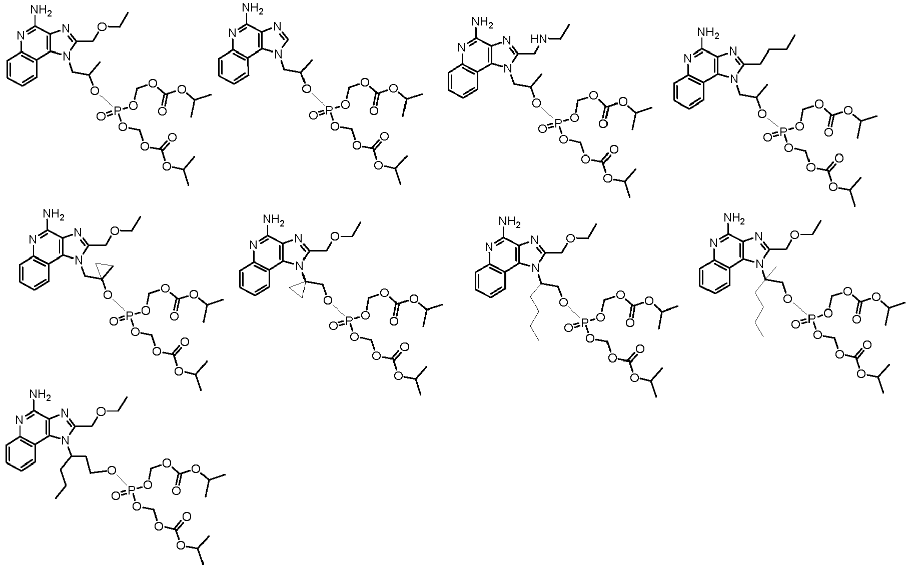

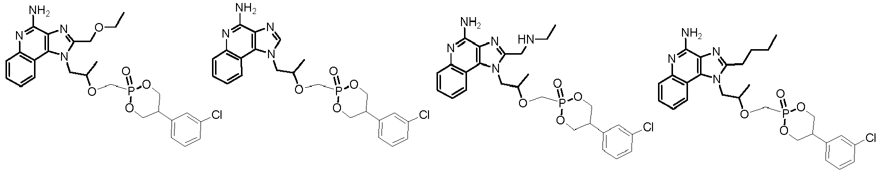

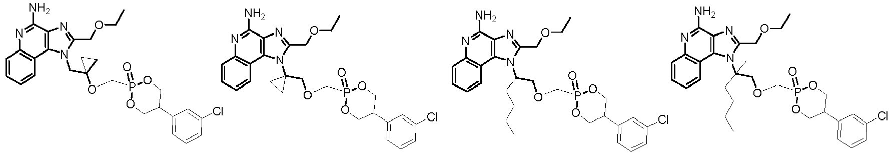

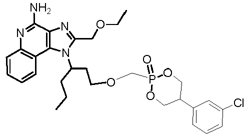

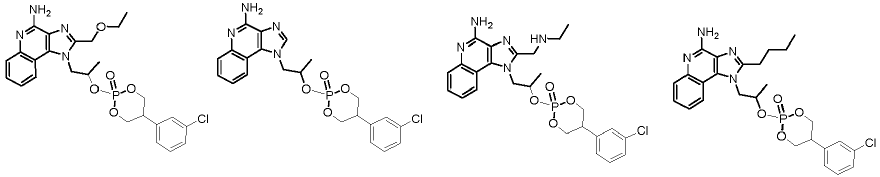

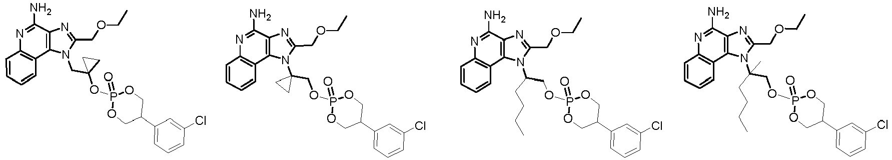

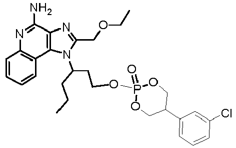

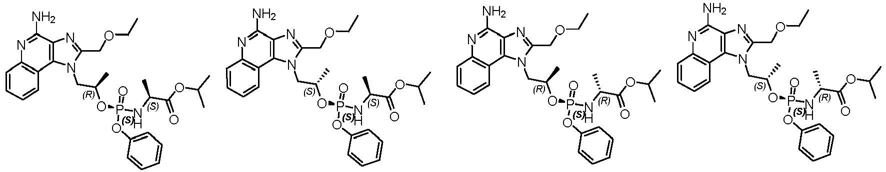

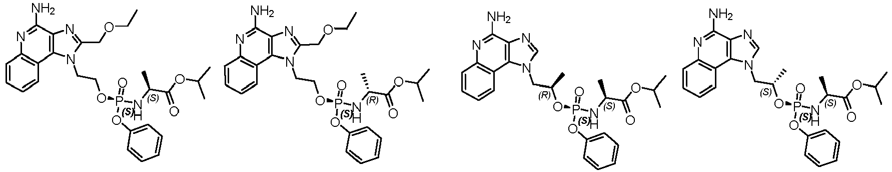

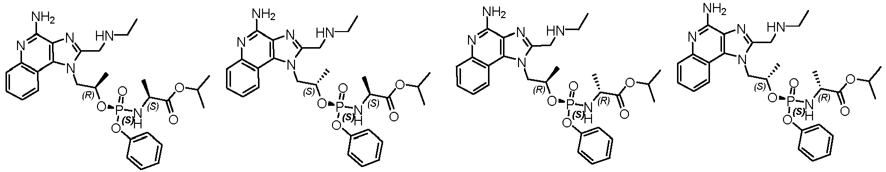

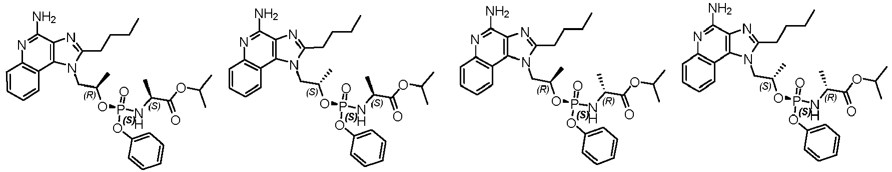

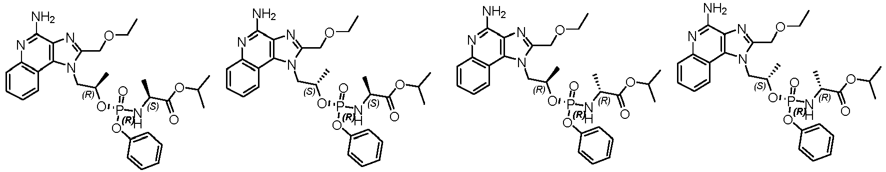

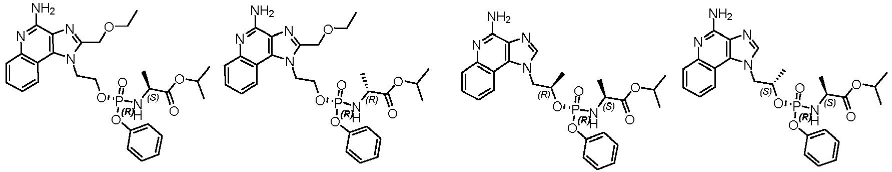

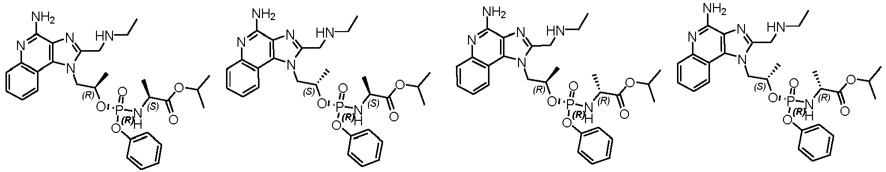

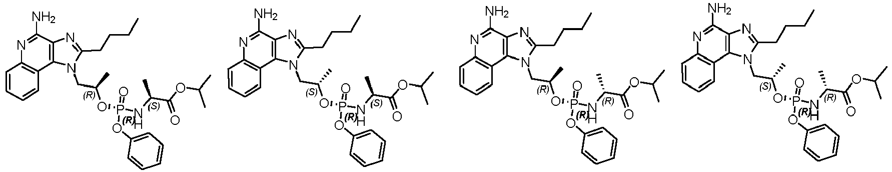

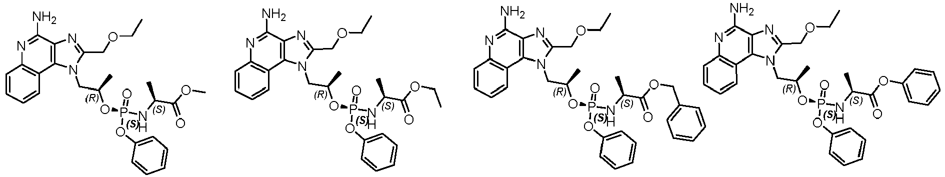

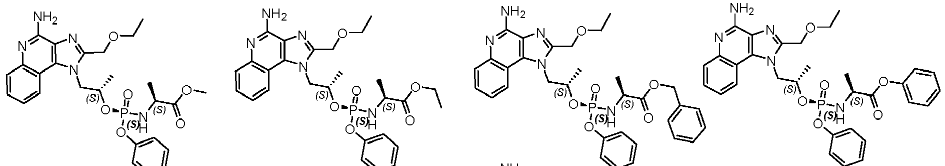

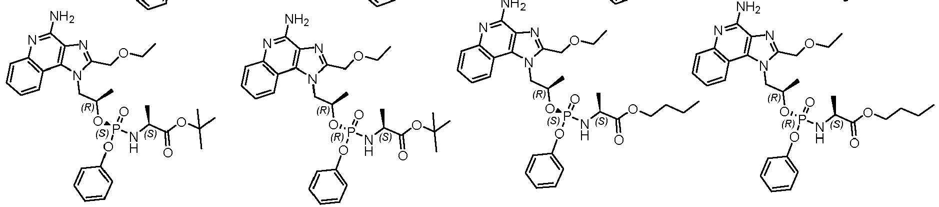

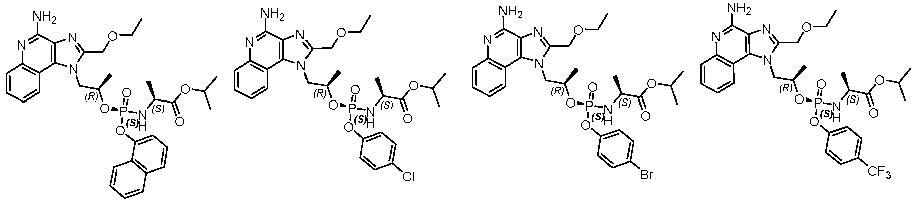

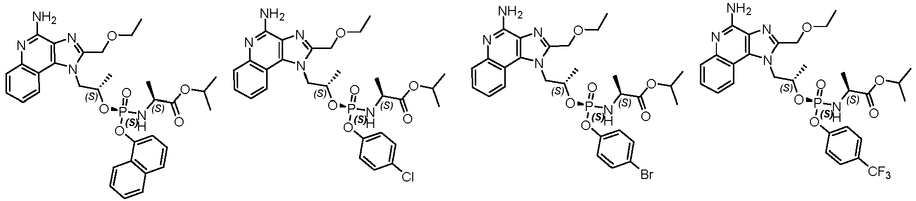

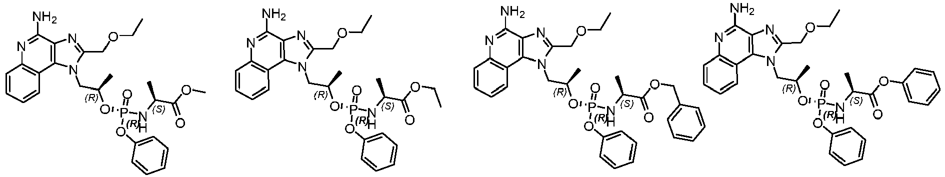

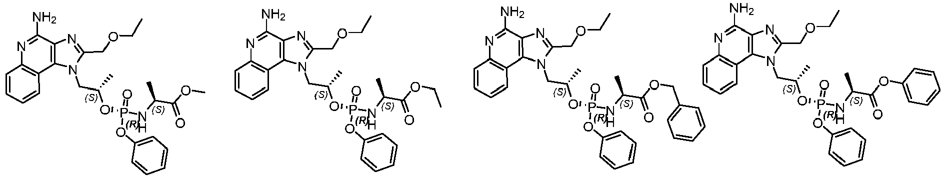

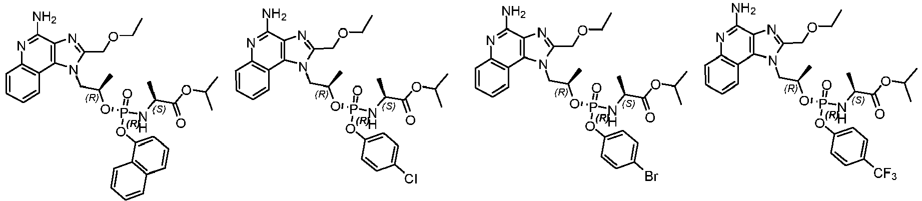

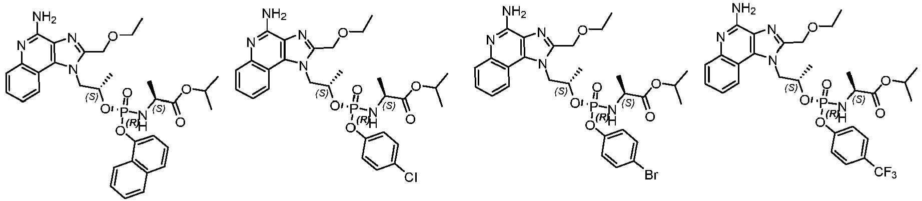

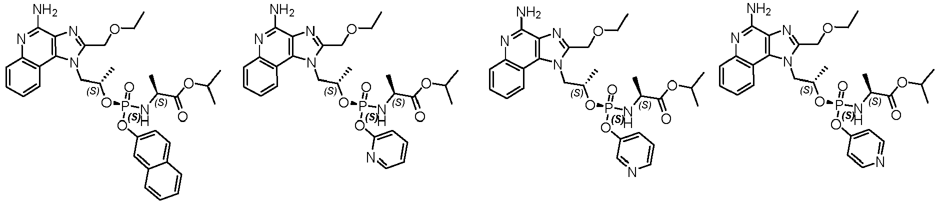

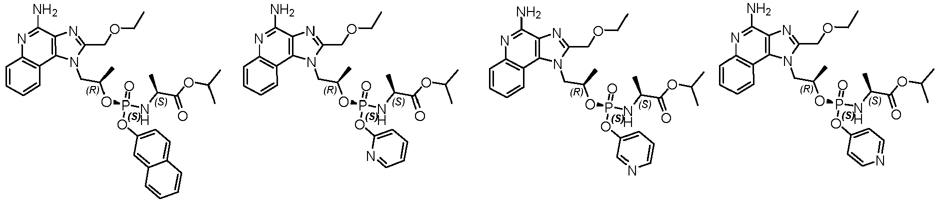

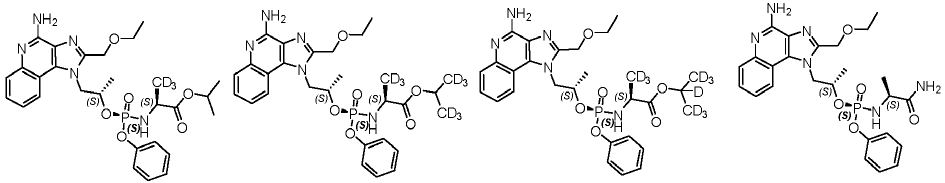

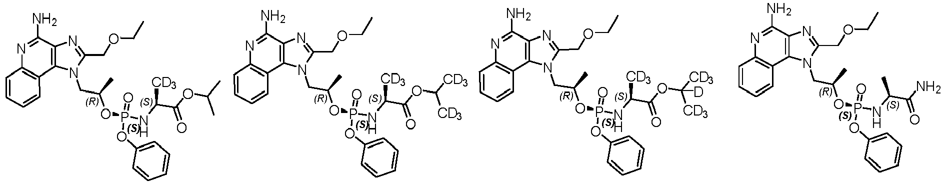

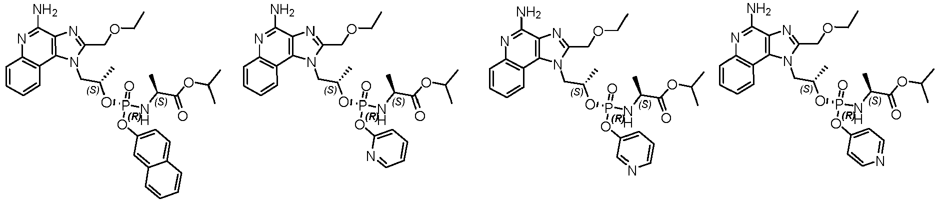

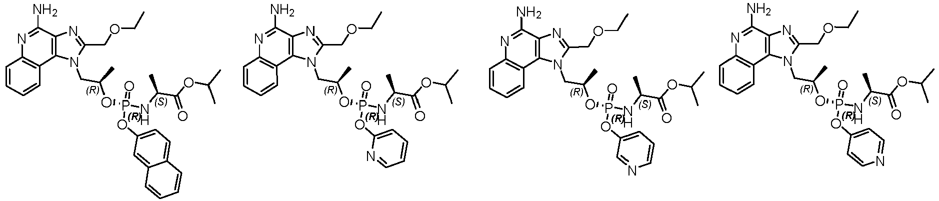

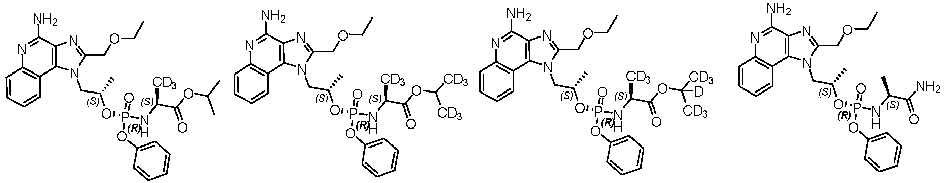

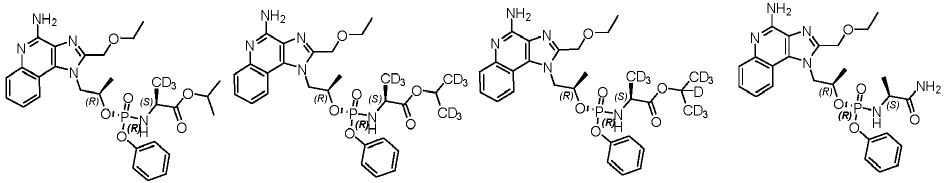

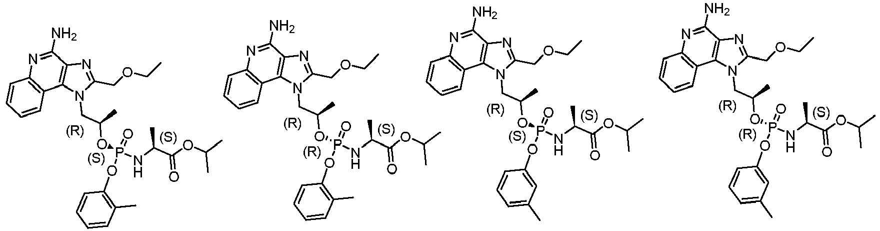

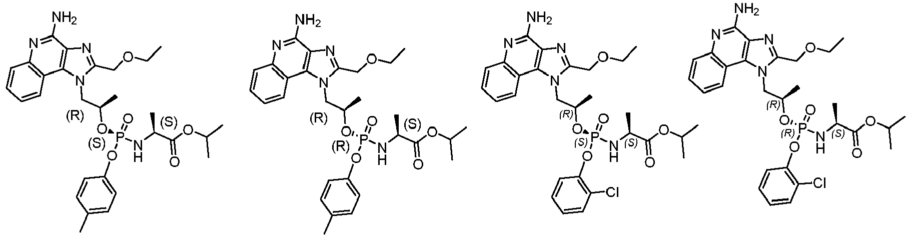

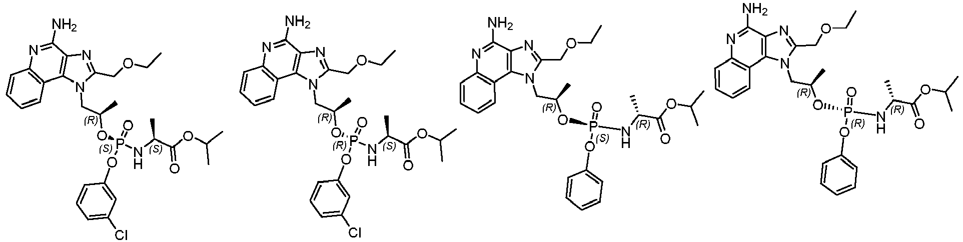

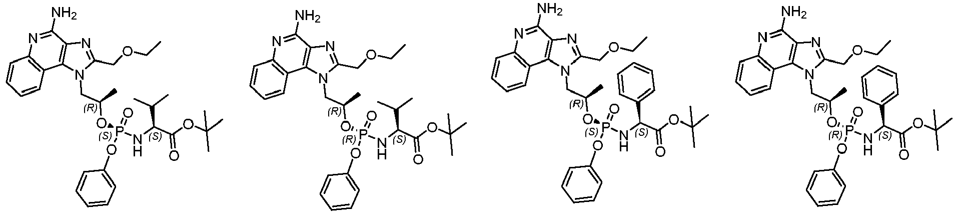

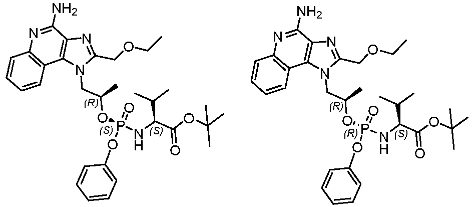

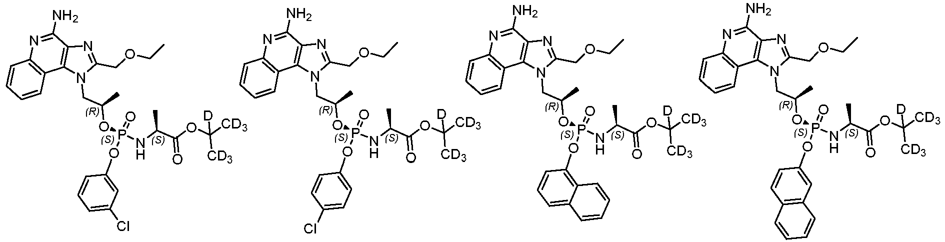

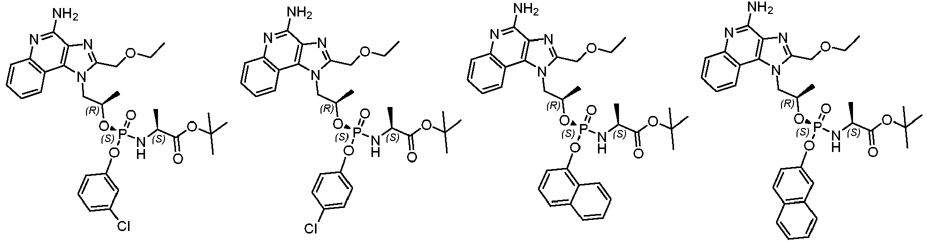

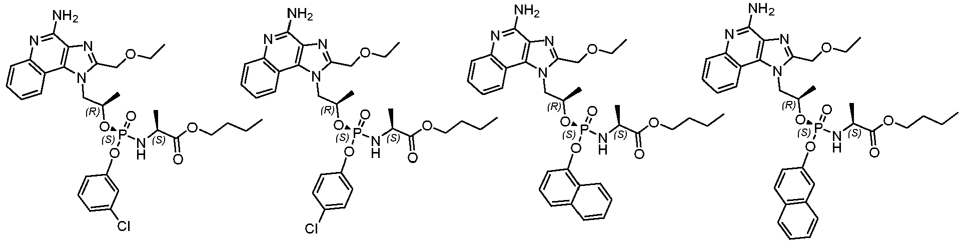

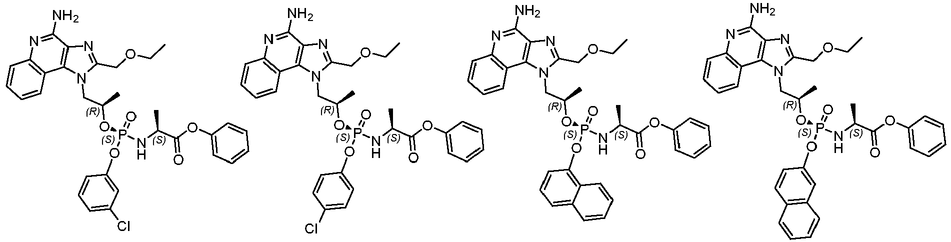

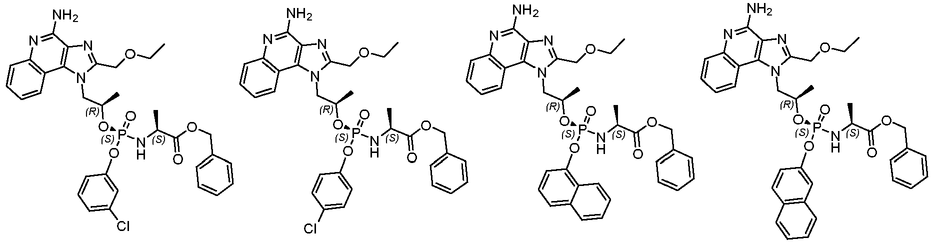

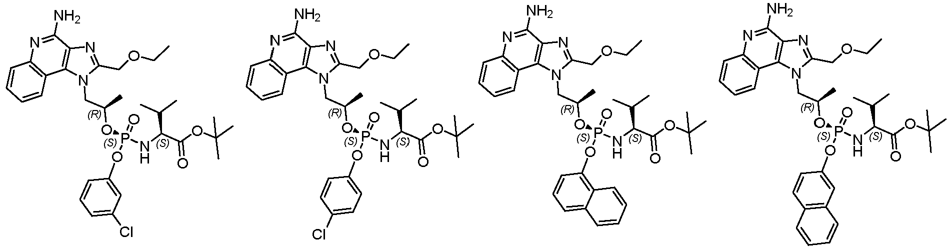

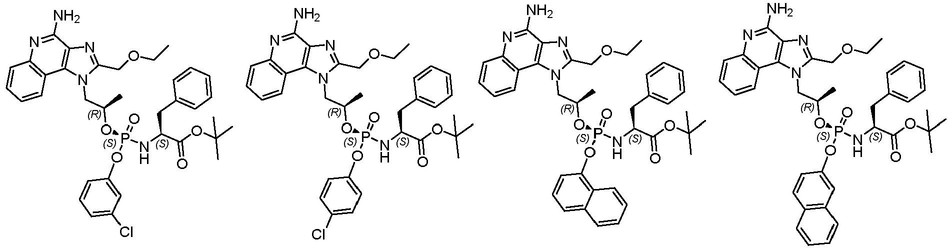

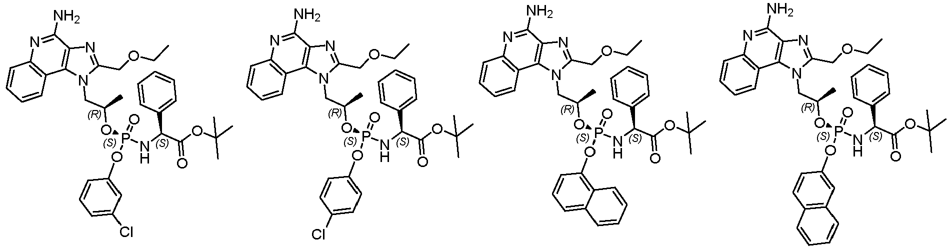

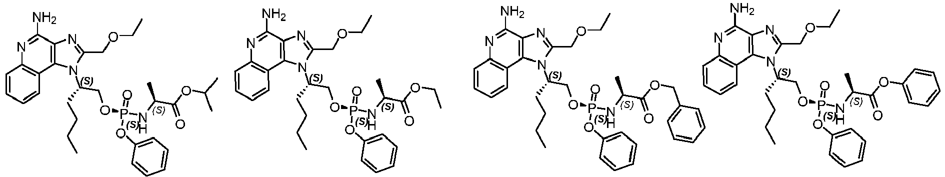

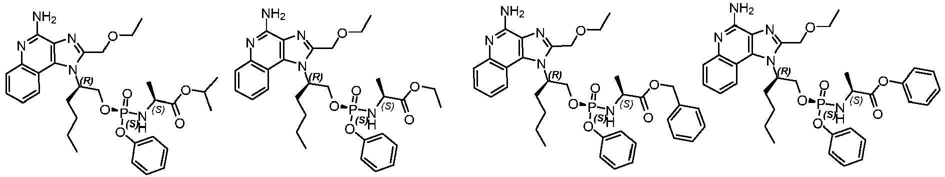

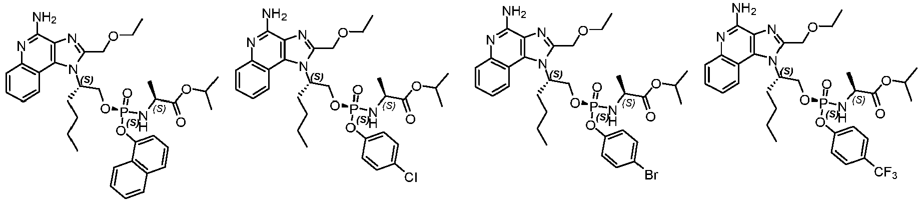

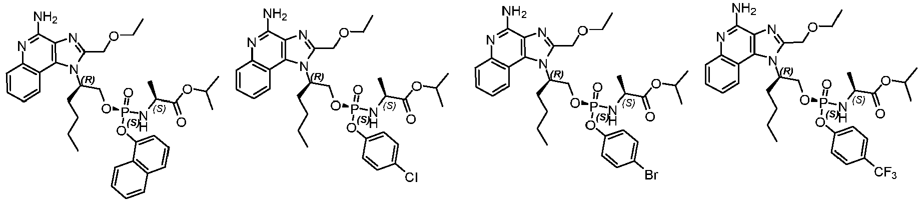

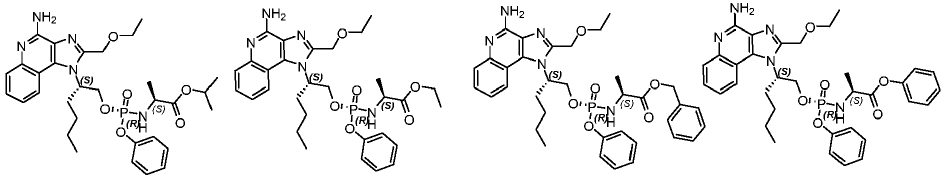

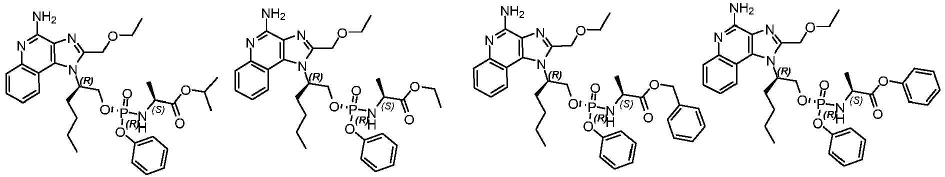

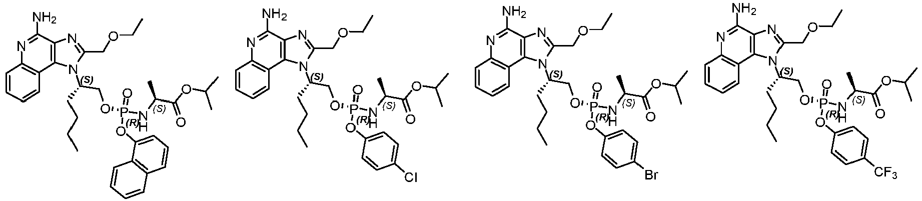

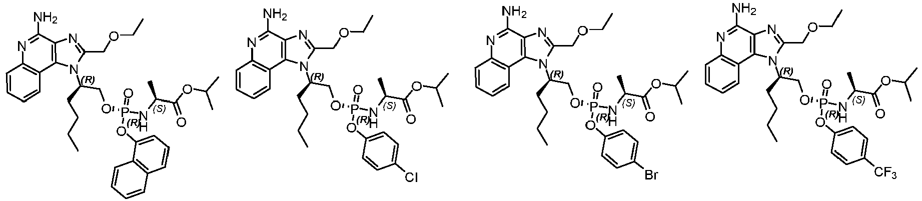

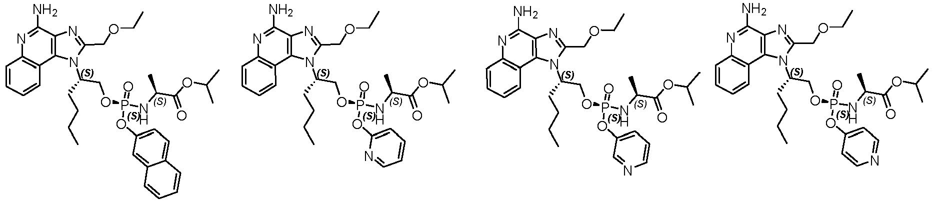

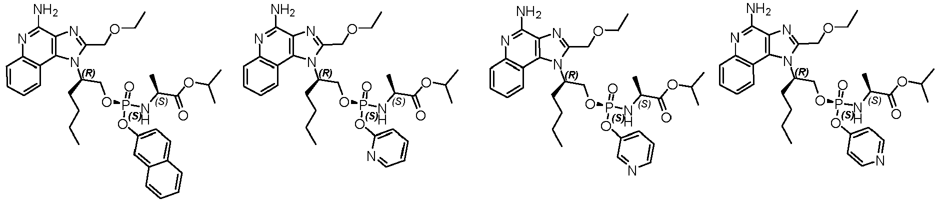

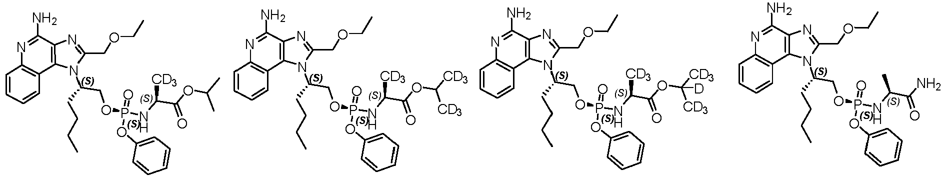

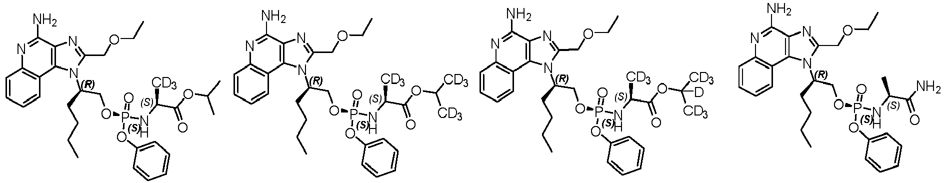

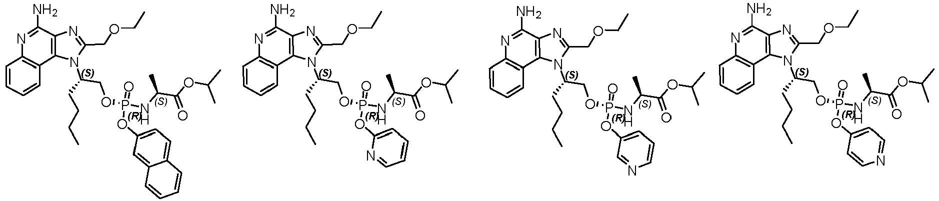

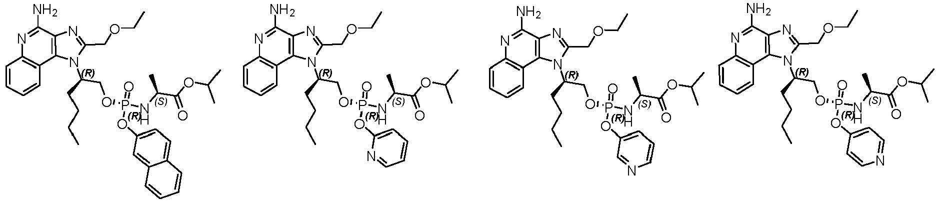

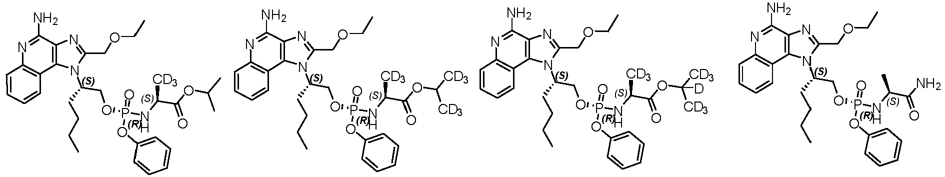

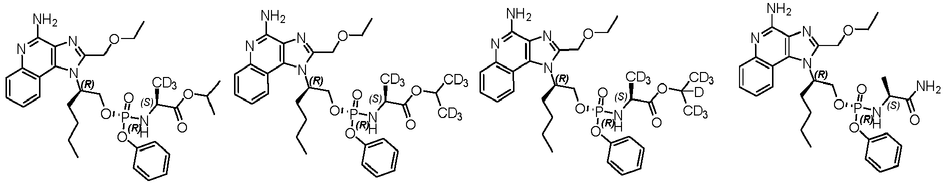

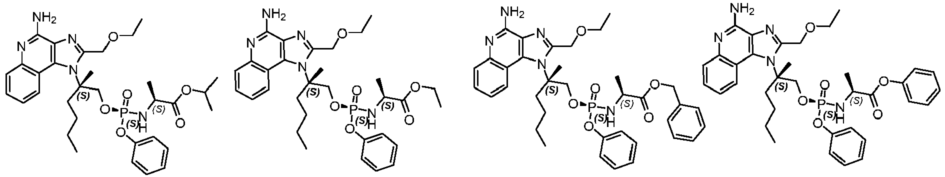

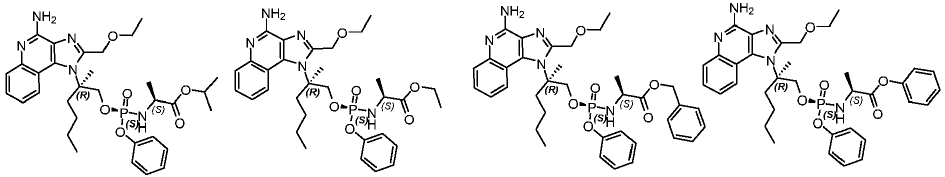

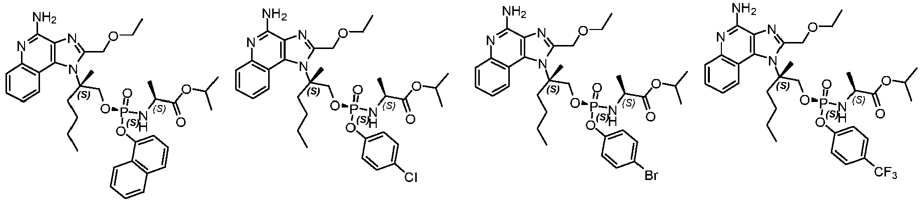

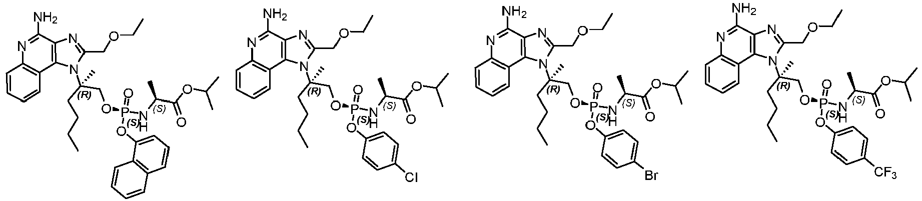

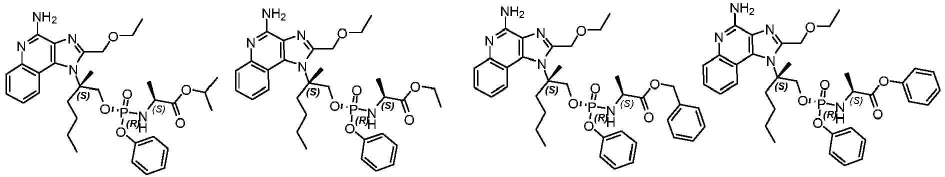

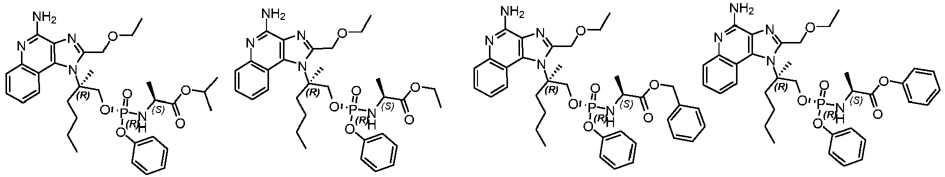

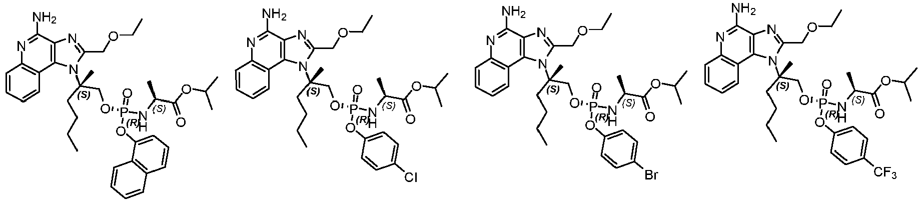

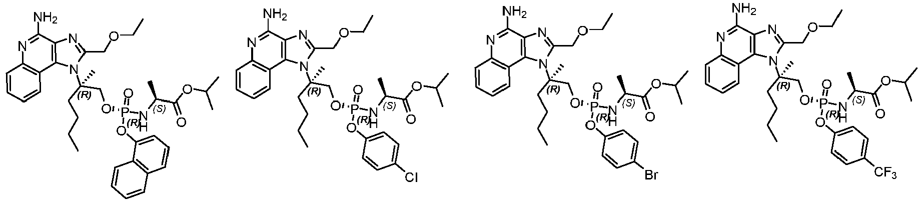

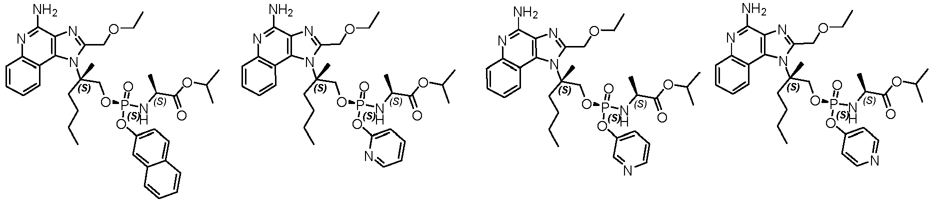

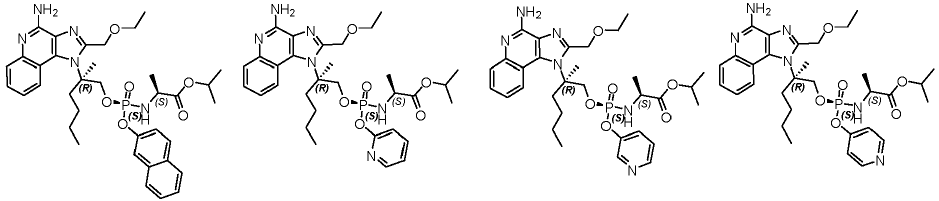

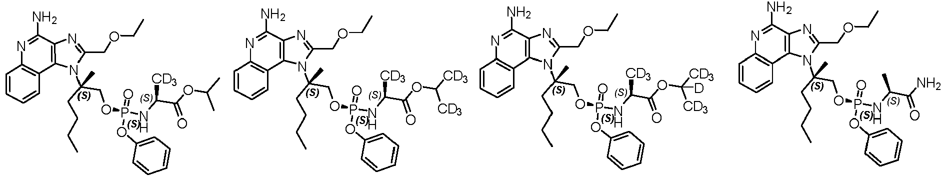

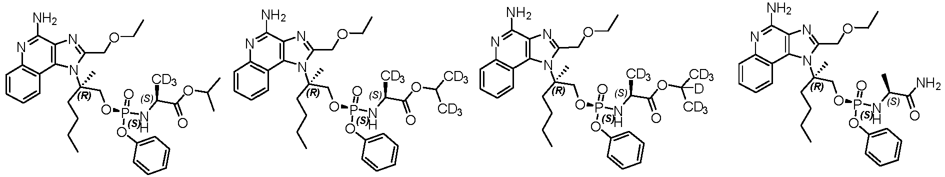

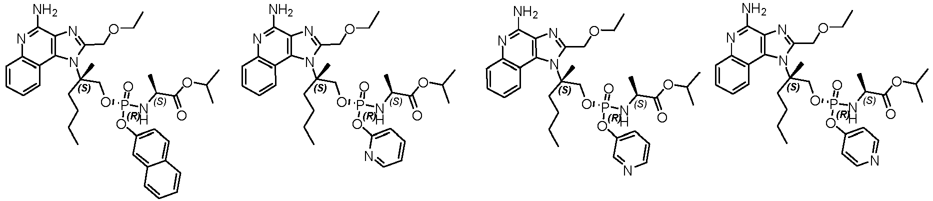

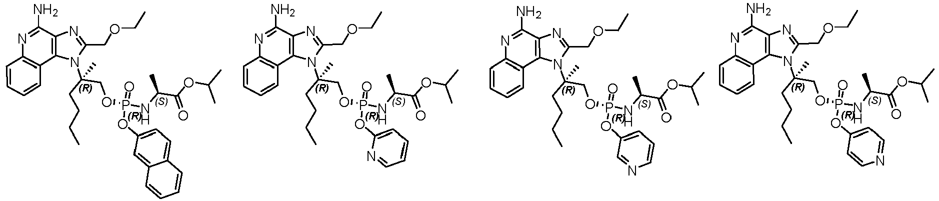

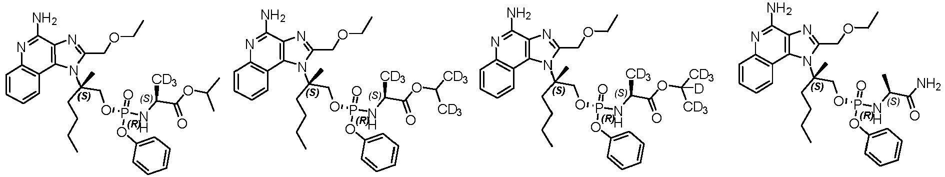

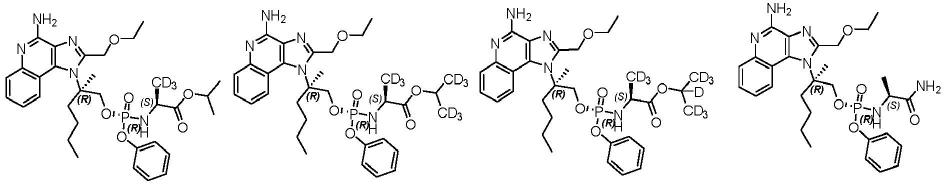

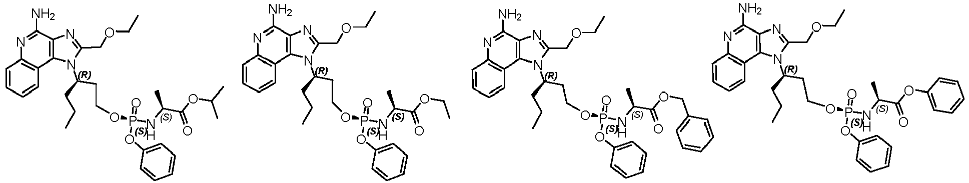

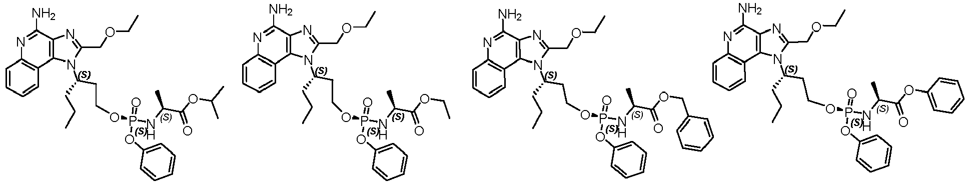

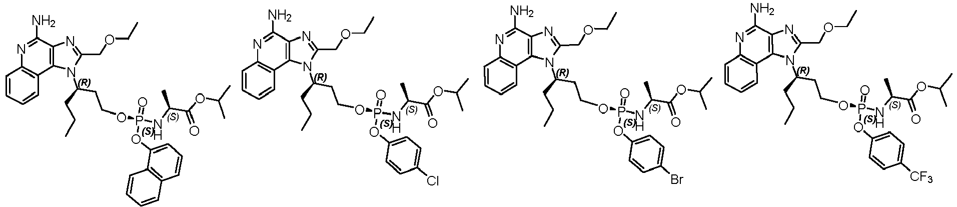

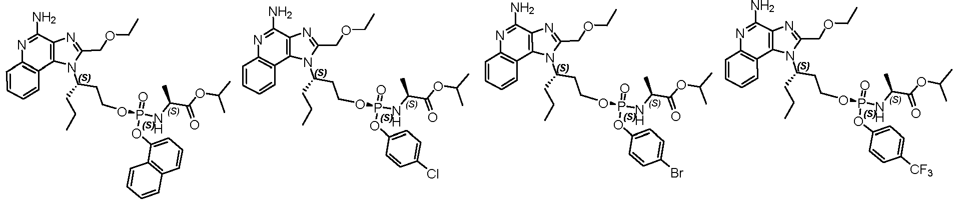

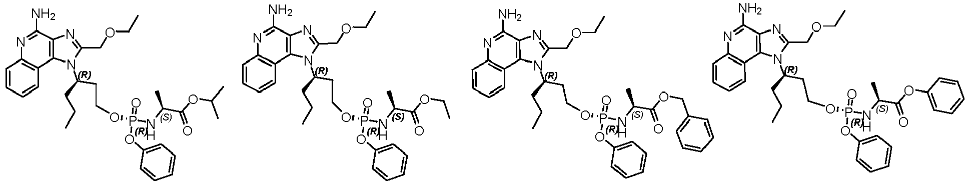

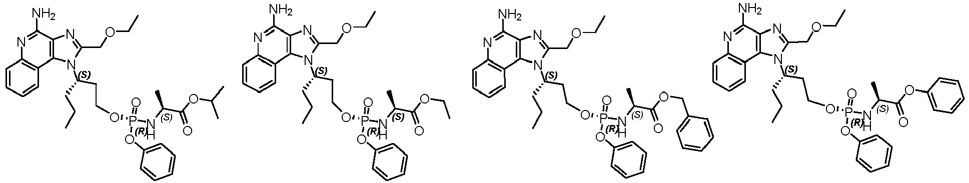

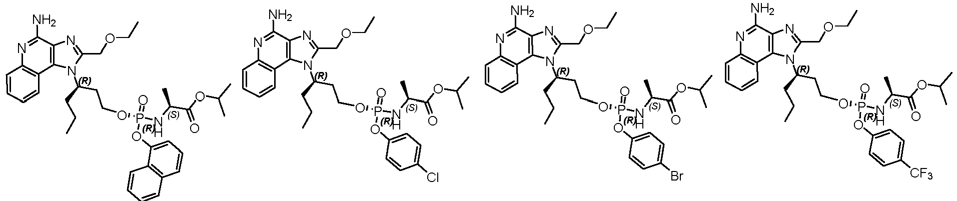

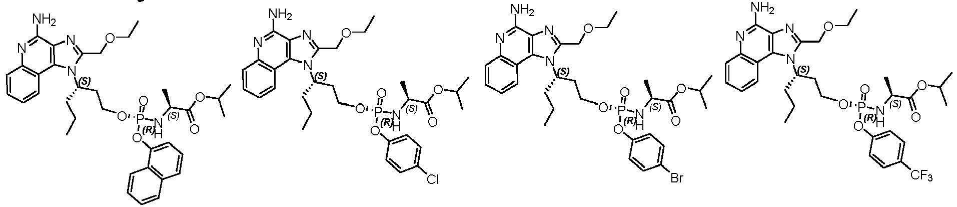

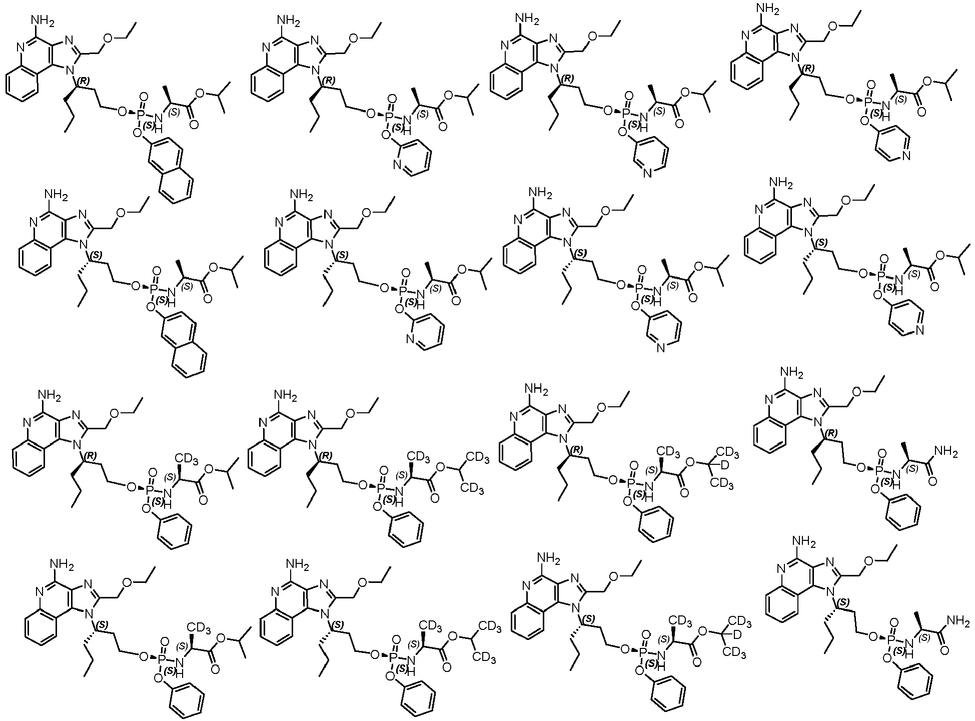

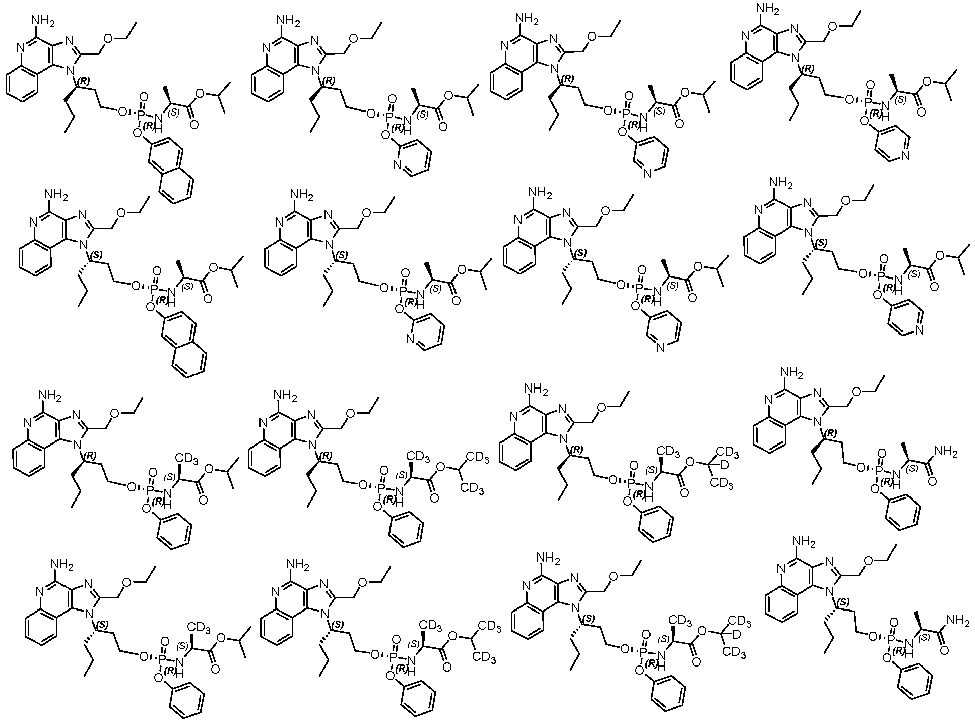

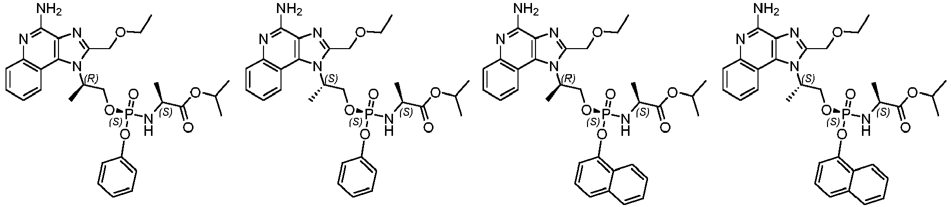

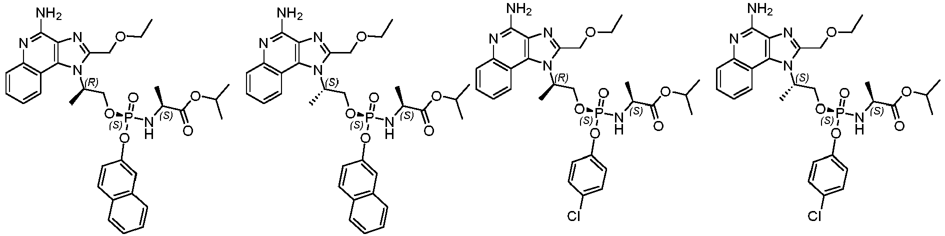

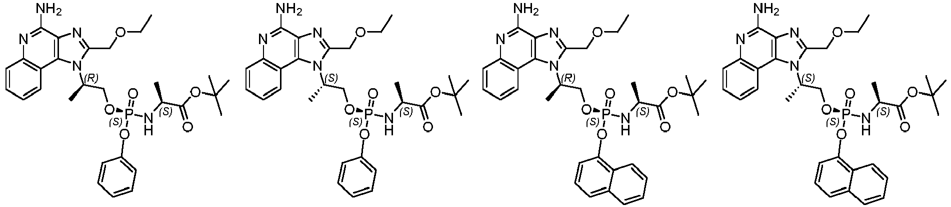

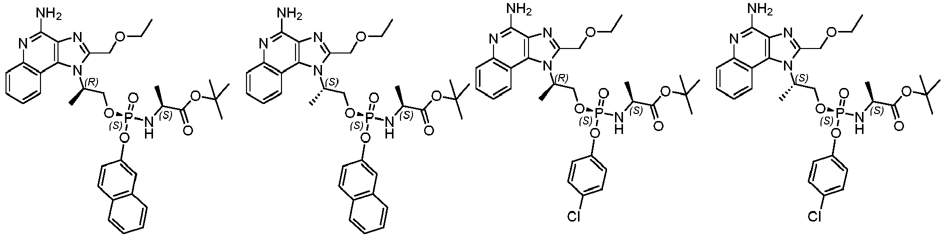

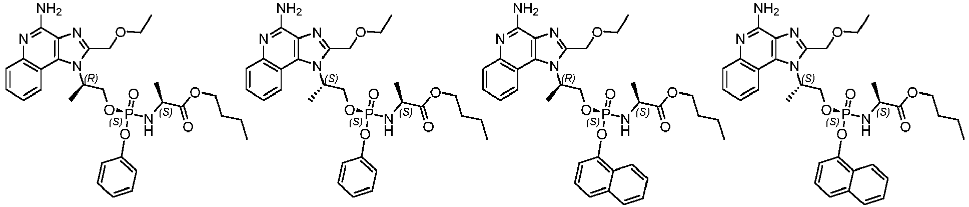

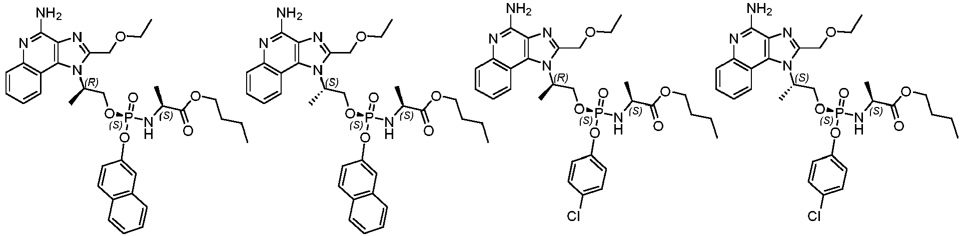

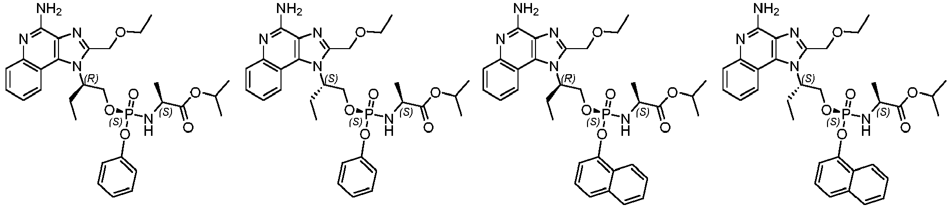

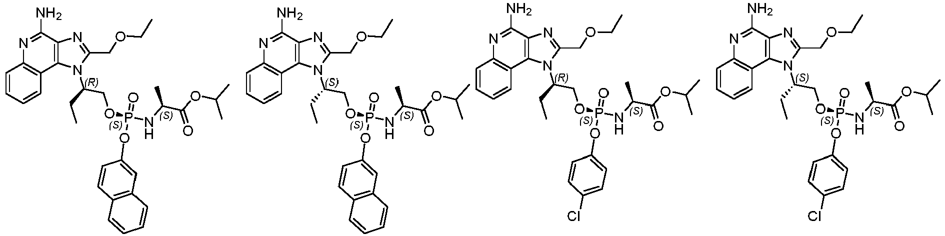

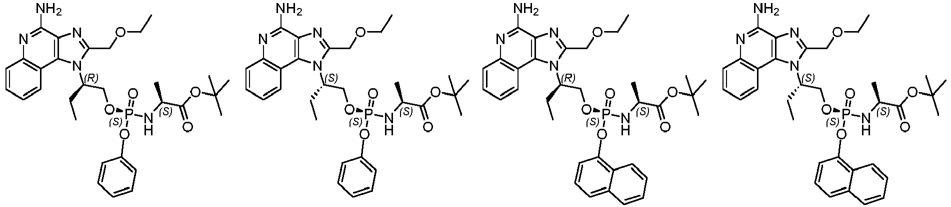

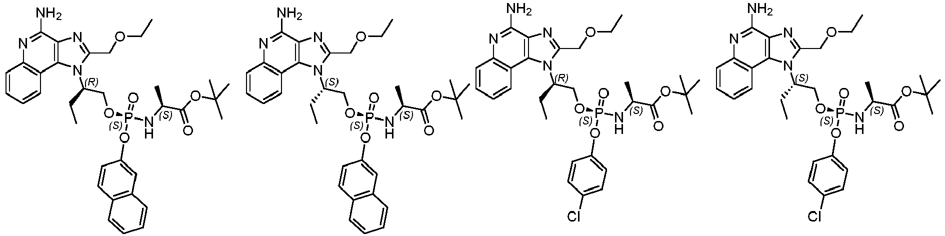

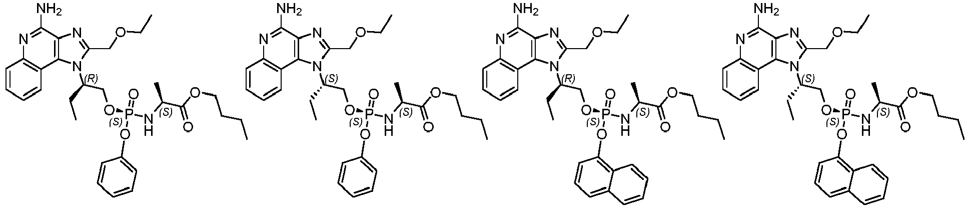

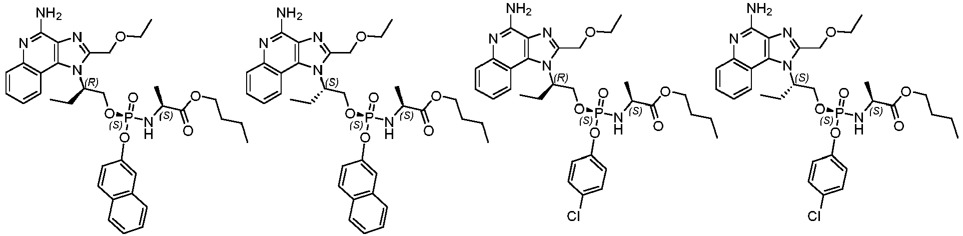

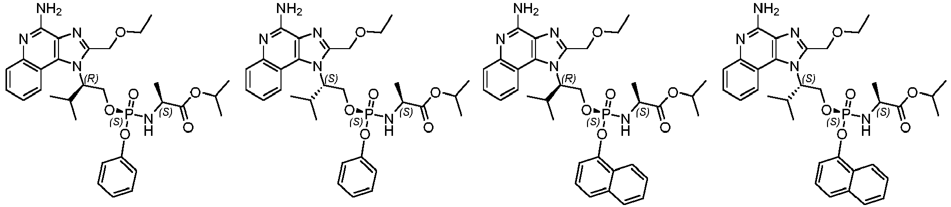

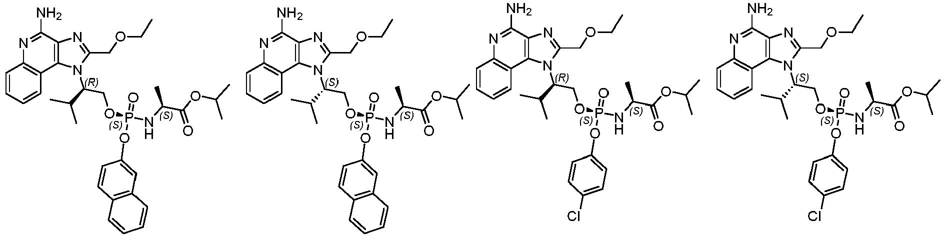

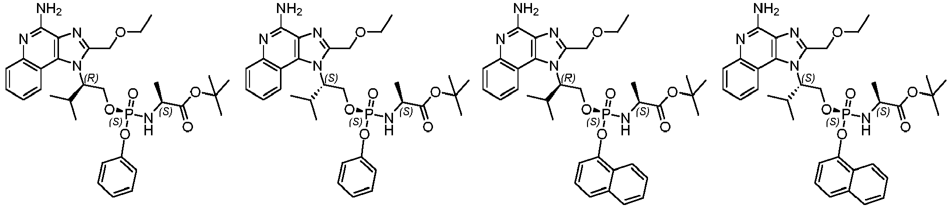

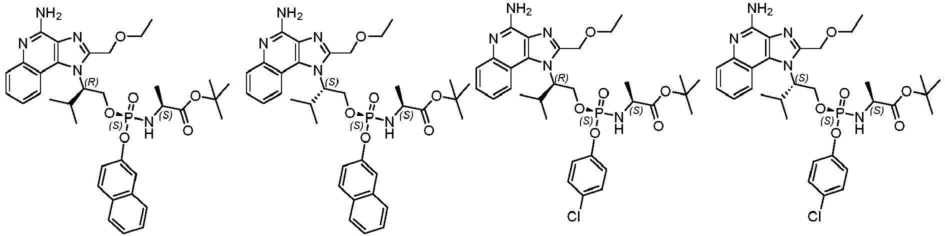

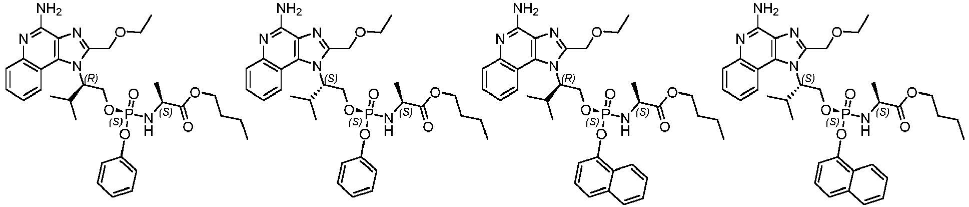

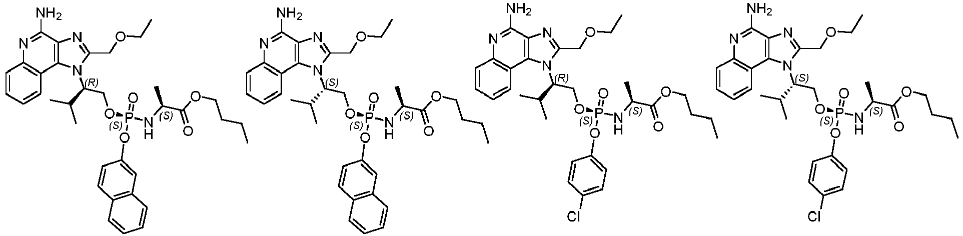

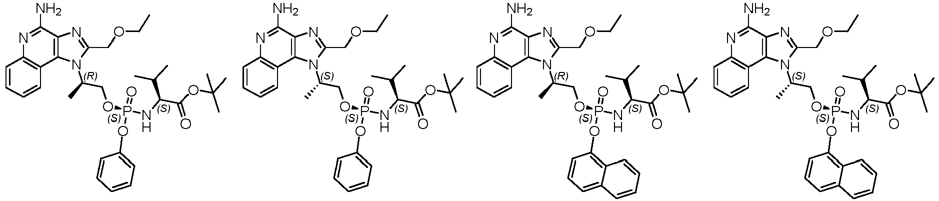

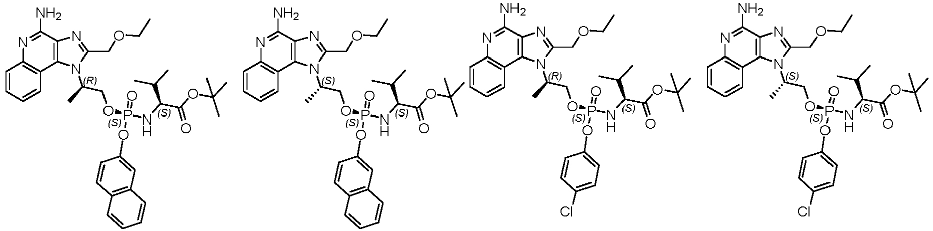

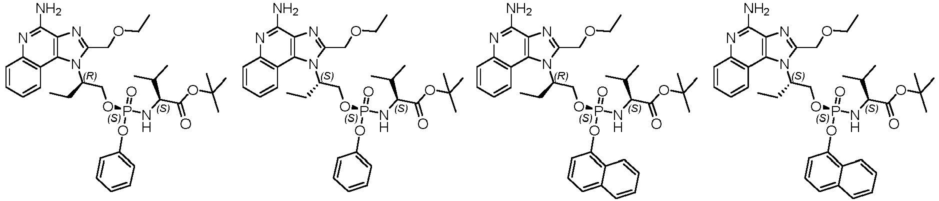

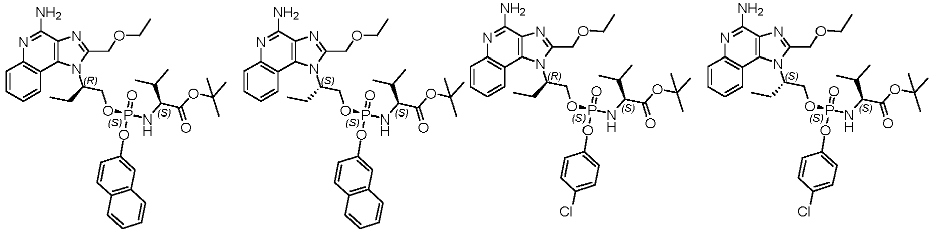

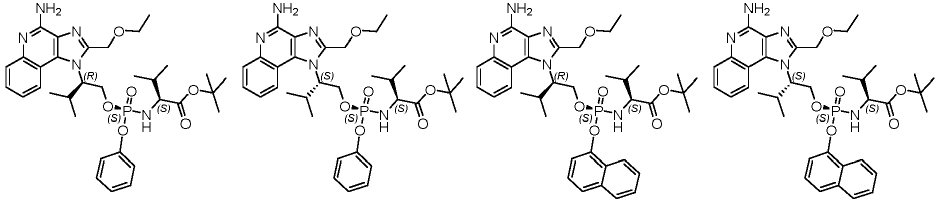

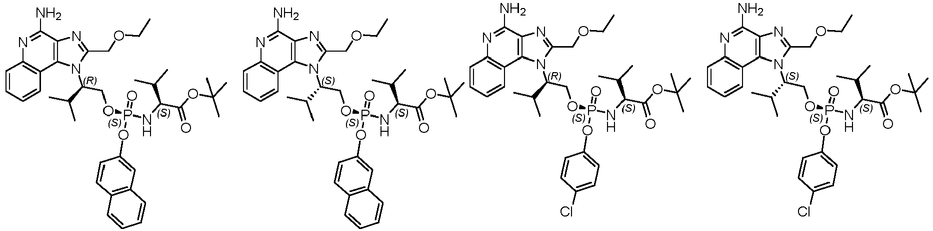

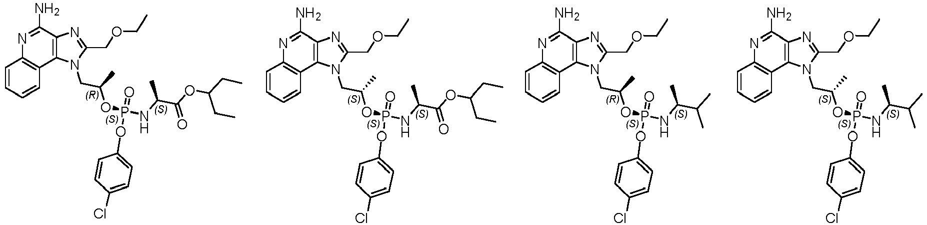

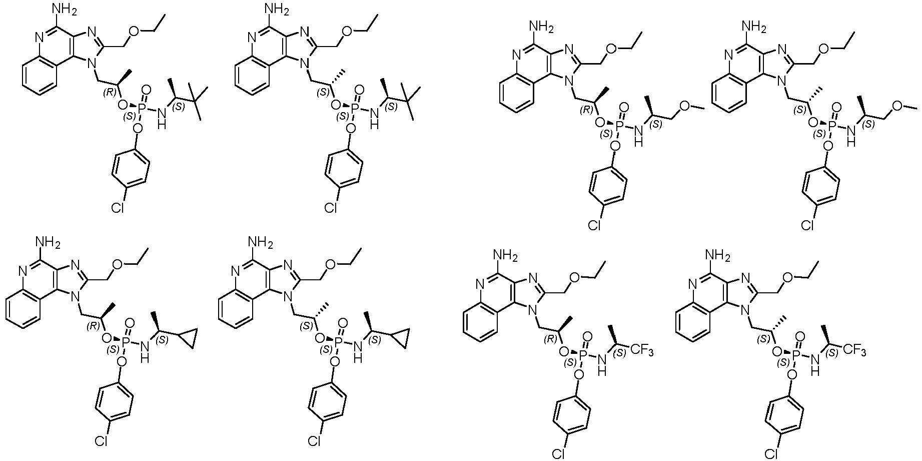

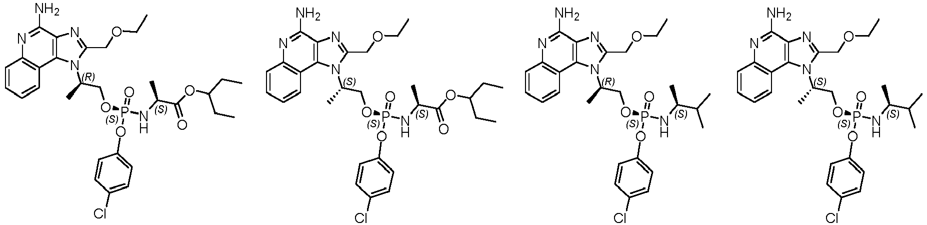

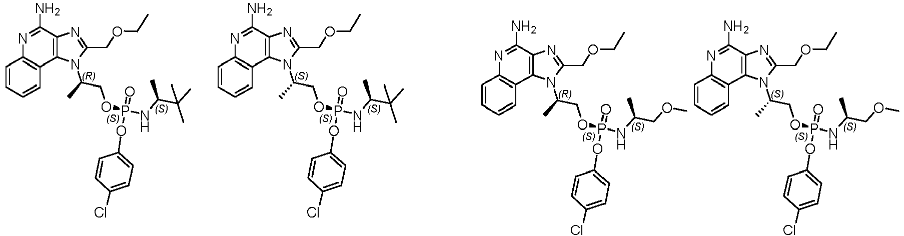

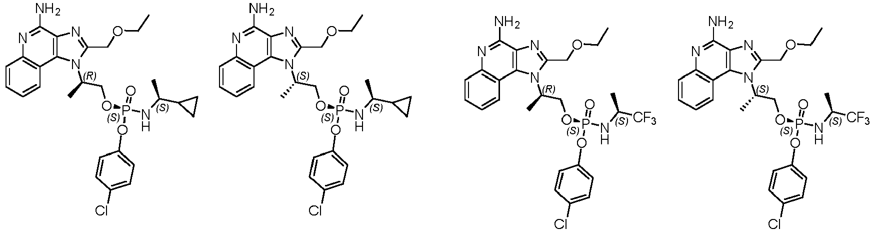

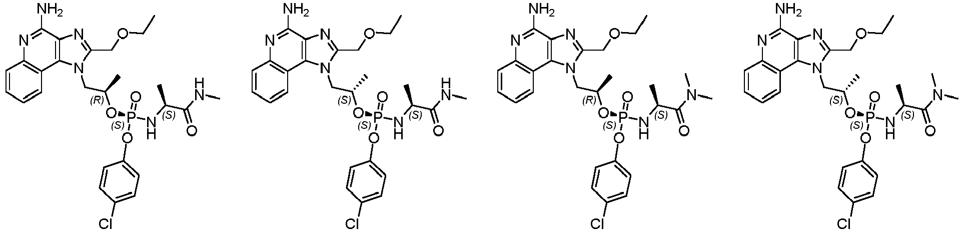

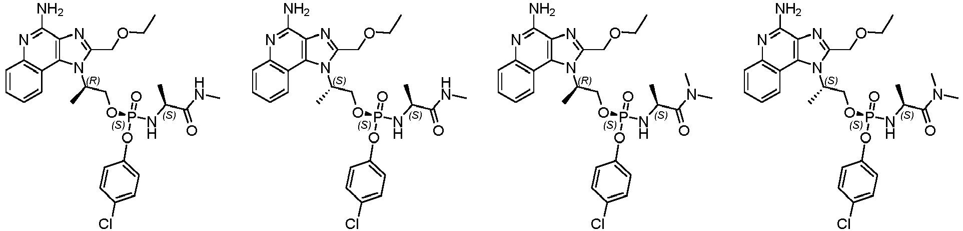

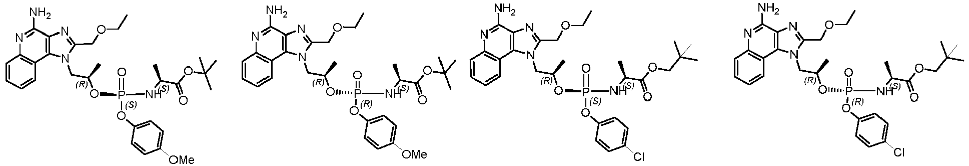

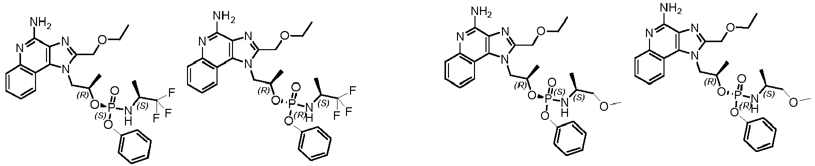

The salts that may be formed by the compounds of the present invention are also within the scope of the present invention. Unless otherwise indicated, the compounds of the present invention are understood to include salts thereof. The term "salt" as used herein refers to an acid or basic salt formed from an inorganic or organic acid and a base. In addition, when a compound in the present invention contains a basic fragment, which includes but is not limited to pyridine or imidazole, and when it contains an acidic fragment, which includes but is not limited to a carboxylic acid, zwitterion (internal salt) that may be formed is included within the scope of the term salt. Pharmaceutically acceptable (i.e. non-toxic, physiologically acceptable) salts are preferred, although other salts are also useful, such as used in separation or purification steps in the preparation process. The compounds of the present invention may form salts, for example, compound I is reacted with an equivalent amount of acid or base, salted out in a medium, or freeze-dried in an aqueous solution.

The compounds of the present invention contain basic fragments, including but not limited to amine or pyridine or imidazole ring, which may form salts with organic or inorganic acids. Typical acids that can form salts include acetate (such as acetic acid or trihalogenated acetic acid, such as trifluoroacetic acid), adipate, alginate, ascorbic acid salt, aspartic acid salt, benzoic acid salt, benzenesulfonate, bisulfate, borate, butyrate, citrate, camphor salt, camphor sulfonate, cyclopentane propionate, diethylene glycol salt, dodecyl sulfate, ethane sulfonate, fumarate, gluconate, glycerophosphate, hemisulfate, heptanoate, caproate, hydrochloride, hydrobromide, hydroiodide, isethionate (e.g. 2-hydroxyethanesulfonate), lactate, maleate, methanesulfonate, naphthalene sulfonate (e.g. 2-naphthalenesulfonate), nicotinate, nitrate, oxalate, pectate, persulfate, phenylpropanate (e.g. 3-phenylpropanate), phosphate, picric acid salt, pivalate, propionate, salicylate, succinate, sulfate (such as formed with sulfuric acid), sulfonate, tartrate, thiocyanate, toluene sulfonate such as p-toluene sulfonate, dodecanoate, etc.

Certain compounds of the present invention may contain acidic fragments, including but not limited to carboxylic acids, that may form salts with various organic or inorganic bases. Typical alkali-formed salts include ammonium salts, alkali metal salts such as sodium, lithium, and potassium salts, alkaline earth metal salts such as calcium and magnesium salts, and organic alkali-formed salts (such as organic amines), such as benzathine, bicyclohexylamine, hydrabamine (a salt formed with *N,N*-bis(dehydroabietyl)ethylenediamine), *N*-methyl-D-glucosamine, *N*-methyl-D-glucosamide, tert-butyl amine, and salts formed with amino acids such as arginine and lysine. Basic nitrogen-containing groups can be combined with halide quaternary ammonium salts, such as small molecule alkyl halides (such as methyl, ethyl, propyl and butyl chlorides, bromides and iodides), dialkyl sulfates (such as dimethyl, diethyl, dibutyl and diamyl sulfate), long-chain halides (such as decyl, dodecyl, tetradecyl and tetradecyl chlorides, bromides and iodides), aralkyl halides (such as benzyl and phenyl bromides) and the like.

The prodrugs and solvates of the compounds of the present invention are also within the scope of the present invention. The term "prodrug" herein refers to a compound that, in the treatment of a related disease, undergoes a chemical transformation of a metabolic or chemical process to produce a compound, salt, or solvate of the present invention. The compounds of the present invention include solvates, such as hydrates.

The compound, salt or solvate of the present invention may be in a tautomeric form (e. g. amide and imine ether). All these tautomers are part of the present invention.

The stereoisomers of all compounds (e.g., those asymmetric carbon atoms that may exist due to various substitutions), including their enantiomeric and diastereomeric forms, fall within the scope of the present invention. The independent stereoisomer of the compound of the present invention may not be present with other isomers (e.g., as a pure or substantially pure optical isomer with special activity) at the same time, or may also be a mixture, such as a racemate, or a mixture formed with all other stereoisomers or a part thereof. The chiral center of the present invention has two configurations, S or R, and is defined and suggested by the International Union of Pure and Applied Chemistry (IUPAC) in 1974. The racemic form can be solved by physical methods, such as fractional crystallization, or separation of crystallization by derivatization of diastereoisomers, or separation by chiral column chromatography. Individual optical isomers can be obtained from racemates by suitable methods, including but not limited to conventional methods, such as salt-forming with an optically active acid and recrystallization.

The compound of the present invention, which is obtained sequentially by preparation, separation and purification, has a weight content equal to or greater than 90%, for example, equal to or greater than 95%, equal to or greater than 99% (very pure compounds), listed in the text description. Such "very pure" compounds of the invention herein are also intended to be part of the invention.

All configuration isomers of the compounds of the present invention are within the scope of coverage, whether in mixture, pure or very pure form. The compounds of the present invention are defined to include two olefin isomers, cis (Z) and trans (E), as well as cis and trans isomers of carbocyclic and heterocyclyl rings.

Throughout the specification, groups and substituents can be selected to provide stable fragments and compounds.

Specific functional groups and definitions of chemical terms are described in detail below. For the present invention, the chemical element is consistent with that as defined in Periodic Table of the Elements, CAS version, *Handbook of Chemistry and Physics*, 75^{th} Ed. The definition of a specific functional group is also described therein. In addition, basic principles of organic chemistry and specific functional groups and reactivities are described in "Organic Chemistry", Thomas Sorrell, University Science Books, Sausalito: 1999, which is incorporated by reference in its entirety.

Certain compounds of the present invention may be present in specific geometric or stereoisomeric forms. The invention covers all compounds, including their cis and trans isomers, R and S enantiomers, diastereomers, (D) isomers, (L) isomers, racemic mixtures and other mixtures. In addition, asymmetric carbon atoms can represent substituents, such as alkyl. All isomers and their mixtures are included in the present invention.

According to the present invention, a mixture of isomers can contain isomers in various ratios. For example, a mixture of only two isomers can have the following combinations: 50:50, 60:40, 70:30, 80:20, 90:10, 95:5, 96:4, 97:3, 98:2, 99:1, or 100:0. All ratios of isomers are within the scope of the present invention. Similar ratios, and ratios for mixtures of more complex isomers, as readily understood by those skilled in the art, are also within the scope of the present invention.

The invention also includes is otopically labeled compounds, equivalent to the original compounds disclosed herein. But in fact, it usually appears that one or more atoms are replaced by atoms with different atomic weights or mass numbers. Examples that can be listed as isotopes of compounds of the present invention include hydrogen, carbon, nitrogen, oxygen, phosphorus, sulfur, fluorine and chlorine isotopes such as ²H, ³H, ¹³C, ¹¹C, ¹⁴C, ¹⁵N, ¹⁸O, ¹⁷O, ³¹P, ³²P, ³⁵S, ¹⁸F and ³⁶Cl, respectively. The compounds of the present invention, or enantiomers, diastereomers, isomers, or pharmaceutically acceptable salts or solvates, and the above compounds containing isotopes or other isotopic atoms are within the scope of the present invention. Certain otopically labeled compounds in the present invention, such as ³H and ¹⁴C's radioisotopes are also among them, and are useful in drug and substrate tissue distribution experiments. Tritium, i.e.³H and carbon-14, i.e.¹⁴C, are relatively easy to be prepared and detected. They are the first choice among isotopes. In addition, heavier isotope substitutions such as deuterium, i.e. ²H, due to its good metabolic stability, have advantages in some therapies, such as increasing half-life or reducing dosage in the body, so it can be preferred in some cases. Isotope-labeled compounds can be prepared by general methods, by replacing non-isotopic reagents with readily available isotope-labeled reagents, and by means of the scheme disclosed in the example.

If the synthesis of a specific enantiomer of a compound of the present invention is to be designed, it can be prepared by asymmetric synthesis, or derivatized with a chiral auxiliary, separating the resulting diastereomeric mixture, and removing the chiral auxiliary to obtain a pure enantiomer. In addition, if the molecule contains a basic functional group, such as an amino acid, or an acidic functional group, such as a carboxyl, a suitable optically active acid or base can be used to form a diastereomer salt with it, and then it is separated by conventional means such as separation and crystallization or chromatography, and then a pure enantiomer is obtained.

As described herein, the compounds in the present invention can be substituted with any number of substituents or functional groups to expand its scope. Generally, for the term "substituted", whether it appears before or after the term "optional", the general formula including substituents in the formulation of the present invention refers to substituting hydrogen radicals with specified substituents. When multiple positions in a particular structure are replaced by multiple specific substituents , each of the substituents may be the same or different. The term "substituted" as used herein includes all permissible organic compound substitution. Broadly speaking, permissible substituents include non-cyclic, cyclic, branched, non-branched, carbocyclic and heterocyclyl, aromatic and non-aromatic ring organic compounds. In the present invention, for example, heteroatomic nitrogen, its valence state may be supplemented by a hydrogen substituent or by any permitted organic compound described above. In addition, the present invention is not intended to limit the permissible substitution of organic compounds in any way. The present invention considers the combination of substituents and variable groups to be very good in the treatment of diseases (such as infectious diseases or proliferative diseases) in the form of stable compounds. The term "stable" herein refers to a compound that is stable enough to be detected over a long enough period of time to maintain the structural integrity of the compound, preferably effective over a long enough period of time. This article is used for the above purposes.

The metabolites of the compounds and their pharmaceutically acceptable salts involved in this application, as well as prodrugs that can be transformed into the structure of the compounds and their pharmaceutically acceptable salts involved in this application in vivo, are also included in the claims of this application.

### PREPARATION METHOD

The processes for the preparation of the compounds of formula (I) of the present invention will be described in more detail below, but these specific processes do not pose any limitations to the present invention. The compounds of the present invention may also be conveniently prepared by optionally combining various synthetic methods described in the specification or known in the art, and such combinations are readily made by those skilled in the art to which the present invention pertains.

Typically, the preparation process of the compounds of the present invention is as follows, wherein the starting materials and reagents used are commercially available unless otherwise specified.

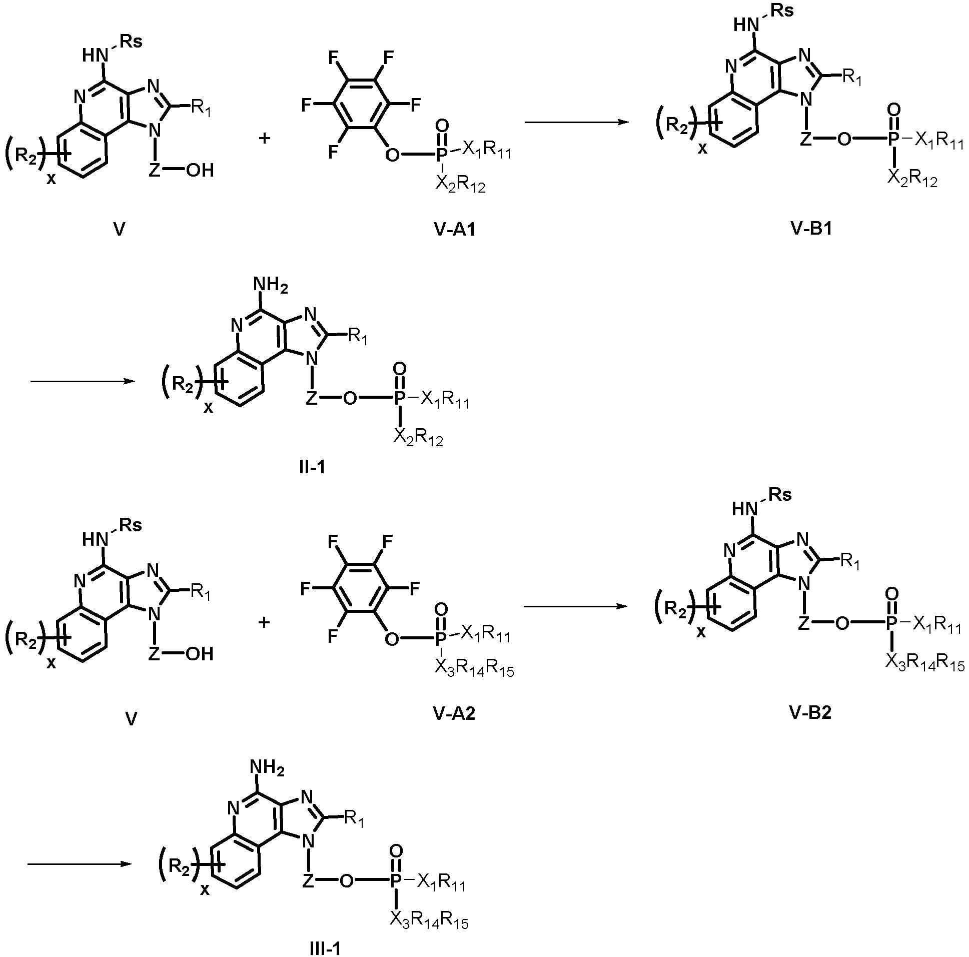

The compound of the general formula (V-A1) or (V-A2) and the compound of the general formula (V) are coupled to obtain the intermediate (V-B1) or (V-B2), and then the protective group Rs is removed to obtain the target compound of the general formula (II-1) or (III-1). Wherein, R₁, R₂, x, Z, X₁, X₂, X₃, R₁₁, R₁₂, R₁₄, and R₁₅ are as described above. Rs is the protective group of the amino.

### Pharmaceutical composition and method for administration

The pharmaceutical composition of the present invention is used to prevent and/or treat the following diseases: inflammation, cancer, cardiovascular disease, infection, immune disease, metabolic disease.

The compound of general formula (I) may be used in combination with other drugs known to treat or improve similar conditions. When administered in combination, the original drug administration mode and dosage can remain unchanged, while the compound of formula I is administered at the same time or subsequently. When the compound of formula I is administered simultaneously with one or more other drugs, a pharmaceutical composition containing one or more known drugs and the compound of formula I at the same time may be preferably used. Drug combination also includes administration of the compound of formula I with one or more other known drugs in overlapping time periods. When a compound of formula I is administered with one or more other drugs, the compound of formula I or known drugs may be administered at a lower dose than they are administered alone.

Drugs or active ingredients that may be used in combination with the compound of general formula (I)include, but are not limited: interferon α (standard INFα and polyethanolated INFα), nucleoside drugs (such as Telbivudine, Lamivudine, Clevudine, Adefovir, Tenofovir, Besifovir, Tenofovir Disoproxil Fumarate (TDF), Tenofovir Alafenamide Fumarate (TAF) and HDP-PMPA (CMX157), etc.), shell protein allosteric regulators (such as BAY41-4109, RG-7907, NVR 3-778, ABI-H0731, ABI-H2158, JNJ-56136379, GLS 4JHS, etc.), cccDNA inhibitor, TLR3/7/8/9 agonists (such as RG-7854, GS9620, etc.), hepatitis B virus entry inhibitors (such as Myrcludex B, etc.), interfering nucleotides (such as ARB 1467, ARB 1740, etc.), HBV surface antigen inhibitors (such as RG7834, REP2139, REP2165, etc.),CRISPER/Cas9, PD-1 inhibitors (nivolumab, pembrolizumab, pidilizumab, cemiplimab, JS-001, SHR-120, BGB-A317, IBI-308, GLS-010, GB-226,STW204, HX008, HLX10, BAT 1306, AK105, LZM 009 or biosimilars of the above drugs), PD-L1 inhibitors (durvalumab, atezolizumab, avelumab, CS1001, KN035, HLX20, SHR-1316, BGB-A333, JS003, CS1003, KL -A167, F 520, GR1405, MSB2311 or biosimilars of the above drugs), CD20 antibodies (such as rituximab, obinutuzumab, ofatumumab, veltuzumab, tositumomab, ¹³¹I- tositumomab, ibritumomab, ⁹⁰Y- ibritumomab, ⁹⁰In- ibritumomab, ibritumomab tiuxetan), CD47 antibodies (Hu5F9-G4, CC-90002, TTI-621, TTI-622, OSE-172, SRF-231, ALX-148, NI-1701, SHR-1603, IBI188, IMM01), etc.

The dosage form of the pharmaceutical composition of the present invention includes (but is not limited to):injection, tablet, capsule, aerosol, suppository, film, drop pill, topical liniment, controlled release or sustained release or nano-preparation.

The pharmaceutical composition of the present invention comprises a safe and effective amount of a compound of the present invention or a pharmacologically acceptable salt thereof, and a pharmacologically acceptable excipient or carrier. In which, "safe and effective amount" is meant that the amount of the compound is sufficient to significantly improve the condition without causing serious side effects. Generally, the pharmaceutical composition contains 1-2000 mg of the compound of the present invention/dose, more preferably, 10-1000 mg of the compound of the present invention/dose. Preferably, the "dose" is a capsule or tablet.

"Pharmaceutically acceptable carrier" means one or more compatible solid or liquid fillers or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agent (such as sodium dodecyl sulfate), coloring agents, flavoring agents, stabilizers, antioxidants, preservatives, pyrogen-free water, etc..

The administration mode of the compound or pharmaceutical composition of the present invention is not particularly limited, and representative administration modes include, but are not limited to, oral, intratumoral, rectal, parenteral (intravenous, intramuscular or subcutaneous) and topical administration.

Solid dosage forms for oral administration include capsules, tablets, pills, powders and granules. In these solid dosage forms, the active ingredient is mixed with at least one conventional inert excipient (or carrier), such as sodium citrate or dicalcium phosphate, or mixed with any of the following components: (a) fillers or compatibilizer, such as starch, lactose, sucrose, glucose, mannitol and silicic acid; (b) binders, such as hydroxymethyl cellulose, alginate, gelatin, polyvinylpyrrolidone, sucrose and arabic gum; (c) humectants, such as glycerol; (d) disintegrating agents such as agar, calcium carbonate, potato starch or tapioca starch, alginic acid, certain composite silicates, and sodium carbonate; (e) retarding solvents, such as wax, (f) absorption accelerators, such as quaternary ammonium compound; (g) wetting agents, such as cetyl alcohol and glyceryl monostearate; (h) adsorbents, such as kaolin; and (i) lubricants, such as talc, calcium stearate, magnesium stearate, solid polyethylene glycol, sodium dodecyl sulfate or mixture thereof. In capsules, tablets and pills, the dosage forms may also contain buffering agents.

Solid dosage forms such as tablets, dragees, capsules, pills and granules can be prepared with coatings and shells such as enteric coatings and other materials known in the art. They may contain opacifying agents and the release of the active compound or compound in such compositions may be released in a portion of the digestive tract in a delayed manner. Examples of embedding components that can be employed are polymeric materials and waxy materials. If necessary, the active compound may also be in microencapsulated form with one or more of the above-mentioned excipients.

Liquid dosage forms for oral administration include pharmaceutically acceptable emulsions, solutions, suspensions, syrups or tinctures. In addition to the active compound, the liquid dosage form may contain inert diluents conventionally used in the art, such as water or other solvents, solubilizers and emulsifiers, such as ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures of these substances.

In addition to these inert diluents, the compositions may contain adjuvants such as wetting agents, emulsifying and suspending agents, sweetening agents, flavoring agents and spices.

In addition to the active compound, the suspension may contain suspending agent, such as ethoxylated isooctadecanol, polyoxyethylene sorbitol and dehydrated sorbitan ester, microcrystalline cellulose, aluminum methoxide and agar, or the mixture thereof etc..

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

Dosage forms for the compounds of the invention for topical administration include ointments, powders, patches, aerosols and inhalants. The active ingredient is mixed under sterile conditions with a physiologically acceptable carrier and any preservatives, buffers, or propellants which may be required if necessary.

The treatment method of the present invention can be administered alone or in combination with other treatment means or therapeutic drugs.

When the pharmaceutical composition is used, a safe and effective amount of the compound of the present invention is administered to a mammal (such as a human) in need of treatment, wherein the dosage at the time of administration is the pharmaceutically effective dosage, for people having a body weight of 60kg, the daily dose is usually 1∼2000mg, preferably 50∼1000mg. Of course, specific doses should also consider factors such as the administration route, the health of the patient, etc., which are within the skill of the skilled physician.

The present invention also provides a method for preparing a pharmaceutical composition, which comprises the step of: mixing a pharmaceutically acceptable carrier with the compound of general formula (I) or its crystal form, pharmaceutically acceptable salt, hydrate or solvate of the present invention, so as to form a pharmaceutical composition.

The present invention also provides a method for treatment comprising the step of administering to a subject in need thereof a compound of general formula (I) of the present invention, or a crystal form, pharmaceutically acceptable salt, hydrate or solvate thereof, or administering a pharmaceutical composition of the present invention for selectively agonizing TLR7and/or TLR8.

Compared with the prior art, the present invention has the following main advantages:
(1) the compound has a selective agonistic effect on TLR7 and TLR8;
(2) the compound has a good selective agonistic effect on TLR8;
(3) the compound has better pharmacodynamic performance and lower toxic and side effects.

The present invention will be further illustrated below with reference to the specific examples. It should be understood that these examples are only to illustrate the invention but not to limit the scope of the invention. Experimental methods in which the specific conditions are not specified in the following examples are usually in accordance with conventional conditions such as the conditions described in Sambrook et al., Molecular Cloning: Laboratory Manual (New York: Cold Spring Harbor Laboratory Press, 1989), or in accordance with the conditions recommended by the manufacturer. Unless indicated otherwise, percentage and parts are calculated by weight.

Unless otherwise defined, all professional and scientific terminology used in the text have the same meanings as known to the skilled in the art. In addition, any methods and materials similar or equal with the recorded content can apply to the methods of the invention. The method of the preferred embodiment described herein and the material are only for demonstration purposes.

The structure of the compound of the present invention is determined by nuclear magnetic resonance (NMR) and Liquid Chromatography-Mass Spectrometry (LC-MS).

NMR is detected by Bruker AVANCE-400 nuclear magnetic resonance instrument. The determination solvent includes deuterated dimethyl sulfoxide (DMSO-d₆), deuterated acetone (CD₃COCD₃), deuterated chloroform (CDCl₃) and deuterated methanol (CD₃OD). The internal standard is tetramethylsilane (TMS), and the chemical shift is measured in units of one millionth (ppm).

Liquid Chromatography-Mass Spectrometry (LC-MS) is detected using Waters SQD2 mass spectrometer. HPLC is determined using a Agilent 1100 high pressure chromatograph (Microsorb 5 micron C18 100 x 3.0mm column).

Qingdao GF254 silica gel plate is used as thin layer chromatography silica gel plate, 0.15-0.20mm is used for TLC, and 0.4 mm-0.5mm is used for preparative thin layer chromatography. Column chromatography generally uses 200-300 mesh silica gel (Qingdao silica gel) as the carrier.

The starting materials in the examples of the present invention are known and commercially available, or can be synthesized using or in accordance with the literature reported in the art.

Unless otherwise specified, all reactions of the present invention are carried out by continuous magnetic stirring under the protection of dry inert gas (such as nitrogen or argon), and the reaction temperature is degrees Celsius.

### Example 1

### Preparation of 1-(4-amino-2-(ethoxymethyl)-1H-imidazo[4,5-C]quinolin-1-yl-2-propanol

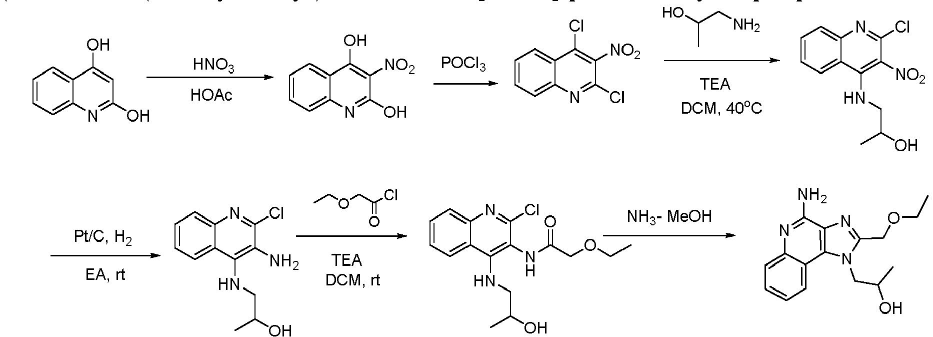

### Step 1: Preparation of 3-nitro-2,4-dihydroxyquinoline

Nitric acid (35 mL) was slowly added dropwise to a uniformly stirred solution of 2, 4-dihydroxyquinoline (23g, 142.7 mmol) in acetic acid (140 mL) at room temperature. After dropping, the reaction solution was reacted at 105°C for 2 hours, then cooled to room temperature, and then quenched with water (30 mL). The obtained mixture was stirred for 15 min and then filtered. The filter cake was washed with water and then dried in vacuum to obtain the target product (17.5g, yield 59%).

LC-MS:m/z 207 (M + H)⁺.

### Step 2: Preparation of 3-nitro-2,4-dichloroquinoline

Triethylamine (8.6g, 84.9 mmol) was slowly added dropwise to a solution of -nitro-2, 4-dihydroxyquinoline (17.5g, 84.9 mmol) in phosphorus oxychloride (60 mL) at room temperature. After dropping, the reaction solution was reacted at 120 °C for 2 hours and then cooled to room temperature, and then concentrated under reduced pressure. The obtained residue was poured into ice water (100 mL), pH was adjusted to 7-8 with saturated sodium bicarbonate aqueous solution, and then extracted with dichloromethane (100 mLx3). The combined organic phase was dried over anhydrous magnesium sulfate and filtered. The filtrate was concentrated under reduced pressure, and the obtained crude product was purified by column chromatography to obtain the target product (18g, yield 87%).

LC-MS:m/z 243 (M + H)⁺.

### Step 3: Preparation of 1-((2-chloro-3-nitroquinolin-4-yl)amino)-2-propanol

A solution of 3-nitro-2, 4-dichloroquinoline (10g, 41.14 mmol), triethylamine (5g, 49.37 mmol) and isopropanolamine (3.79g, 50.44 mmol) in dichloromethane (70 mL) was reacted at 46 °C for 21 hours, and then petroleum ether (60mL) was added. The resulting mixture was naturally cooled to precipitate solids and then filtered. The filter cake was collected to obtain the target product (14.3g, quantitative yield).

LC-MS:m/z 282 (M + H)⁺.

### Step 4: Preparation of

### 1-((2-chloro-3-aminoquinolin-4-yl)amino)-2-propanol

A mixed suspension of 1-(2-chloro-3-nitroquinolin-4-yl) amino)-2-propanol (15g, 53.25mmol) and platinum carbon (10% wt, 3.2g) in ethyl acetate (300 mL) and methanol (100 mL) was reacted under hydrogen (15 psi) atmosphere for 4 hours at room temperature, and then filtered through diatomite. The obtained filtrate was concentrated under reduced pressure to obtain the target product (14.7g), which was directly used for the next reaction without purification.

LC-MS:m/z 252 (M + H)⁺.

### Step 5: Preparation of N-(2-chloro-4-((2-hydroxy)-propyl)aminoquinolin-3-yl)-2-ethoxyacetamide

A solution of ethoxyacetyl chloride (15g, 122.36 mmol, 2.2 eq) in methylene chloride (30 mL) was added dropwise to a solution of 1-((2-chloro-3-aminoquinolin-4-yl) amino)-2-propanol (14g, 55.62 mmol, 1.0 eq) and triethylamine (9g, 90 mmol, 1.6 eq) in methylene chloride (150 mL) at room temperature. After dropping, the reaction solution was reacted at 25°C for 3 hours and then washed with water (50 mL). The organic phase was dried over anhydrous magnesium sulfate and filtered. The filtrate was concentrated under reduced pressure, and the obtained crude product was purified by column chromatography to obtain the target product (5.3g, yield 28.2%).

LC-MS:m/z 338 (M + H)⁺.

### Step 6: Preparation of

### 1-(4-amino-2-(ethoxymethyl)-1H-imidazo[4,5-C]quinolin-1-yl)-2-propanol

A solution of N-(2-chloro-4-((2-hydroxy)-propyl) aminoquinolin-3-yl)-2-ethoxyacetamide (2.7g, 8 mmol, 1.0 eq) in ammonia-methanol (7M, 25 mL, 175 mmol) in a closed container was reacted at 180°C for 12 hours and then cooled to room temperature. The obtained mixture was concentrated under reduced pressure, and the obtained crude product was purified by column chromatography to obtain the target product (1.5g, yield 62%).

LC-MS:m/z 301 (M + H)⁺. ¹H NMR (400 MHz, CDCl₃) δ 7.82 (d, *J* =12Hz, 1 H), 7.63 (d, *J* =12Hz, 1 H),7.41 (t, J=8Hz, 1 H), 7.25-7.22 (m, 1 H), 5.42 (s, 2 H), 4.93 (d, *J* =12Hz, 1 H), 4.68 (d, *J* =12Hz, 1 H), 4.61-4.57 (m, 1 H), 4.52-4.46 (m, 2 H), 3.67-3.59 (m, 2 H), 1.48 (d, *J* =8Hz, 3 H), 1.24 (t, *J* =6Hz, 3 H).

### Examples 1A and 1B preparation of (S)-1-(4-amino-2-(ethoxymethyl)-1H-imidazo[4, 5-C]quinolin-1-yl-2-propanol and (R)-1-(4-amino-2-(ethoxymethyl)-1H-imidazo[4,5-C]quinolin-1-yl-2-propanol

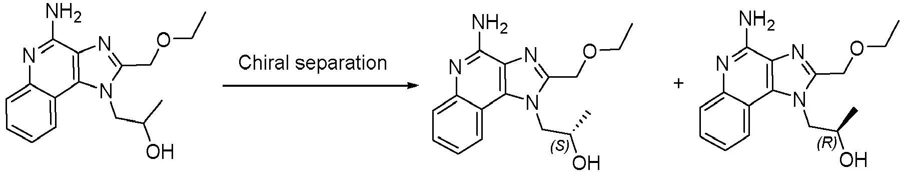

Example 1 was separated by chiral HPLC (chiral column: Cellulose-SC 4.6 ^{∗} 100mm 5 µm; mobile phase: MeOH(0.2% Methanol Ammonia)) and two isomers were obtained.

### Example 1A (isomer A, (S)-1-(4-amino-2-(ethoxymethyl)-1H-imidazo[4,5-C]quinolin-1-yl-2-propanol):

**RT (retention time) = 2.05 min.** LC-MS:m/z 301 (M + H)⁺. ¹H NMR (400 MHz, CDCl₃) δ 7.82 (d, *J* =12Hz, 1 H), 7.63 (d, *J* =12Hz, 1 H),7.41 (t, *J* =8Hz, 1 H), 7.25-7.22 (m, 1 H), 5.42 (s, 2 H), 4.93 (d, *J* =12Hz, 1 H), 4.68 (d, *J* =12Hz, 1 H), 4.61-4.57 (m, 1 H), 4.52-4.46 (m, 2 H), 3.67-3.59 (m, 2 H), 1.48 (d, J=8Hz, 3 H), 1.24 (t, *J* =6Hz, 3 H).

### Example 1B (isomer B, (R)-1-(4-amino-2-(ethoxymethyl)-1H-imidazo[4,5-C]quinolin-1-yl)-2-propanol):

**RT (retention time) = 3.19 min.** LC-MS:m/z 301 (M + H)⁺. ¹H NMR (400 MHz, CDCl₃) δ 7.82 (d, *J* =12Hz, 1 H), 7.63 (d, *J* =12Hz, 1 H),7.41 (t, *J* =8Hz, 1 H), 7.25-7.22 (m, 1 H), 5.42 (s, 2 H), 4.93 (d, *J* =12Hz, 1 H), 4.68 (d, *J* =12Hz, 1 H), 4.61-4.57 (m, 1 H), 4.52-4.46 (m, 2 H), 3.67-3.59 (m, 2 H), 1.48 (d, J=8Hz, 3 H), 1.24 (t, *J* =6Hz, 3 H).

### Examples 2A and 2B preparation of isopropyl ((S)-(((R)-1-(4-amino -2-(ethoxymethyl)-1H-imidazo[4,5-c] quinolin-1-yl) propan-2-yl) oxy) (phenoxy) phosphoryl)-L-alaninate and isopropyl ((S)-(((S)-1-(4-amino -2-(ethoxymethyl)-1H-imidazo [4, 5-c] quinolin-1-yl) propan-2-yl) oxy )(phenoxy) phosphoryl)-L-alaninate

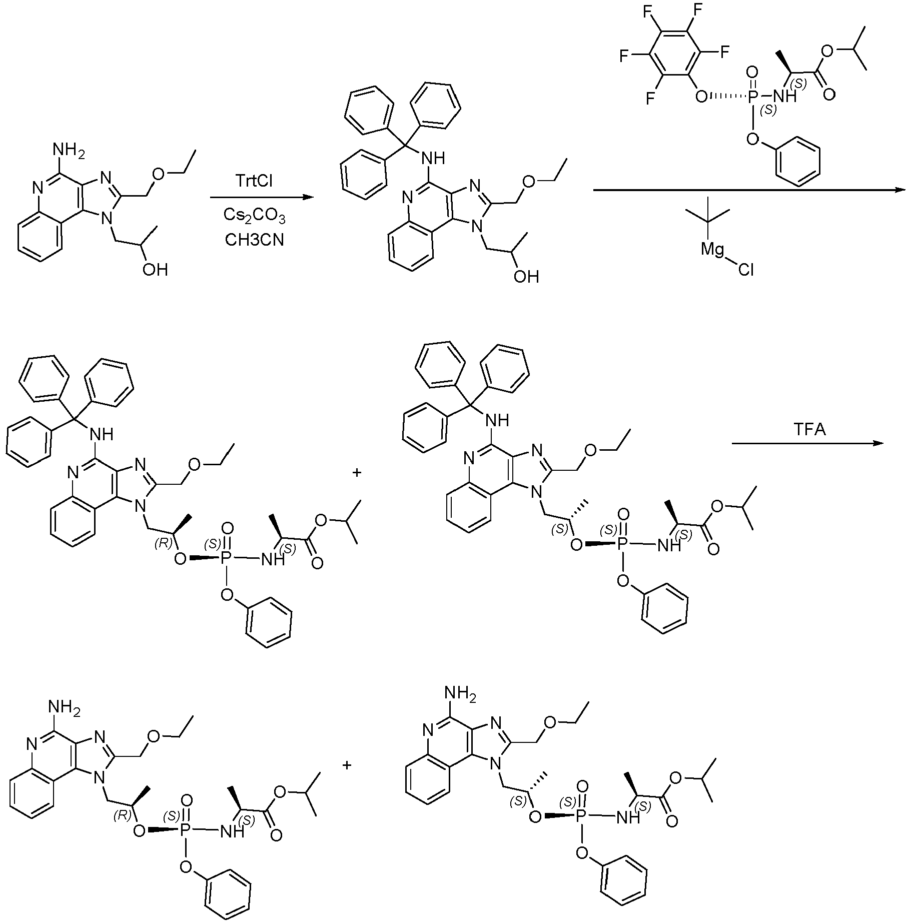

### Step 1: Preparation of 1-(2-(ethoxymethyl)-4-(triphenylmethylamino)-1H-imidazo [4,5-C] quinolin-1-yl)-2-propanol

A solution of 1-(4-amino-2-(ethoxymethyl)-1H-imidazo [4,5-C] quinolin-1-yl)-2-propanol (250 mg, 0.83 mmol), triphenylchloromethane (325 mg, 1.17 mmol) and cesium carbonate (1g, 3.33 mmol) in acetonitrile (20 mL) was reacted at 140°C for 8 hours and then reacted at 100°C for 16 hours. The resulting mixture was cooled to room temperature and then concentrated under reduced pressure. The obtained crude product was purified by column chromatography to obtain the target product (340 mg, yield 75%).

LC-MS:m/z 543 (M + H)⁺.

### Step 2: preparation of isopropyl ((S)-(((R)-1-(2-(ethoxymethyl)-4-(triphenylmethylamino)-1H-imidazo[4,5-c] quinolin-1-yl) propan-2-yl) oxy)(phenoxy) phosphoryl)-L-alaninate and isopropyl ((S)-(((S)-1-(2-(ethoxymethyl)- 4-(triphenylmethylamino)-1H-imidazo[4, 5-c] quinolin-1-yl) propan-2-yl) oxy) (phenoxy) phosphoryl)-L-alaninate

A solution of tert-butyl magnesium chloride in tetrahydrofuran (1M, 0.7 mL,0.7 mmol) was added dropwise to 1-(2-(ethoxymethyl)-4-(triphenylmethylamino)-1H-imidazo [4,5-C] quinolin-1-yl)-2-propanol (180 mg, 0.33 mmol) in tetrahydrofuran (6 mL, 33.3vol) at -7°C under nitrogen protection. After dropping, the reaction solution was reacted at -7°C for 1.5h and then a solution of isopropyl ((S)-(perfluorophenoxy) (phenoxy) phosphonyl)-L-alaninate (230 mg,0.51 mmol) in tetrahydrofuran (2 mL) was added dropwise. The obtained mixture was reacted at -7°C for 7 hours, then diluted hydrochloric acid (1 N) was added dropwise to adjust the pH to 4, and then extracted with ethyl acetate. The combined organic phase was dried over anhydrous magnesium sulfate and filtered. The filtrate was concentrated under reduced pressure, and the obtained crude product was purified by column chromatography to obtain a mixture of isopropyl ((S)-(((R)-1-(2-(ethoxymethyl)- 4-(triphenylmethylamino)-1H-imidazo [4,5-c] quinolin-1-yl) propan-2-yl) oxy) (phenoxy) phosphoryl)-L-alaninate and isopropyl ((S)-(((S)-1-(2-(ethoxymethyl)- 4-(triphenylmethylamino)-1H-imidazo[4, 5-c] quinolin-1-yl) propan-2-yl) oxy) (phenoxy) phosphoryl)-L-alaninate (1:1, 165 mg).

LC-MS:m/z 813 (M + H)⁺.

### Step 3: preparation of isopropyl ((S)-(((R)-1-(4-amino -2-(ethoxymethyl)-1H-imidazo [4,5-c] quinolin-1-yl) propan-2-yl) oxy )(phenoxy) phosphoryl)-L-alaninate and isopropyl ((S)-(((S)-1-(4-amino -2-(ethoxymethyl)-1H-imidazo [4, 5-c] quinolin-1-yl) propan-2-yl) oxy) (phenoxy) phosphoryl)-L-alaninate

Trifluoroacetic acid (2 mL) was added dropwise to a mixture of isopropyl ((S)-(((S)-1-(2-(ethoxymethyl)- 4-(triphenylmethylamino)-1H-imidazo[4,5-c] quinolin-1-yl) propyl-2-yl) oxy) (phenoxy) phosphoryl)-L-alaninate (1:1, 80 mg, 0.1 mmol) in dichloromethane (6 mL, 75) at 0°C. After dropping, the reaction solution was reacted at 0 °C for 1 hour, then reacted at room temperature for 3 hours, and then concentrated under reduced pressure. The residue was purified by preparative chromatography to obtain trifluoroacetate of two isomers isopropyl ((S)-(((R)-1-(4-amino -2-(ethoxymethyl)-1H-imidazo [4,5-c] quinolin-1-yl) propan-2-yl) oxy) (phenoxy) phosphoryl)-L-alaninate and isopropyl ((S)-(((S)-1-(4-amino-2-(ethoxymethyl)-1H-imidazo [4, 5-c] quinolin-1-yl) propan-2-yl) oxy) (phenoxy) phosphoryl)-L-alaninate.

**Example 2A (Isomer A)**:LC-MS:m/z 570 (M+H)⁺. ¹H NMR (400 MHz, CDCl₃) δ 11.08 (s, 1 H), 8.00 (d, J=8Hz, 1 H), 7.95 (d, J=8Hz, 1 H), 7.62 (t, J=8Hz, 1 H), 7.46 (t, *J* =8Hz, 1 H), 7.28 (s, 1 H), 7.24 (s, 1 H), 7.12 (t, *J* =8Hz, 1 H), 7.00 (d, *J* =8Hz, 2 H), 6.69 (s, 1 H), 5.05 (s, 1 H), 4.92-4.85 (m, 3 H), 4.76-4.67 (m, 2 H), 3.63 (q, J=8Hz, 2 H), 3.54 (q, J=8Hz, 1 H), 3.06 (t, *J* =12Hz, 1 H), 1.56 (d, J=4Hz, 3 H), 1.27-1.24 (m, 4 H), 1.17-1.15 (m, 6 H), 0.88 (d, J=8Hz, 3 H). ³¹P NMR (162 MHz, CDCl₃) δ 1.90 (s,1P).

**Example 2B (Isomer B)**:LC-MS:m/z 570 (M+H)⁺. ¹H NMR (400 MHz, CDCl₃) δ 10.99 (s,1H), 8.00 (d, *J* =8 Hz, 1H), 7.93 (d, *J* = 8Hz, 1H), 7.62 (t, *J* =8 Hz, 1H), 7.47 (t, *J* =8 Hz, 1H), 7.07-7.02 (m, 3 H), 6.59 (d, *J* =8 Hz, 2 H), 6.55 (s, 1 H), 5.15 (s, 1 H), 5.03-4.97 (m, 1 H), 4.87-4.81 (m, 2 H), 4.71-4.62 (m, 2 H), 3.73 (q, J=8 Hz,1H), 3.59 (q, *J* =8 Hz, 2 H), 3.49 (t, *J* =10 Hz,1 H), 1.61 (d, *J* =4 Hz, 3 H), 1.27-1.22 (m, 13 H). ³¹P NMR (162 MHz, CDCl₃) δ 1.33 (s,1P).

### The following examples were synthesized with different starting materials in the same way:

### Examples 3A and 3B isopropyl ((R)-(((R)-1-(4-amino-2-(ethoxymethyl)-1H-imidazo [4,5-c] quinolin-1-yl) propan-2-yl) oxy) (phenoxy) phosphoryl)-L-alaninate and isopropyl ((R)-(((S)-1-(4-amino-2-(ethoxymethyl)-1H-imidazo [4,5-c] quinolin-1-yl) propan-2-yl) oxy) (phenoxy) phosphoryl)-L-alaninate

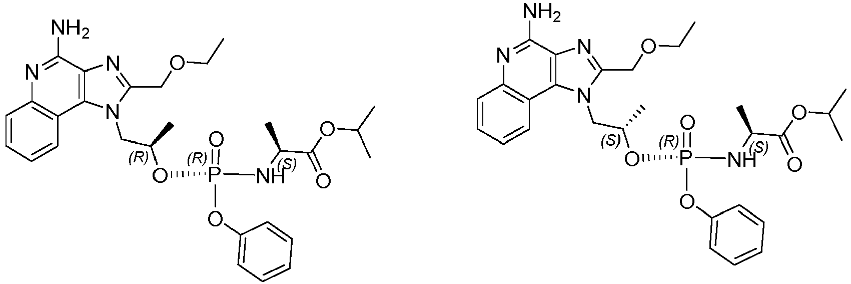

**Example 3A (Isomer A)**:LC-MS:m/z 570 (M+H)⁺. ¹H NMR (400 MHz, CDCl₃) δ 11.05(s, 1H), 8.01 (d, J=8Hz, 1H), 7.96 (d, J=8Hz, 1H), 7.62 (t, J=8Hz, 1H), 7.46 (t, *J* =8Hz, 1H), 7.28 (s, 1H), 7.24 (s, 1H), 7.12 (t, *J* =8Hz, 1H), 7.00(d, *J* =8Hz, 2H), 6.64 (s, 1H), 5.06(s, 1H), 4.92-4.85 (m, 3H), 4.76-4.68 (m, 2H), 3.63 (q, *J* =8Hz, 2H),3.54 (q, J=8Hz, 1H), 3.08 (t, J=8Hz, 1H), 1.57 (d, J=4Hz, 3H), 1.25 (t, J=6Hz, 3H), 1.16 (m, 6H),0.88 (d, J=8Hz, 3H). ³¹P NMR (162 MHz, CDCl₃) δ1.88 (s,1P).

**Example 3B (Isomer B)**:LC-MS:m/z 570 (M+H)⁺. ¹H NMR (400 MHz, CDCl₃) δ 10.71(s, 1H), 8.01(d, *J* =8Hz, 1H), 7.94 (d, J=8Hz, 1H), 7.63 (t, J=8Hz, 1H), 7.48 (t, J=8Hz, 1H), 7.05 (m, 3H), 6.59 (d, J=8Hz, 2H), 6.51(s, 1H),5.15(s, 1H), 5.04-4.98 (m, 1H), 4.88-4.81 (m, 2H), 4.72-4.63 (m, 2H), 3.73 (q, *J* =8Hz, 1H), 3.60 (q, *J* =8Hz, 2H), 3.50 (t, J=8Hz, 1H), 1.62(d, J=8Hz,3H), 1.25-1.22 (m,12H). ³¹P NMR (162 MHz, CDCl₃) δ 1.27 (s,1P).

### Examples 4A and 4B isopropyl ((S)-(((S)-1-(4-amino-2-(ethoxymethyl)-1H-imidazo [4,5-c] quinolin-1-yl) propan-2-yl) oxy) (naphth-1-yloxy) phosphoryl)-L-alaninate and isopropyl ((R)-(((S)-1-(4-amino-2-(ethoxymethyl)-1H-imidazo [4,5-c] quinolin-1-yl) propan-2-yl) oxy) (naphth-1-yloxy) phosphoryl)-L-alaninate

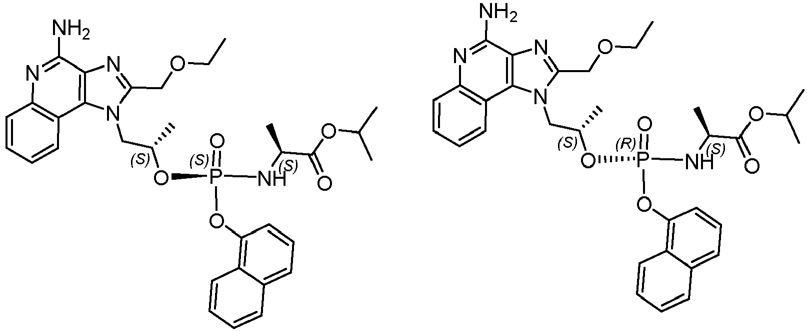

**Example 4A (Isomer A)**:LC-MS:m/z 620 (M+H)⁺.

**Example 4B (Isomer B)**:LC-MS:m/z 620 (M+H)⁺.

### Examples 5A and 5B isopropyl ((S)-(((R)-1-(4-amino-2-(ethoxymethyl)-1H-imidazo [4,5-c] quinolin-1-yl) propan-2-yl) oxy) (naphth-1-yloxy) phosphoryl)-L-alaninate and isopropyl ((R)-(((R)-1-(4-amino-2-(ethoxymethyl)-1H-imidazo [4,5-c] quinolin-1-yl) propan-2-yl) oxy) (naphth-1-yloxy) phosphoryl)-L-alaninate

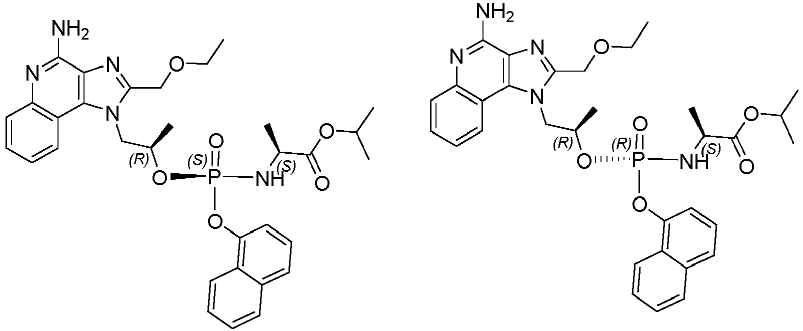

**Example 5A (Isomer A)**:LC-MS:m/z 620 (M+H)⁺. ¹H NMR (400 MHz, CDCl₃) δ 8.67 (s, 1 H), 7.91(d, *J* =8Hz, 1 H), 7.83 (d, *J* =8Hz, 1 H), 7.69 (d, *J* =8Hz, 1 H),7.52-7.44 (m, 3 H), 7.36-7.29 (m, 3 H), 7.06 (d, J=8Hz, 1 H), 7.00 (t, J=8Hz, 1 H), 5.24 (s, 1 H), 5.07-5.00 (m, 1 H), 4.78-4.68 (m, 2 H), 4.59-4.53 (m, 2 H), 3.98-3.88 (m, 1 H), 3.64 (t, J=8Hz, 1 H), 3.58-3.52 (m, 2 H), 1.67 (d, J=4Hz, 3 H), 1.36 (d, J=8Hz, 3 H), 1.25-1.22 (m, 9H).

**Example 5B (Isomer B)**:LC-MS:m/z 620 (M+H)⁺. ¹H NMR (400 MHz, CDCl₃) δ 8.78 (s, 1 H), 8.00 (d, *J* =8Hz, 1 H), 7.93 (d, *J* =8Hz, 1 H), 7.83 (d, *J* =12Hz, 1 H), 7.72 (d, J=8Hz, 1 H), 7.64 (d, J=8Hz, 1 H), 7.55-7.48 (m, 2 H), 7.46-7.41(m, 2 H), 7.36-7.29(m, 2 H),5.15 (s, 1 H), 4.88-4.67 (m, 5 H), 3.57-3.50 (m, 2 H), 3.42 (q,J =8Hz, 1 H), 2.98 (t, *J* =8Hz, 1 H), 1.60 (d, *J* =4Hz, 3 H), 1.21 (t, *J* =8Hz, 3 H), 1.09-1.06 (m, 6 H),0.89 (d, *J* =8Hz, 3 H).

### Examples 6A and 6B isopropyl ((S)-(((R)-1-(4-amino-2-(ethoxymethyl)-1H-imidazo [4,5-c] quinolin-1-yl) propan-2-yl) oxy) (naphth-2-yloxy) phosphoryl)-L-alaninate and isopropyl ((R)-(((R)-1-(4-amino-2-(ethoxymethyl)-1H-imidazo [4,5-c] quinolin-1-yl) propan-2-yl) oxy) (naphth-2-yloxy) phosphoryl)-L-alaninate

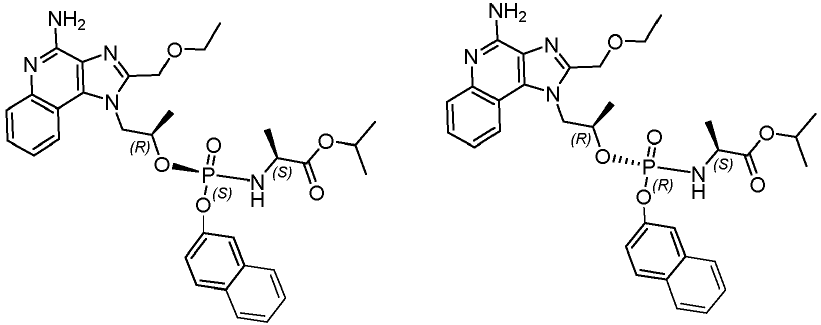

**Example 6A (Isomer A)**:LC-MS:m/z 620 (M+H)⁺. ¹H NMR (400 MHz, CDCl₃) δ 8.73 (s, 1 H), 7.95(d, J=8Hz, 1 H), 7.82 (d, J=8Hz, 1 H), 7.77 (d, *J* =12Hz, 1 H), 7.55 (t, J=8Hz, 1 H), 7.51(d, J=8Hz, 1 H),7.45-7.43 (m, 3 H), 7.39 (s, 1 H), 7.30 (t, *J* =4Hz, 1 H), 7.10 (s, 1 H), 6.69 (d, J=12Hz, 1 H),5.25-5.17 (m, 1 H), 5.04-4.98 (m, 1 H), 4.84-4.77 (m, 2 H), 4.61-4.56 (m, 1 H), 3.86-3.77 (m, 1 H), 3.63 (d, *J* =8Hz, 1 H), 3.56 (q, *J* =8Hz, 3 H), 1.66 (d, *J* =8Hz, 3 H), 1.29 (d, *J* =8Hz, 3 H),1.24 (m, 9 H).

**Example 6B (Isomer B)**:LC-MS:m/z 620 (M+H)⁺. ¹H NMR (400 MHz, CDCl₃) δ 8.77(s, 1 H), 8.00 (d, *J* =8Hz, 1 H), 7.94 (d, *J* =8Hz, 1 H), 7.81 (d, *J* =8Hz, 1 H), 7.73 (t, J=8Hz, 2 H), 7.56 (t, J=8Hz, 1 H), 7.52-7.42 (m, 3 H), 7.34(t, J=8Hz, 1 H), 7.15(d, J=12Hz, 1 H),5.12 (s, 1 H), 4.91-4.69(m, 5 H), 3.58 (q,J=8Hz, 2 H), 3.48-3.39 (m,1 H), 3.09(t, J=8Hz, 2 H), 1.58 (d, J=4Hz, 3 H), 1.23 (t, J=8Hz, 3 H), 1.12-1.08 (m, 6 H),0.98 (d, J=8Hz, 3 H).

### Examples 7A and 7B Isopropyl ((S)-(((R)-1-(4-amino-2-(ethoxymethyl)-1H-imidazo [4,5-c] quinolin-1-yl) propan-2-yl) oxy) (2-methyl-phenoxy) phosphoryl)-L-alaninate and isopropyl ((R)-(((R)-1-(4-amino-2-(ethoxymethyl)-1H-imidazo [4,5-c] quinolin-1-yl) propan-2-yl) oxy) (2-methyl-phenoxy) phosphoryl)-L-alaninate

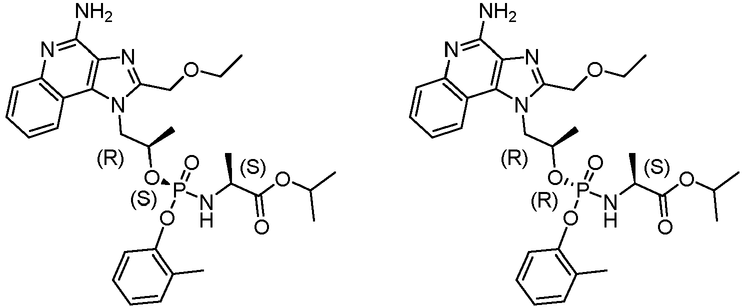

**Example 7A (Isomer A)**:LC-MS:m/z 584 (M+H)⁺. ¹H NMR (400 MHz, CDCl₃) δ 8.77 (s, 1 H), 7.98(d, J=8Hz, 1 H), 7.92 (d, *J* =12Hz, 1 H), 7.57 (t, J=8Hz, 1 H), 7.40(t, *J* =8Hz, 1 H), 6.92-6.77(m, 4H), 5.15 (s, 1 H),5.06-4.99 (m,1 H), 4.83-4.77 (m,2 H), 4.66-4.57 (m,2 H), 3.87-3.79 (m,1 H),3.59 (q, J=8Hz,2 H), 3.51(t, J=8Hz, 1 H),1.73 (s,3 H),1.61 (d, *J* =4Hz, 3 H), 1.31(d, *J* =8Hz, 3 H),1.27-1.23 (m, 9 H). ³¹P NMR (162 MHz, CDCl₃) δ1.22(s,1P).

**Example 7B (Isomer B)**:LC-MS:m/z 584 (M+H)⁺. ¹H NMR (400 MHz, CDCl₃) δ 8.76 (s, 1 H), 7.99(d, J=8Hz, 1 H), 7.90 (d, J=8Hz, 1 H), 7.53 (t, J=8Hz, 1 H), 7.31(t, J=8Hz, 1 H), 7.23(d, J=8Hz, 1 H), 7.13-7.07(m, 2 H), 7.02 (t, J=8Hz, 1 H), 5.10 (s, 1 H),4.91-4.67 (m,5H), 3.59 (q, J=8Hz,2 H), 3.41-3.31(m,1 H), 3.08 (t, J=8Hz, 1 H),2.05(s,3 H),1.56 (d, J=8Hz, 3 H), 1.24(t, J=8Hz, 3 H),1.12-1.10 (m, 6H), 0.93(d, *J* =8Hz, 3 H). ³¹P NMR (162 MHz, CDCl₃) δ 1.81(s,lP).

### Examples 8A and 8B Isopropyl ((S)-(((R)-1-(4-amino-2-(ethoxymethyl)-1H-imidazo [4,5-c] quinolin-1-yl) propan-2-yl) oxy) (3-methyl-phenoxy) phosphoryl)-L-alaninate and isopropyl ((R)-(((R)-1-(4-amino-2-(ethoxymethyl)-1H-imidazo [4,5-c] quinolin-1-yl) propan-2-yl) oxy) (3-methyl-phenoxy) phosphoryl)-L-alaninate

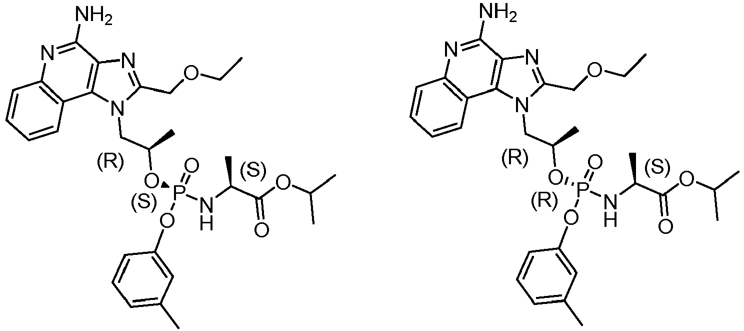

**Example 8A (Isomer A)**:LC-MS:m/z 584 (M+H)⁺. ¹H NMR (400 MHz, CDCl₃) δ 8.74 (s, 1 H), 7.98(d, *J* =12Hz, 1 H), 7.91 (d, J=8Hz, 1 H), 7.58 (t, J=8Hz, 1 H), 7.41 (t, *J* =8Hz, 1 H), 6.91(t, *J* =8Hz, 1 H),6.82(d, *J* =8Hz, 1 H),6.42(J =8Hz, 1 H),6.35(s, 1 H), 5.13 (s, 1 H), 5.04-4.97 (m, 1 H), 4.83 (t, *J* =12Hz, 2 H), 4.71(d, *J* =12Hz, 1 H),4.66-4.60 (m, 1 H), 3.77-3.67 (m, 1 H), 3.59 (q, J=8Hz, 3 H),2.15(s,3 H),1.62 (d, *J* =8Hz, 3 H), 1.27-1.22 (m, 12 H). ³¹P NMR (162 MHz, CDCl₃) δ1.26(s,1P).

**Example 8B (Isomer B)**:LC-MS:m/z 584 (M+H)⁺. ¹H NMR (400 MHz, CDCl₃) δ 8.78(s, 1 H), 8.01 (d, J=4Hz, 1 H), 7.91 (d, J=8Hz, 1 H), 7.56 (t, J=8Hz, 1 H), 7.36 (t, *J* =8Hz, 1 H), 7.14 (t, *J* =8Hz, 1 H), 6.95(d, *J* =8Hz, 1 H),6.81(s, 2 H),5.09 (s, 1 H), 4.92-4.68(m, 4 H), 3.60 (q,J=8Hz, 2 H), 3.43-3.33 (m,1 H), 2.98(t, J=8Hz, 1 H), 2.30 (s, 3 H),1.56 (d, J=8Hz, 3 H), 1.24 (t, J=8Hz, 3 H), 1.14-1.11 (m, 6 H),0.94(d, *J* =8Hz, 3 H). ³¹P NMR (162 MHz, CDCl₃) δ1.50(s,1P).

### Examples 9A and 9B isopropyl ((S)-(((R)-1-(4-amino-2-(ethoxymethyl)-1H-imidazo[4,5-c] quinolin-1-yl) propan-2-yl) oxy) (4-methyl-phenoxy) phosphoryl)-L-alaninate and isopropyl ((R)-(((R)-1-(4-amino-2-(ethoxymethyl)-1H-imidazo [4,5-c] quinolin-1-yl) propan-2-yl) oxy) (4-methyl-phenoxy) phosphoryl)-L-alaninate

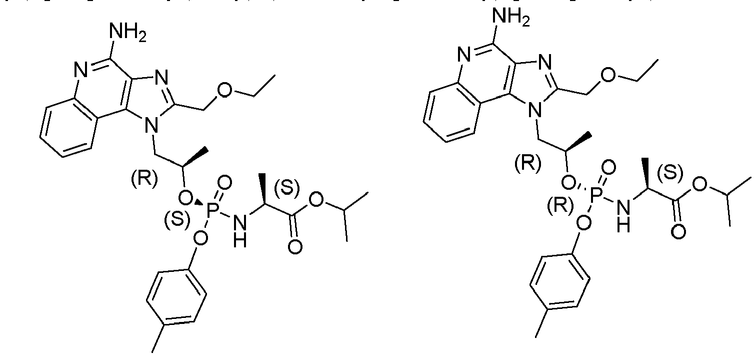

**Example 9A (Isomer A)**:LC-MS:m/z 584 (M+H)⁺. ¹H NMR (400 MHz, CDCl₃) δ 8.74 (s, 1 H), 7.99(d, J=8Hz, 1 H), 7.93 (d, *J* =12Hz, 1 H), 7.58 (t, J=8Hz, 1 H), 7.41(t, *J* =8Hz, 1 H), 6.83 (d, *J* =8Hz, 2 H), 6.49 (d, *J* =8Hz, 2 H), 5.13 (s, 1 H),5.03-4.97 (m, 1 H), 4.86-4.80 (m,2 H), 4.72 (d, *J* =12Hz, 1 H), 4.65-4.60 (m, 1 H), 3.77-3.71(m, 1 H),3.59 (q, J=8Hz, 2 H), 3.50(t, J=8Hz, 1 H),2.24 (s,3 H),1.61 (d, *J* =8Hz, 3 H), 1.27-1.21 (m, 12 H). ³¹P NMR (162 MHz, CDCl₃) δ1.46(s,1P).

**Example 9B (Isomer B)**:LC-MS:m/z 584 (M+H)⁺. ¹H NMR (400 MHz, CDCl₃) δ 8.77 (s, 1 H), 8.00(d, J=8Hz, 1 H), 7.90 (t, J=8Hz, 1 H), 7.55 (q, J=8Hz, 1 H), 7.35(t, J=8Hz, 1 H), 7.06(d, J=8Hz, 2 H), 6.90(d, J=4Hz, 2 H), 5.09 (s, 1 H),4.91-4.68 (m,4 H), 3.60 (q, *J* =8Hz, 2 H), 3.41-3.35(m,1 H), 3.12-2.99 (m,1 H),2.29(s,3 H),1.55 (d, *J* =8Hz, 3 H), 1.24(t, *J* =8Hz, 3 H), 1.13-1.11 (m, 6 H), 0.94(d, *J* =8Hz, 3 H). ³¹P NMR (162 MHz, CDCl₃) δ1.78(s,1P).

### Examples 10A and 10B isopropyl ((S)-(((R)-1-(4-amino-2-(ethoxymethyl)-1H-imidazo[4,5-c] quinolin-1-yl) propan-2-yl) oxy) (2-chloro-phenoxy) phosphoryl)-L-alaninate and isopropyl ((R)-(((R)-1-(4-amino-2-(ethoxymethyl)-1H-imidazo [4,5-c] quinolin-1-yl) propan-2-yl) oxy) (2-chloro-phenoxy) phosphoryl)-L-alaninate

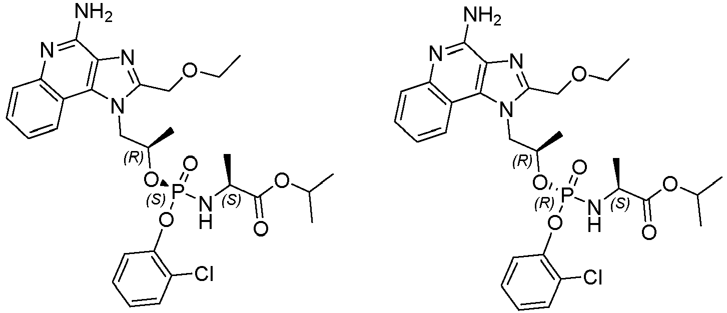

**Example 10A (Isomer A)**:LC-MS:m/z 604 (M+H)⁺. ¹H NMR (400 MHz, CDCl₃) δ 8.80(s, 1H), 7.94(d, *J* =8Hz, 1H), 7.85(d, *J* =8Hz, 1H), 7.52(t, *J* =8Hz, 1H), 7.33(t, *J* =8Hz, 1H), 7.18 (t, J=8Hz, 1H), 7.01-6.87(m, 3H), 5.21(m, 1H), 5.50(m, 1H), 4.90-4.84(m, 2H), 4.71-4.61(m, 2H), 3.87(m, 1H), 3.60(q, J=8Hz, 3H), 1.63(d, *J* =8Hz, 3H), 1.28-1.22(m, 12H). ³¹P NMR (162 MHz, CDCl₃) δ 1.20 (s).

**Example 10B (Isomer B)**:LC-MS:m/z 604 (M+H)⁺. ¹H NMR (400 MHz, CDCl₃) δ 8.78(s, 1H), 7.99(d, *J* =8Hz, 1H), 7.89(d, *J* =8Hz, 1H), 7.54(t, 1H, *J* =8Hz), 7.39-7.32(m, 3H), 7.20 (t, J=8Hz, 1H), 7.07 (t, J=8Hz, 1H), 5.15(m, 1H), 4.96-4.71(m, 5H), 3.60(q, J=8Hz, 2H), 3.49(m, 1H), 3.19(t, J=8Hz, 1H), 1.60(d, *J* =8Hz, 3H), 1.24(t, *J* =8Hz, 3H), 1.13(t, *J* =8Hz, 6H), 0.92(d, *J* =8Hz, 3H). ³¹P NMR (162 MHz, CDCl₃) δ 1.87 (s).

### Examples 11A and 11B isopropyl ((S)-(((R)-1-(4-amino-2-(ethoxymethyl)-1H-imidazo [4,5-c] quinolin-1-yl) propan-2-yl) oxy) (3-chloro-phenoxy) phosphoryl)-L-alaninate and isopropyl ((R)-(((R)-1-(4-amino-2-(ethoxymethyl)-1H-imidazo [4,5-c] quinolin-1-yl) propan-2-yl) oxy) (3-chloro-phenoxy) phosphoryl)-L-alaninate

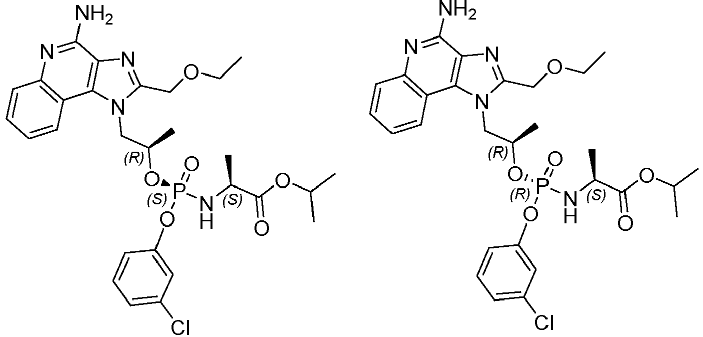

**Example 11A (Isomer A):LC-MS:m/z** 604 (M+H)⁺. ¹H NMR (400 MHz, CDCl₃) δ 8.79(s, 1H), 7.99(d, J=8Hz, 1H), 7.88(d, J=8Hz, 1H), 7.56(t, J=8Hz, 1H), 7.38(t, *J* =8Hz, 1H), 7.00 (m, 2H), 6.70(s, 1H), 6.49(d, J=8Hz, 1H), 5.15(m, 1H), 5.00(m, 1H), 4.84(m, 2H), 4.73-4.62(m, 2H), 3.68(m, 1H), 3.62-3.53(m, 3H), 1.63(d, J=8Hz, 3H), 1.25-1.18(m, 12H). ³¹P NMR (162 MHz, CDCl₃) δ 1.14 (s).

**Example 11B (Isomer B):LC-MS:m/z** 604 (M+H)⁺. LCMS m/z 604.59 (M+H)^{+ 1}H NMR (400 MHz, CDCl₃) δ 8.78(s, 1H), 8.01(d, J=8Hz, 1H), 7.92(d, J=8Hz, 1H), 7.58(t, J=8Hz, 1H), 7.37(t, J=8Hz, 1H), 7.20(d, J=8Hz, 1H), 7.14(d, J=8Hz, 1H), 7.03(s, 1H), 6.96(d, J=8Hz, 1H), 5.12(m, 1H), 4.89-4.70(m, 5H), 3.65(q, 2H, *J* =8Hz), 3.36(m, 1H), 3.02(t, J=8Hz, 1H), 1.58(d, *J* =8Hz, 3H), 1.26(t, *J* =8Hz, 3H), 1.13(m, *J* =8Hz, 6H), 0.96(d, *J* =8Hz, 3H). ³¹P NMR (162 MHz, CDCl₃) δ 1.61 (s).

### Examples 12A and 12B isopropyl ((S)-(((R)-1-(4-amino-2-(ethoxymethyl)-1H-imidazo [4,5-c] quinolin-1-yl) propan-2-yl) oxy) (4-chloro-phenoxy) phosphoryl)-L-alaninate and isopropyl ((R)-(((R)-1-(4-amino-2-(ethoxymethyl)-1H-imidazo [4,5-c] quinolin-1-yl) propan-2-yl) oxy) (4-chloro-phenoxy) phosphoryl)-L-alaninate

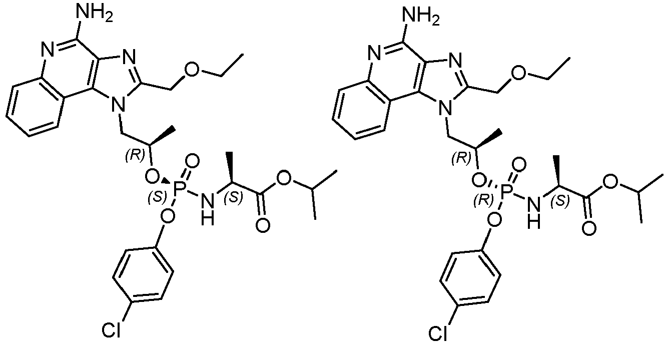

**Example 12A (Isomer A)**:LC-MS:m/z 604 (M+H)⁺. ¹H NMR (400 MHz, CDCl₃) δ 8.78(s, 1H), 8.00(d, J=8Hz, 1H), 7.88(d, *J* =8Hz, 1H), 7.55(t, *J* =8Hz, 1H), 7.35(t, *J* =8Hz, 1H), 7.05(d, *J* =8Hz, 2H), 6.60(d, *J* =8Hz, 2H), 5.15(m, 1H), 4.98(m, 1H), 4.90-4.84(m, 2H), 4.72-4.63(m, 2H), 3.70-3.64(m, 1H), 3.59(q, J=8Hz, 2H), 3.47(t*J* =8Hz, 1H,) 1.61(d, J=4Hz, 3H), 1.26-1.20(m, 9H), 1.16(d, J=8Hz, 3H). ³¹P NMR (162 MHz, CDCl₃) δ 1.29 (s).

**Example 12B (Isomer B)**:LC-MS:m/z 604 (M+H)⁺. ¹H NMR (400 MHz, CDCl₃) δ 8.71(s, 1H), 7.99(d, J=8Hz, 1H), 7.88(d, *J* =8Hz, 1H), 7.54(t, *J* =8Hz, 1H), 7.33(t, *J* =8Hz, 1H), 7.22(d, J=8Hz, 2H), 6.98(d, *J* =8Hz, 2H), 5.13(m, 1H), 4.91-4.69(m, 5H), 3.61(q, *J* =8Hz, 2H), 3.33(m, 1H), 3.12(t, *J* =8Hz, 1H), 1.57(d, *J* =8Hz, 3H), 1.27(t, *J* =8Hz, 3H), 1.13(t, *J* =8Hz, 6H), 0.93(d, *J* =8Hz, 3H). ³¹P NMR (162 MHz, CDCl₃) δ 1.72 (s).

### Examples 13A and 13B

### Isopropyl ((S)-(((R)-1-(4-amino-2-(ethoxymethyl)-1H-imidazo [4,5-c] quinolin-1-yl) propan-2-yl) oxy) (phenoxy) phosphoryl)-D-alaninate and isopropyl ((R)-(((R)-1-(4-amino-2-(ethoxymethyl)-1H-imidazo [4,5-c] quinolin-1-yl) propan-2-yl) oxy) (phenoxy) phosphoryl)-D-alaninate

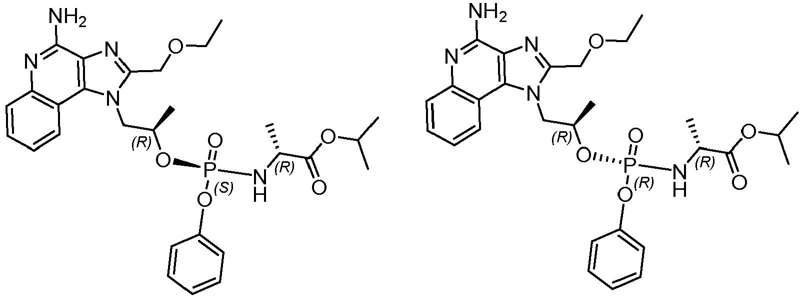

**Example 13A (Isomer A)**:LC-MS:m/z 570 (M+H)⁺. ¹H NMR (400 MHz, CDCl₃) δ 8.72 (s, 1 H), 8.03(d, *J* =8Hz, 1 H), 7.95 (d, *J* =8Hz, 1 H), 7.60 (t, *J* =8Hz, 1 H), 7.44 (t, *J* =8Hz, 1 H), 7.09-7.03 (m, 3 H), 6.64 (d, *J* =8Hz, 2 H), 5.15 (s, 1 H), 4.94-4.82 (m, 3 H), 4.72-4.62 (m, 2 H), 3.74-3.65(m, 1 H), 3.59 (q, *J* =8Hz, 2 H), 3.36 (t, *J* =8Hz, 1 H), 1.64(d, *J* =4Hz, 3 H), 1.25 (t, *J* =8Hz, 3 H), 1.21-1.17 (m, 9 H).

**Example 13B (Isomer B)**:LC-MS:m/z 570 (M+H)⁺. ¹H NMR (400 MHz, CDCl₃) δ 8.76 (s,1H), 7.99 (d, *J* = 12Hz, 1H), 7.94 (d, *J* = 8Hz, 1H), 7.58 (t, *J* =8 Hz, 1H), 7.39 (t, *J* =8 Hz, 1H), 7.29 (s, 1H), 7.26 (t, *J* =4 Hz, 1H), 7.13 (t, *J* =8 Hz, 1H),7.05 (d, *J* =8 Hz, 2 H), 5.03 (s, 1 H), 4.91-4.84 (m,3 H), 4.74-4.66 (m, 2 H), 3.60 (q, *J* =8 Hz,2H), 3.53(t, *J* =8 Hz, 2H), 2.93 (t, *J* =8Hz, 1 H), 1.56 (d, *J* =8 Hz, 3 H), 1.25(t, *J* =8 Hz, 3H), 1.16-1.14 (m, 6H), 0.82(d, *J* =8 Hz, 3H). ³¹P NMR (162 MHz, CDCl₃) δ2.04 (s,1P).

### Examples 14A and 14B tert-butyl ((S)-(((R)-1-(4-amino-2-(ethoxymethyl)-1H-imidazo [4,5-c] quinolin-1-yl) propan-2-yl) oxy) (phenoxy) phosphoryl)- L-alaninate and tert-butyl ((R)-(((R)-1-(4-amino -2-(ethoxymethyl)-1H-imidazo [4,5-c] quinolin-1-yl) propan-2-yl) oxy) (phenoxy) phosphoryl)- L-alaninate

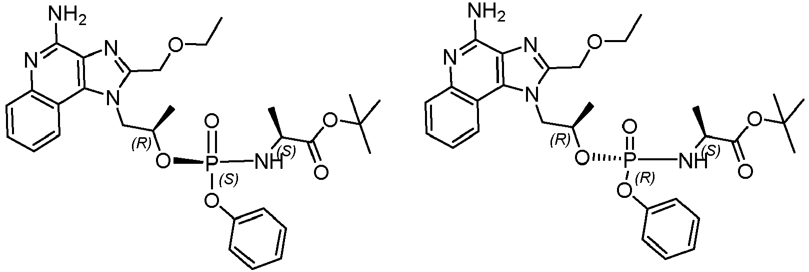

**Example 14A (Isomer A)**:LC-MS:m/z 584 (M+H)⁺. ¹H NMR (400 MHz, CDCl₃) δ8.02 (d, *J* =8Hz, 1 H), 7.87 (d, *J* =8Hz, 1 H), 7.55 (t, *J* =8Hz, 1 H), 7.36 (t, *J* =8Hz, 1 H), 7.08 (t, *J* =8Hz, 2 H), 7.01 (t, *J* =8Hz, 1 H), 6.67 (d, *J* =8Hz, 2 H), 5.14 (s, 1 H), 4.90-4.84 (m, 2 H), 4.72-4.62(m, 2 H), 3.68-3.63(m, 1 H), 3.6-3.55 (q, *J* =8Hz, 2 H), 3.43 (t, *J* = 12Hz, 1 H), 1.60 (d, *J* =4Hz, 3 H), 1.43 (s, 9H),1.23 (t, *J* =8Hz, 3 H),1.16 (d, J=8Hz, 3 H). ³¹P NMR (162 MHz, CDCl₃) δ1.34 (s, 1P).

**Example 14B (Isomer B)**:LC-MS:m/z 584 (M+H)⁺. ¹H NMR (400 MHz, CDCl₃) δ8.01 (d, *J* =8Hz, 1 H), 7.88 (d, *J* =8Hz, 1 H), 7.55 (t, *J* =8Hz, 1 H), 7.34 (t, *J* =8Hz, 1 H), 7.30 (d, *J* =8Hz, 2 H), 7.13 (t, *J* =8Hz, 1 H), 7.05 (d, *J* =8Hz, 2 H), 5.11 (s, 1 H), 4.91-4.84 (m, 2 H), 4.77-4.68 (m, 2 H), 3.61-3.56 (q, *J* =8Hz, 2 H), 3.33-3.23 (m, 1 H), 2.97 (t, *J* =8Hz, 1 H), 1.60 (d, *J* =4Hz, 3 H), 1.32(s,9 H),1.24 (t, *J* =8Hz, 3 H),0.89 (d, *J* =8Hz, 3 H). ³¹P NMR (162 MHz, CDCl₃) δ1.65 (s,1P).

### Examples 15A and 15B methyl ((S)-(((R)-1-(4-amino-2-(ethoxymethyl)-1H-imidazo [4,5-c] quinolin-1-yl) propan-2-yl) oxy) (phenoxy) phosphoryl)- L-alaninate and methyl ((R)-(((R)-1-(4-amino -2-(ethoxymethyl)-1H-imidazo [4,5-c] quinolin-1-yl) propan-2-yl) oxy) (phenoxy) phosphoryl)- L-alaninate

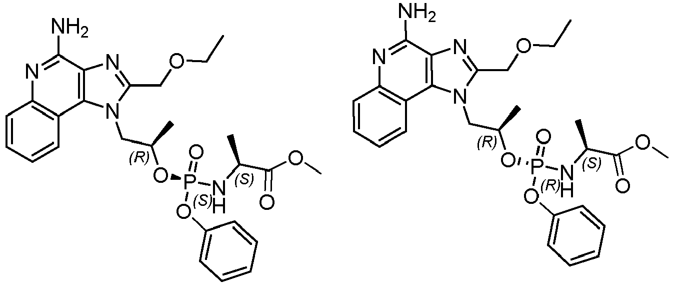

**Example 15A (Isomer A)**:LC-MS:m/z 542 (M+H)⁺. ¹H NMR (400 MHz, CDCl₃) δ8.01(d, *J* =8Hz, 1 H), 7.93 (d, *J* =8Hz, 1 H), 7.63 (t, *J* =8Hz, 1 H), 7.47 (t, *J* =8Hz, 1 H), 7.08-7.03(m, 3 H),6.60-6.55 (m, 3 H), 5.15 (s, 1 H), 4.88-4.81 (m, 2 H), 4.72-4.63 (m, 2 H), 3.77(t, *J* =8Hz, 1 H), 3.71 (s, 3 H), 3.60 (q, *J* =8Hz,2 H), 3.50(t, *J* =8Hz, 1 H),1.63 (d, *J* =4Hz, 3 H), 1.27-1.23 (m, 6 H). ³¹P NMR (162 MHz, CDCl₃) δ1.08 (s,1P).

**Example 15B (Isomer B)**:LC-MS:m/z 542 (M+H)⁺. ¹H NMR (400 MHz, CDCl₃) δ8.05 (d, *J* =8Hz, 1 H), 7.97 (d, *J* =8Hz, 1 H), 7.62 (t, *J* =8Hz, 2 H), 7.43 (t, *J* =8Hz, 1 H), 7.31-7.29(m, 1 H), 7.28 (s, 1 H), 7.16(t, *J* =8Hz, 1 H), 7.04(d, *J* =8Hz, 2 H),5.10 (s, 1 H), 4.93-4.92(d, *J* =4Hz, 1 H), 4.88(t, *J* =8Hz, 1 H), 4.81-4.71(m, 2 H), 3.64 (q,*J* =8Hz, 2 H), 3.56(s,3 H), 3.46 (q,*J* =4Hz, 1 H),2.99(t, *J* =8Hz, 1 H), 1.60 (d, *J* =4Hz, 3 H), 1.28 (t, *J* =8Hz, 3 H), 1.01 (d, *J* =8Hz, 3 H). ³¹P NMR (162 MHz, CDCl₃) δ1.65 (s,1P).

### Examples 16A and 16B Benzyl ((S)-(((R)-1-(4-amino-2-(ethoxymethyl)-1H-imidazo [4,5-c] quinolin-1-yl) propan-2-yl) oxy) (phenoxy) phosphoryl)- L-alaninate and benzyl ((R)-(((R)-1-(4-amino -2-(ethoxymethyl)-1H-imidazo [4,5-c] quinolin-1-yl) propan-2-yl) oxy) (phenoxy) phosphoryl)- L-alaninate

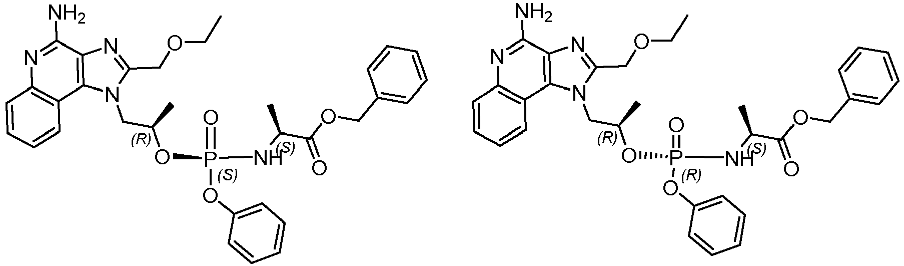

**Example 16A (Isomer A)**:LC-MS:m/z 618(M+H)⁺.

**Example 16B (Isomer B)**:LC-MS:m/z 618 (M+H)⁺.

### Examples 17A and 17B tert-butyl ((S)-(((R)-1-(4-amino-2-(ethoxymethyl)-1H-imidazo [4,5-c] quinolin-1-yl) propan-2-yl) oxy) (4-methylphenoxy) phosphoryl)- L-alaninate and tert-butyl ((R)-(((R)-1-(4-amino-2-(ethoxymethyl)-1H-imidazo [4,5-c] quinolin-1-yl) propan-2-yl) oxy) (4-methylphenoxy) phosphoryl)- L-alaninate

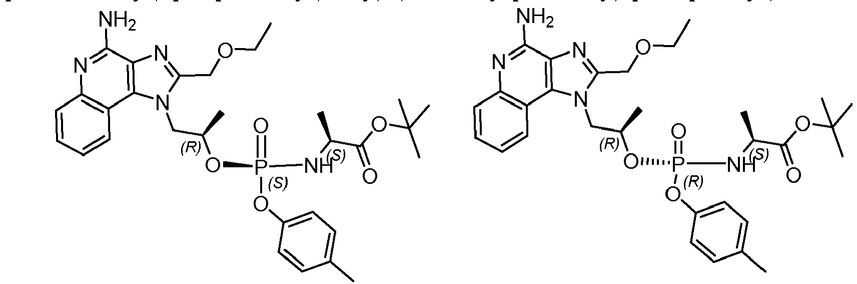

**Example 17A (Isomer A)**:LC-MS:m/z 598 (M+H)⁺. ¹H NMR (400 MHz, CDCl₃) δ8.00(d, *J* =8Hz, 1 H), 7.87 (d, *J* =8Hz, 1 H), 7.54 (t, *J* =8Hz, 1 H), 7.35 (t, *J* =8Hz, 1 H), 6.85 (d, *J* =8Hz, 2 H), 6.54 (d, J=8Hz, 2 H),5.12 (s, 1 H), 4.87 (t, *J* =12Hz, 1 H), 4.88-4.82 (m, 1 H), 4.73 (d, *J* =12Hz, 1 H), 4.66-4.61 (m, 1 H), 3.65 (t, *J* =8Hz, 1 H), 3.57 (q, *J* =8Hz, 2 H), 3.43 (t, *J* =8Hz, 1 H), 2.23 (s,3 H), 1.59 (d, *J* =8Hz, 3 H), 1.43(s,9H), 1.23 (t, J=8Hz, 3 H), 1.17 (d, J=8Hz, 3 H). ³¹P NMR (162 MHz, CDCl₃) δ1.53(s,1P)

**Example 17B (Isomer B)**:LC-MS:m/z 598 (M+H)⁺. ¹H NMR (400 MHz, CDCl₃) δ8.01(d, *J* =8Hz, 1 H), 7.89 (d, *J* =8Hz, 1 H), 7.55 (t, *J* =8Hz, 1 H), 7.35 (t, *J* =8Hz, 1 H),7.07 (d, *J* =8Hz, 2 H), 6.92 (d, *J* =8Hz, 2 H), 5.08 (s, 1 H), 4.91-4.87 (m, 1 H), 4.87 (d, *J* =12Hz, 1 H), 4.77 (d, *J* =12Hz, 1 H),4.73-4.67 (m, 1 H), 3.59 (t, *J* =8Hz, 2 H), 3.34-3.26 (m, 1 H), 2.94 (t, *J* =8Hz, 1 H), 2.30(s,3 H), 1.54 (d, *J* =8Hz, 3 H), 1.32(s,9H), 1.24 (t, J=8Hz, 3 H), 0.90 (d, J=4Hz, 3 H). ³¹P NMR (162 MHz, CDCl₃) δ1.83(s,1P).

### Examples 18A and 18B

### Tert-butyl ((S)-(((R)-1-(4-amino-2-(ethoxymethyl)-1H-imidazo [4,5-c] quinolin-1-yl) propan-2-yl) oxy) (4-methoxyphenoxy) phosphoryl)- L-alaninate and tert-butyl ((R)-(((R)-1-(4-amino-2-(ethoxymethyl)-1H-imidazo [4,5-c] quinolin-1-yl) propan-2-yl) oxy) (4-methoxyphenoxy) phosphoryl)- L-alaninate

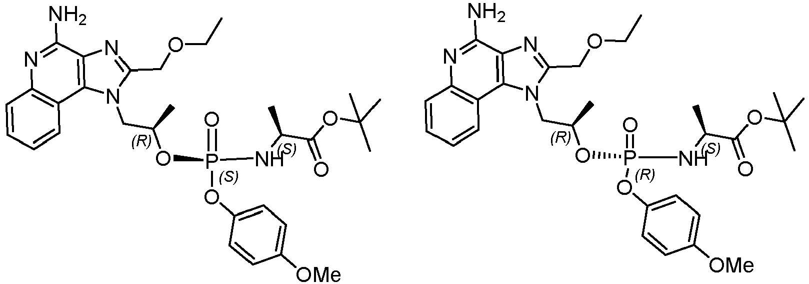

**Example 18A (Isomer A)**:LC-MS:m/z 614 (M+H)⁺. ¹H NMR (400 MHz, CDCl₃) δ8.01(d, *J* =8Hz, 1 H), 7.86 (d, *J* =8Hz, 1 H), 7.54 (t, *J* =8Hz, 1 H), 7.35 (t, *J* =8Hz, 1 H), 6.61-6.56(m, 4 H),5.13 (s, 1 H), 4.90-4.84 (m, 2 H), 4.73 (d, *J* =12Hz, 1 H),4.67-4.62 (m, 1 H), 3.72(s,3 H), 3.64 (t, *J* =8Hz, 1H), 3.57 (q, *J* =8Hz, 2 H), 3.37 (t, *J* =12Hz, 1 H), 1.59 (d, *J* =8Hz, 3 H), 1.43(s,9H), 1.23 (t, *J* =8Hz, 3 H), 1.16 (d, *J* =4Hz, 3 H). ³¹P NMR (162 MHz, CDCl₃) δ1.82(s,1P)

**Example 18B (Isomer B)**:LC-MS:m/z 614 (M+H)⁺. ¹H NMR (400 MHz, CDCl₃) δ8.02(d, *J* =8Hz, 1 H), 7.90 (d, *J* =8Hz, 1 H), 7.56 (t, *J* =8Hz, 1 H), 7.36 (t, *J* =8Hz, 1 H), 6.96 (d, *J* =8Hz, 2 H), 6.79 (d, *J* =8Hz, 2 H), 5.09 (s, 1 H), 4.91-4.86 (m, 2 H), 4.79 (d, *J* =12Hz, 1 H),4.73-4.68 (m, 1 H), 3.77(s,3 H), 3.60 (t, *J* =8Hz, 2 H), 3.30 (q, *J* =8Hz, 1 H), 2.89 (t, *J* =12Hz, 1 H), 1.53 (d, *J* =8Hz, 3 H), 1.32(s,9H), 1.24 (t, *J* =8Hz, 3 H), 0.90 (d, *J* =4Hz, 3 H). ³¹P NMR (162 MHz, CDCl₃) δ2.18(s,1P)

### Examples 19A and 19B tert-butyl ((S)-(((R)-1-(4-amino-2-(ethoxymethyl)-1H-imidazo [4,5-c] quinolin-1-yl) propan-2-yl) oxy) (4-chlorophenoxy) phosphoryl)- L-alaninate and tert-butyl ((R)-(((R)-1-(4-amino-2-(ethoxymethyl)-1H-imidazo [4,5-c] quinolin-1-yl) propan-2-yl) oxy) (4-chlorophenoxy) phosphoryl)- L-alaninate

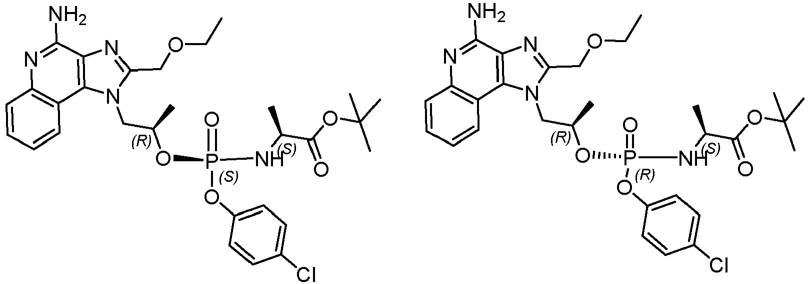

**Example 19A (Isomer A)**:LC-MS:m/z 618(M+H)⁺. ¹H NMR (400 MHz, CDCl₃) δ7.99(d, *J* =8Hz, 1 H), 7.83 (d, *J* =8Hz, 1 H), 7.52 (t, *J* =8Hz, 1 H), 7.32 (t, *J* =8Hz, 1 H), 7.03 (d, *J* =8Hz, 2 H), 6.59 (d, *J* =8Hz, 2 H), 5.14 (s, 1 H), 4.90-4.84 (m, 2 H), 4.72 (d, *J* =16Hz, 1 H),4.66-4.62 (m, 1 H), 3.62-3.54(m,3 H), 3.46 (t, *J* =8Hz, 1 H), 1.60 (d, *J* =8Hz, 3 H), 1.42(s,9H), 1.23 (t, *J* =8Hz, 3 H), 1.12 (d, *J* =8Hz, 3 H). ³¹P NMR (162 MHz, CDCl₃) δ1.38(s,1P)

**Example 19B (Isomer B)**:LC-MS:m/z 618(M+H)⁺. ¹H NMR (400 MHz, CDCl₃) δ7.99(d, *J* =8Hz, 1 H), 7.88 (d, *J* =8Hz, 1 H), 7.54 (t, *J* =8Hz, 1 H), 7.33 (t, *J* =8Hz, 1 H), 7.24 (d, *J* =8Hz, 2 H), 6.98 (d, J=8Hz, 2 H),5.10 (s, 1 H), 4.91-4.84 (m, 2 H), 4.78 (d, *J* =12Hz, 1 H),4.73-4.69 (m, 1 H), 3.60 (q, J=8Hz, 2 H), 3.32-3.23 (m, 1 H), 3.05 (s, 1 H), 1.56 (d, *J* =8Hz, 3 H), 1.31(s,9H), 1.24 (t, *J* =8Hz, 3 H), 0.92 (d, *J* =4Hz, 3 H). ³¹P NMR (162 MHz, CDCl₃) δ1.85(s,1P)

### Examples 20A and 20B neopentyl ((S)-(((R)-1-(4-amino-2-(ethoxymethyl)-1H-imidazo [4,5-c] quinolin-1-yl) propan-2-yl) oxy) (4-chlorophenoxy) phosphoryl)- L-alaninate and neopentyl ((R)-(((R)-1-(4-amino-2-(ethoxymethyl)-1H-imidazo [4,5-c] quinolin-1-yl) propan-2-yl) oxy) (4-chlorophenoxy) phosphoryl)- L-alaninate

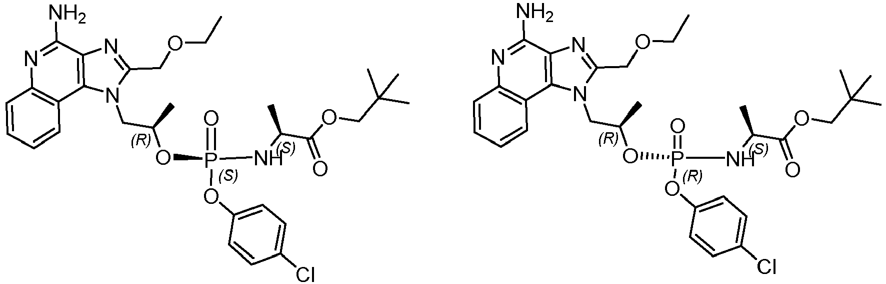

**Example 20A (Isomer A)**:LC-MS:m/z 632(M+H)⁺.

**Example 20B (Isomer B)**:LC-MS:m/z 632 (M+H)⁺.

### Examples 21A and 21B 3-amyl ((S)-(((R)-1-(4-amino-2-(ethoxymethyl)-1H-imidazo [4,5-c] quinolin-1-yl) propan-2-yl) oxy) (4-chlorophenoxy) phosphoryl)- L-alaninate and 3-amyl ((R)-(((R)-1-(4-amino-2-(ethoxymethyl)-1H-imidazo [4,5-c] quinolin-1-yl) propan-2-yl) oxy) (4-chlorophenoxy) phosphoryl)- L-alaninate

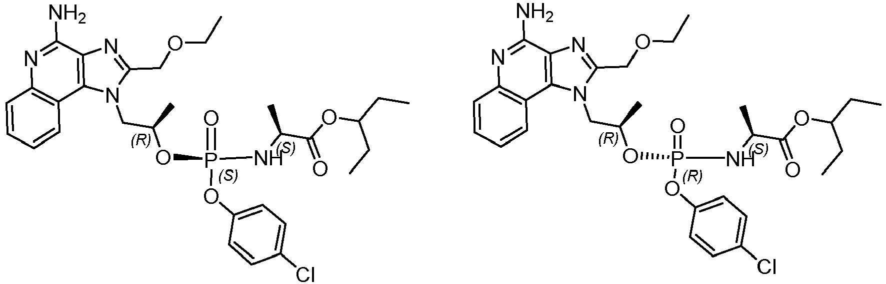

**Example 21A (Isomer A)**:LC-MS:m/z 632 (M+H)⁺.

**Example 21B (Isomer B)**:LC-MS:m/z 632 (M+H)⁺.

### Example 22 Tert-butyl (((R)-1-(4-amino-2-(ethoxymethyl)-1H-imidazo [4,5-c] quinolin-1-yl) propan-2-yl) oxy) (4-chlorophenoxy) phosphoryl)- L-valinate

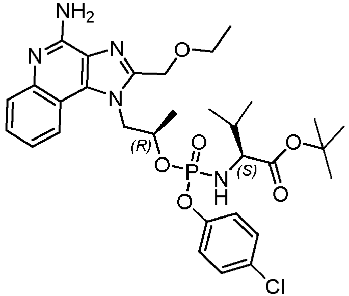

**Example 22:**LC-MS:m/z 646 (M+H)⁺. ¹H NMR (400 MHz, CDCl₃) δ8.00(d, *J* =8Hz, 1 H), 7.90 (d, *J* =8Hz, 1 H), 7.56 (t, *J* =8Hz, 1 H), 7.37 (t, *J* =8Hz, 1 H), 7.02(d, *J* =8Hz, 2 H), 6.57(d, *J* =8Hz, 2 H), 5.13(s, 1 H), 4.89-4.82 (m,2 H), 4.72-4.69 (d, *J* =12Hz, 1 H), 4.66-4.61 (d, J=12Hz, 1 H), 3.58 (q, J=8Hz, 2 H), 3.53-3.47(m,1 H),3.20 (t, *J* =8Hz, 1 H), 1.94-1.86(m,1 H),1.61 (d, *J* =8Hz, 3 H), 1.42(s,9H) ,1.24 (t, *J* =8Hz, 3 H), 0.84(d, *J* =8Hz, 3 H), 0.77(d, *J* =8Hz, 3 H).

### Examples 23A and 23B

### (R)-1-(4-amino-2-(ethoxymethyl)-1H-imidazo [4,5-c] quinolin-1-yl) propan-2-yl (4-chlorophenyl) (S)-((S)-3-methylbutan-2-yl) phosphoramide and (R)-1-(4-amino-2-(ethoxymethyl)-1H-imidazo [4,5-c] quinolin-1-yl) propan-2-yl (4-chlorophenyl) (R)-((S)-3-methylbutan-2-yl) phosphoramide

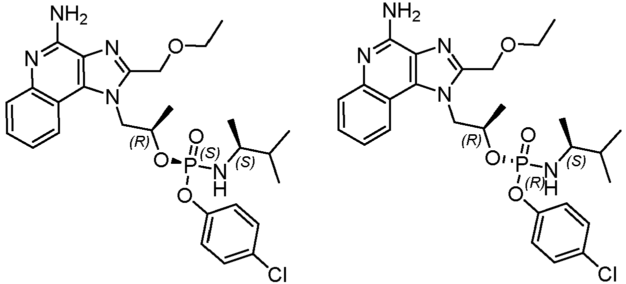

**Example 23A (Isomer A)**:LC-MS:m/z 560(M+H)⁺. ¹H NMR (400 MHz, CDCl₃) δ8.05(d, *J* =8Hz, 1 H), 7.86 (d, *J* =8Hz, 1 H), 7.54 (t, *J* =8Hz, 1 H), 7.33 (t, *J* =8Hz, 1 H), 7.05(d, J=8Hz, 2 H), 6.60(d, J=8Hz, 2 H), 5.12(s, 1 H), 4.91-4.85 (m, 2 H), 4.73 (d, *J* =12Hz, 1 H), 4.68-4.62(m, 1 H),3.57 (q, *J* =8Hz, 2 H), 2.87(s, 1 H),2.18 (t, *J* =8Hz, 1 H), 1.64 (d, *J* =8Hz, 3 H),1.49-1.40(m, 1 H), 1.23 (t, *J* =8Hz, 3 H), 0.85(d, *J* =8Hz, 3 H),0.70 (t, *J* =8Hz, 6H).

**Example 23B (Isomer B)**:LC-MS:m/z 560(M+H)⁺. ¹H NMR (400 MHz, CDCl₃) δ7.99(d, *J* =8Hz, 1 H), 7.86 (d, *J* =8Hz, 1 H), 7.54 (t, *J* =8Hz, 1 H), 7.31 (t, *J* =8Hz, 1 H), 7.27(s, 1 H), 7.25(s, 1 H), 7.05(d, *J* =4Hz, 2 H), 5.04(s, 1 H), 4.87-4.81 (m,2 H), 4.76-4.67 (m, 2H), 3.60 (q, *J* =8Hz, 2 H), 2.63(s,2 H), 1.91(t, *J* =8Hz, 1 H), 1.57 (d, *J* =8Hz, 3 H), 1.25 (t, *J* =8Hz, 3 H), 1.21-1.13(m,1 H), 0.62(d, *J* =8Hz, 3 H),0.45-0.42 (m, 6H).

### Examples 24A and 24B

### (R)-1-(4-amino-2-(ethoxymethyl)-1H-imidazo [4,5-c] quinolin-1-yl) propan-2-yl (4-chlorophenyl) (S)-((S)-1,1, 1-trifluoropropan-2-yl) phosphoramide and (R)-1-(4-amino-2-(ethoxymethyl)-1H-imidazo [4,5-c] quinolin-1-yl) propan-2-yl (4-chlorophenyl) (R)-((S)-1,1,1-trifluoropropan-2-yl) phosphoramide

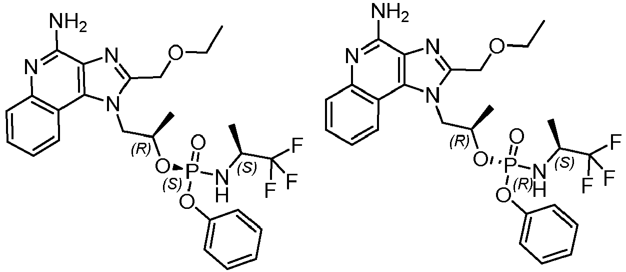

**Example 24A (Isomer A)**:LC-MS:m/z 552 (M+H)⁺. ¹H NMR (400 MHz, CDCl₃) δ8.02(d, *J* =8Hz, 1 H), 7.85 (d, *J* =8Hz, 1 H), 7.54 (t, *J* =8Hz, 1 H), 7.33(t, *J* =8Hz, 1 H), 7.05-6.97 (m, 3 H),6.77(s, 1 H), 6.58 (d, *J* =8Hz, 2 H), 5.13 (s, 1 H),4.89-4.83 (m,1 H), 4.85(d, *J* =12Hz, 1 H),4.71(d, *J* =12Hz, 1 H),4.64-4.60 (m, 1 H), 3.59-3.54 (m, 3 H), 3.20(s, 1 H), 1.64 (d, *J* =4Hz, 3 H), 1.23(t, *J* =8Hz, 3 H),1.13 (d, *J* =4Hz, 3 H). ³¹P NMR (162 MHz, CDCl₃) δ0.52 (s,1P)

**Example 24B (Isomer B)**:LC-MS:m/z 552 (M+H)⁺. ¹H NMR (400 MHz, CDCl₃) δ7.98(d, *J* =8Hz, 1 H), 7.89 (d, *J* =8Hz, 1 H), 7.57 (t, *J* =8Hz, 1 H), 7.36(t, *J* =8Hz, 1 H), 7.29(t, *J* =8Hz, 2H), 7.15(t, *J* =8Hz, 1 H), 7.05 (d, *J* =8Hz, 2H),5.05(s, 1 H), 4.93 (d, *J* =16Hz, 1 H), 4.90-4.84 (m, 1 H), 4.80(d, *J* =16Hz, 1 H), 4.75-4.70 (m, 1 H), 3.63 (m, 3 H), 3.56(s, 1 H),1.58 (d, *J* =4Hz, 3 H), 1.26(t, *J* =8Hz, 3 H),0.92 (d, *J* =8Hz, 3 H). ³¹P NMR (162 MHz, CDCl₃) δ1.87 (s,1P)

### Examples 25A and 25B

### (R)-1-(4-amino-2-(ethoxymethyl)-1H-imidazo [4,5-c] quinolin-1-yl) propan-2-yl (4-chlorophenyl) (S)-((S)-1-methoxypropan-2-yl) phosphoramide and (R)-1-(4-amino-2-(ethoxymethyl)-1H-imidazo [4,5-c] quinolin-1-yl) propan-2-yl (4-chlorophenyl) (R)-((S)-1-methoxypropan-2-yl) phosphoramide

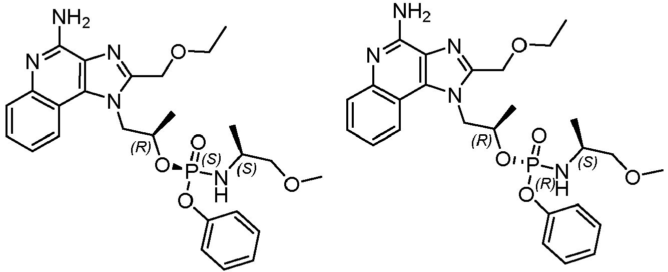

**Example 25A (Isomer A)**:LC-MS:m/z 528 (M+H)⁺. ¹H NMR (400 MHz, CDCl₃) δ 8.07(d, *J* =8Hz, 1 H), 7.88 (d, *J* =8Hz, 1 H), 7.57 (t, *J* =8Hz, 1 H), 7.42 (t, *J* =8Hz, 1 H), 7.09-7.00 (m, 4 H), 6.66 (d, *J* =8Hz, 2 H), 5.12 (s, 1 H), 4.90-4.65 (m, 4 H), 3.59 (q, *J* =8Hz, 2 H), 3.23(s,3 H), 3.16(s, 1 H), 3.08-3.07(m,2 H), 2.95 (t, *J* =8Hz, 1 H), 1.65(d, *J* =8Hz, 3 H), 1.24 (t, *J* =8Hz, 3 H),0.96 (d, *J* =8Hz, 3 H). ³¹P NMR (162 MHz, CDCl₃) δ2.65 (s,1P)

**Example 25B (Isomer B)**:LC-MS:m/z 528 (M+H)⁺. ¹H NMR (400 MHz, CDCl₃) δ8.10 (d, *J* = 8Hz, 1H), 7.93 (d, *J* = 8Hz, 1H), 7.58 (t, *J* =8 Hz, 1H), 7.45 (t, *J* =8 Hz, 1H), 7.29 (d, *J* = 8Hz, 1H), 7.24 (s, 1H), 7.11 (t, *J* =8 Hz, 1H),7.05 (d, *J* =8 Hz, 2 H), 5.06 (s, 1 H), 4.95-4.89 (m,2 H), 4.78-4.73 (m, 2 H), 3.62 (q, J=8 Hz,2H), 3.11(s,3H), 3.04(s, 1H),2.89-2.81(m,3H), 1.60(d, *J* =8 Hz, 3 H), 1.25(t, *J* =8 Hz, 3H), 0.83(d, *J* =8 Hz, 3H). ³¹P NMR (162 MHz, CDCl₃) δ3.05 (s,1P)

### Biological test evaluation

The following biological test examples further describe and explain the present invention, but these examples are not meant to limit the scope of the present invention.

Experiments on the agonistic activity of TLR7 and TLR8 by the compounds in vitro

### Experimental steps

The compound was diluted with 1:3 series with DMSO for 9 concentration points, duplicated wells, and added to 96-well plate with Echo.

The HEK-Blue^{™} hTLR cells were suspended in the culture medium and then seeded into a 96-well plate containing the compound at a density of 50,000 cells/well. Cells were cultured at 5% CO₂ and 37 °C for 1 day. The final concentration of DMSO in the cell culture medium was 0.5%.

20uL cell culture supernatant was taken, according to the QUANTI-Blue instructions, the reporter gene SEAP in the supernatant was detected, and the OD650 signal value was read with a microplate reader to calculate the agonist activity of the compound on the hTLR7and hTLR8.

According to the CellTiter-Glo instructions, the cell viability was measured, and the Luminescence signal value was read by the microplate reader, which was used to calculate the cytotoxicity of the compound.

The EC₅₀ and CCsovalues of the compound were calculated by GraphPad Prism software, and the fitting curve was plotted.

The control compound R848 has the following structure:

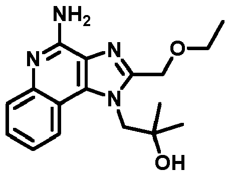

### See Table1 for the agonistic activity on TLR7 and TLR8 of the compounds of the examples of the present invention.

**Table 1 Enzymatic activity of compounds in examples of the present invention**

| **Example** | **TLR 7 EC₅₀ (µM)** | **TLR 8 EC₅₀ (µM)** | **CC₅₀ (µM)** | **TLR 7 selectivity TLR 8/TLR 7** | **TLR 8 selectivity TLR 7/TLR 8** |
|---|---|---|---|---|---|
| **R848** | 0.344 | 3.594 | > 10 | 10.4 | / |
| **Example 1** | 0.666 | 1.595 | > 30 | 2.4 | / |
| **Example 1A** | 0.440 | 1.078 | > 30 | 2.5 | / |
| **Example 1B** | 1.272 | 3.596 | > 30 | 2.8 | / |
| **Example 2A** | 2.8 | 0.881 | > 30 | / | 3.2 |
| **Example 2B** | 3.033 | 0.077 | > 30 | / | 39.4 |
| **Example 3A** | > 30 | 9.081 | > 30 | / | > 3.3 |
| **Example 3B** | 3.614 | 1.855 | > 30 | / | 1.9 |
| **Example 5A** | > 10 | 0.0092 | 17.93 | / | > 1086 |
| **Example 5B** | / | 0.017 | > 30 | / | / |
| **Example 6A** | 7.29 | 0.0056 | > 30 | / | 1301 |
| **Example 6B** | / | 0.023 | > 30 | / | / |
| **Example 7A** | / | 0.213 | > 30 | / | / |
| **Example 7B** | / | 0.041 | > 30 | / | / |
| **Example 8A** | / | 0.036 | > 30 | / | / |
| **Example 8B** | / | 0.065 | > 30 | / | / |
| **Example 9A** | 9.502 | 0.0079 | > 30 | / | 1202 |
| **Example 9B** | / | 0.071 | > 30 | / | / |
| **Example 10A** | / | 0.034 | > 30 | / | / |
| **Example 10B** | / | 0.013 | > 30 | / | / |
| **Example 11A** | 5.827 | 0.0091 | > 30 | / | 640 |
| **Example 11B** | / | 0.071 | > 30 | / | / |
| **Example 12A** | 6.073 | 0.0035 | > 30 | / | 1735 |
| **Example 12B** | / | 0.049 | > 30 | / | / |
| **Example 13A** | / | 0.764 | > 30 | / | / |
| **Example 13B** | / | 1.851 | > 30 | / | / |
| **Example 14A** | 15.76 | 0.3663 | > 30 | / | 43 |
| **Example 14B** | / | 1.2860 | > 30 | / | / |
| **Example 15A** | / | 0.0680 | > 30 | / | / |
| **Example 15B** | / | 0.0480 | > 30 | / | / |
| **Example 16A** | 5.829 | 0.03536 | > 30 | / | 165 |
| **Example 16B** | / | 0.01151 | > 30 | / | / |
| **Example 17A** | > 30 | 0.1328 | > 30 | / | > 226 |
| **Example 17B** | / | 2.92 | > 30 | / | / |
| **Example 18A** | > 30 | 0.2029 | > 30 | / | > 148 |
| **Example 18B** | / | 2.453 | > 30 | / | / |
| **Example 19A** | > 30 | 0.05851 | > 30 | / | > 513 |
| **Example 19B** | / | 2.536 | > 30 | / | / |
| **Example 20A** | / | / | / | / | / |
| **Example 20B** | / | / | / | / | / |
| **Example 21A** | / | / | / | / | / |
| **Example 21B** | / | / | / | / | / |
| **Example 22** | / | 1.819 | / | / | / |
| **Example 23A** | / | > 30 | / | / | / |
| **Example 23B** | / | 18 | / | / | / |
| **Example 24A** | / | > 30 | / | / | / |
| **Example 24B** | / | > 30 | / | / | / |
| **Example 25A** | / | > 30 | / | / | / |
| **Example 25B** | / | > 30 | / | / | / |

Wherein "/" indicates that it is not tested or empty.

As can be seen from Table 1:
1) Compared with the control compound R848 and the compound of example 1, the compound of the present invention has a good selective TLR7 agonistic activity (relative to TLR8 agonistic activity), or has an ultra-high TLR8 selective agonistic activity (relative to TLR7 agonistic activity);
2) From the CC₅₀ data in Table 1, it can be seen that the compound of the present invention has lower cytotoxicity than the control compound R848, thereby having better safety.

All literatures mentioned in the present invention are incorporated by reference herein, as though individually incorporated by reference. Additionally, it should be understood that after reading the above teaching, many variations and modifications may be made by the skilled in the art, and these equivalents also fall within the scope as defined by the appended claims.

## Claims

1. An imidazoquinoline-substituted phosphoric ester compound having a structure of general formula (I), or a stereoisomer, a tautomer, a crystal form, or a pharmaceutically acceptable salt, a hydrate, a solvate or a prodrug thereof: 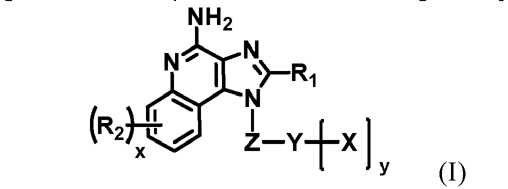 wherein:
R₁ is selected from the substituted or unsubstituted group consisting of hydrogen, C1-C18 alkyl, deuterated C1-C18 alkyl, halogenated C1-C18 alkyl, C1-C18 alkoxy, deuterated C1-C18 alkoxy, halogenated C1-C18 alkoxy, halogen, amino, nitro, hydroxyl, cyano, C3-C18 cycloalkyl, heterocyclyl, C6-C10 aryl, heteroaryl, -(CH₂)ₙOR₅, -(CH₂)ₙO(CH₂)ₘR₅, -(CH₂)ₙSR₅, -(CH₂)ₙCOR₅, -(CH₂)ₙC(O)OR₅, -(CH₂)ₙS(O)ₘR₅, -(CH₂)ₙNR₆R₇, -(CH₂)ₙC(O)NR₆R₇, -(CH₂)ₙNR₆C(O)R₅, and -(CH₂)ₙNR₆S(O)ₘR₅; the substituted means to be substituted by one or more groups selected from the group consisting of hydrogen, deuterium, C1-C18 alkyl, deuterated C1-C18 alkyl, halogenated C1-C18 alkyl, C1-C18 alkoxy, deuterated C1-C18 alkoxy, halogenated C1-C18 alkoxy, halogen, amino, nitro, hydroxyl, cyano, C3-C18 cycloalkyl, heterocyclyl, C6-C10 aryl, heteroaryl, -(CH₂)ₙOR₅, -(CH₂)ₙSR₅, -(CH₂)ₙCOR₅, -(CH₂)ₙC(O)OR₅, -(CH₂)ₙS(O)ₘR₅, -(CH₂)ₙNR₆R₇, -(CH₂)ₙC(O)NR₆R₇, -(CH₂)ₙC(O)NHR₆, -(CH₂)ₙNR₆C(O)R₅, and -(CH₂)ₙNR₆S(O)ₘR₅;
R₂ is each the same or different, and is independently selected from the group consisting of hydrogen, deuterium, C1-C18 alkyl, deuterated C1-C18 alkyl, halogenated C1-C18 alkyl, C1-C18 alkoxy, deuterated C1-C18 alkoxy, halogenated C1-C18 alkoxy, halogen, amino, nitro, hydroxyl, cyano, C3-C18 cycloalkyl, heterocyclyl, C6-C10 aryl, heteroaryl, -(CH₂)ₙOR₅, -(CH₂)ₙSR₅, -(CH₂)ₙCOR₅, -(CH₂)ₙC(O)OR₅, -(CH₂)ₙS(O)ₘR₅, -(CH₂)ₙNR₆R₇, -(CH₂)ₙC(O)NR₆R₇, -(CH₂)ₙC(O)NHR₆, -(CH₂)ₙNR₆C(O)R₅, and -(CH₂)ₙNR₆S(O)ₘR₅;
Z is selected from the substituted or unsubstituted group consisting of C1-C18 alkylene, deuterated C1-C18 alkylene, halogenated C1-C18 alkylene, C1-C18 alkyleneoxy, halogenated C1-C18 alkyleneoxy, C3-C18 cycloalkylene, C1-C18 alkylene C3-C18 cycloalkylene, C3-C18 cycloalkylene C1-C18 alkylene, C1-C18 alkylene C3-C18 cycloalkylene C1-C18 alkylene, heterocyclylene, C6-C10 arylene, and heteroarylene, wherein the substituted means to be substituted by one or more groups selected from the group consisting of hydrogen, deuterium, C1-C18 alkyl, deuterated C1-C18 alkyl, halogenated C1-C18 alkyl, C1-C18 alkoxy, deuterated C1-C18 alkoxy, halogenated C1-C18 alkoxy, halogen, amino, nitro, hydroxyl, cyano, C3-C18 cycloalkyl, heterocyclyl, C6-C10 aryl, heteroaryl, -(CH₂)ₙOR₅, -(CH₂)ₙSR₅, -(CH₂)ₙCOR₅, -(CH₂)ₙC(O)OR₅, -(CH₂)ₙS(O)ₘR₅, -(CH₂)ₙNR₆R₇, -(CH₂)ₙC(O)NR₆R₇, -(CH₂)ₙC(O)NHR₆, -(CH₂)ₙNR₆C(O)R₅, and -(CH₂)ₙNR₆S(O)ₘR₅;
Y is selected from O, N or NR₄;
R₄ is selected from the substituted or unsubstituted group consisting of hydrogen, C1-C18 alkyl, deuterated C1-C18 alkyl, halogenated C1-C18 alkyl, C1-C18 alkoxy, deuterated C1-C18 alkoxy, halogenated C1-C18 alkoxy, C3-C18 cycloalkyl, heterocyclyl, C6-C10 aryl, and heteroaryl, wherein the substituted means to be substituted by one or more groups selected from the group consisting of hydrogen, deuterium, C1-C18 alkyl, deuterated C1-C18 alkyl, halogenated C1-C18 alkyl, C1-C18 alkoxy, deuterated C1-C18 alkoxy, halogenated C1-C18 alkoxy, halogen, amino, nitro, hydroxyl, cyano, C3-C18 cycloalkyl, heterocyclyl, C6-C10 aryl, heteroaryl, -(CH₂)ₙOR₅, -(CH₂)ₙSR₅, -(CH₂)ₙCOR₅, -(CH₂)ₙC(O)OR₅, -(CH₂)ₙS(O)ₘR₅, -(CH₂)ₙNR₆R₇, -(CH₂)ₙC(O)NR₆R₇, -(CH₂)ₙC(O)NHR₆, -(CH₂)ₙNR₆C(O)R₅, and -(CH₂)ₙNR₆S(O)ₘR₅;
R₅ is selected from the substituted or unsubstituted group consisting of hydrogen, deuterium, C1-C18 alkyl, deuterated C1-C18 alkyl, halogenated C1-C18 alkyl, C1-C18 alkoxy, deuterated C1-C18 alkoxy, halogenated C1-C18 alkoxy, amino, hydroxyl, cyano, C3-C18 cycloalkyl, heterocyclyl, C6-C10 aryl, and heteroaryl, wherein the substituted means to be substituted by one or more groups selected from the group consisting of hydrogen, deuterium, C1-C18 alkyl, deuterated C1-C18 alkyl, halogenated C1-C18 alkyl, C1-C18 alkoxy, deuterated C1-C18 alkoxy, halogenated C1-C18 alkoxy, halogen, amino, nitro, hydroxyl, cyano, C3-C18 cycloalkyl, heterocyclyl, C6-C10 aryl, heteroaryl, -(CH₂)ₙOR₈, -(CH₂)ₙSR₈, -(CH₂)ₙCOR₈, -(CH₂)ₙC(O)OR₈, -(CH₂)ₙS(O)ₘR₈, -(CH₂)ₙNR₈R₉, -(CH₂)ₙC(O)NR₈R₉, -(CH₂)ₙC(O)NHR₉, -(CH₂)ₙNR₉C(O)R₈, and -(CH₂)ₙNR₉S(O)ₘR₈;
R₆ and R₇ are the same or different, and are each independently selected from the substituted or unsubstituted group consisting of hydrogen, deuterium, C1-C18 alkyl, deuterated C1-C18 alkyl, halogenated C1-C18 alkyl, C1-C18 alkoxy, deuterated C1-C18 alkoxy, halogenated C1-C18 alkoxy, amino, hydroxyl, C3-C18 cycloalkyl, heterocyclyl, C6-C10 aryl, and heteroaryl; wherein the substituted means to be substituted by one or more groups selected from the group consisting of hydrogen, deuterium, C1-C18 alkyl, deuterated C1-C18 alkyl, halogenated C1-C18 alkyl, C1-C18 alkoxy, deuterated C1-C18 alkoxy, halogenated C1-C18 alkoxy, halogen, amino, nitro, hydroxyl, cyano, C3-C18 cycloalkyl, heterocyclyl, C6-C10 aryl, heteroaryl, -(CH₂)ₙOR₈, -(CH₂)ₙSR₈, -(CH₂)ₙCOR₈, -(CH₂)ₙC(O)OR₈, -(CH₂)ₙS(O)ₘR₈, -(CH₂)ₙNR₈R₉, -(CH₂)ₙC(O)NR₈R₉, -(CH₂)ₙC(O)NHR₉, -(CH₂)ₙNR₉C(O)R₈, and -(CH₂)ₙNR₉S(O)mR₈;
R₈ and R₉ are the same or different, and are each independently selected from the substituted or unsubstituted group consisting of hydrogen, deuterium, C1-C18 alkyl, deuterated C1-C18 alkyl, halogenated C1-C18 alkyl, C1-C18 alkoxy, deuterated C1-C18 alkoxy, halogenated C1-C18 alkoxy, amino, hydroxyl, -COOR₁₆, C3-C18 cycloalkyl, heterocyclyl, C6-C10 aryl, and heteroaryl, wherein the substituted means to be substituted by one or more groups selected from the group consisting of deuterium, C1-C20 alkyl, C1-C6 alkoxy, C3-C10 cycloalkyl, C4-C10 heterocyclyl, C6-C10 aryl, C5-C10 heteroaryl, halogen, amino, nitro, -COOR₁₆, cyano, hydroxyl, amido, and sulfonamido;
X is selected from the group consisting of -P(=O)(OH)₂, -P(=O)(OH)OP(=O)(OH)₂, -P(=O)(OH)OP(=O)(OH)OP(=O)(OH)₂, -P(=O)(X₁R₁₁)(X₂R₁₂), -P(=O)(X₁R₁₁)(X₃R₁₄R₁₅), -P(=O)(X₃R₁₄R₁₅)(X₃R₁₄R₁₅), -CH₂P(=O)(X₁R₁₁)(X₂R₁₂), -CH₂P(=O)(X₁R₁₁)(X₃R₁₄R₁₅), -CH₂P(=O)(X₃R₁₄R₁₅)(X₃R₁₄R₁₅), - P(=S)(X₁R₁₁)(X₂R₁₂), - P(=S)(X₁R₁₁)(X₃R₁₄R₁₅), -P(=S)(X₃P₁₄P₁₅)(X₃R₁₄R₁₅), -CH₂P(=S)(X₁R₁₁)(X₂R₁₂), -CH₂P(=S)(X₁R₁₁)(X₃R₁₄R₁₅), -CH₂P(=S)(X₃R₁₄R₁₅)(X₃R₁₄R₁₅), -P(=NR₁₃)(X₁R₁₁)(X₂R₁₂), -P(=NR₁₃)(X₁R₁₁)(X₃R₁₄R₁₅), -P(=NR₁₃)(X₃R₁₄R₁₅)(X₃R₁₄R₁₅), -CH₂P(=NR₁₃)(X₁R₁₁)(X₂R₁₂), -CH₂P(=NR₁₃)(X₁R₁₁)(X₃R₁₄R₁₅), and -CH₂P(=NR₁₃)(X₃R₁₄R₁₅)(X₃R₁₄R₁₅);
X₁ and X₂ are independently selected from the group consisting of oxygen, sulfur, and -OCH₂O-;
X3 is nitrogen;
R₁₁, R₁₂, R₁₃, R₁₄ and R₁₅ are independently selected from the substituted or unsubstituted group consisting of hydrogen, C1-C20 alkyl, deuterated C1-C20 alkyl, C3-C10 cycloalkyl, C4-C10 heterocyclyl, C6-C10 aryl, and C5-C10 heteroaryl, or R₁₁ and R₁₂ combine with adjacent X₁, X₂ and P to form substituted 5-7-membered heterocyclyl, and the substituted means to be substituted by one or more substituents selected from the group consisting of deuterium, C1-C20 alkyl, halogenated C1-C20 alkyl, C1-C6 alkoxy, C3-C10 cycloalkyl, C4-C10 heterocyclyl, C6-C10 aryl, halogenated C6-C10 aryl, C5-C10 heteroaryl, halogen, amino, nitro, -COR₁₆, -COOR₁₆, -OCOOR₁₆, cyano, hydroxyl, amido, and sulfonamido;
R₁₆ is selected from the substituted or unsubstituted group consisting of hydrogen, C1-C18 alkyl, deuterated C1-C20 alkyl, C3-C10 cycloalkyl, C3-C10 cycloalkenyl, C6-C10 aryl, amino, and heterocyclyl, wherein the substituted means to be substituted by one or more C6-C10 aryl;
x is an integer of 0, 1, 2, 3 or 4;
y is an integer of 1 or 2;
m is an integer of 0, 1 or 2;
and n is an integer of 0, 1, 2, 3, 4 or 5;
and each heterocyclyl is independently 5-15-membered heterocycloalkyl containing 1-3 heteroatoms selected from N, O or S;
each heteroaryl is independently 5-15-membered heteroaryl containing 1-3 heteroatoms selected from N, O or S.

2. The imidazoquinoline-substituted phosphoric ester compound having a structure of general formula (I), or a stereoisomer, a tautomer, a crystal form, or a pharmaceutically acceptable salt, a hydrate, a solvate or a prodrug thereof according to claim 1, wherein it is a compound of general formula (II-1) or (II-2), or a stereoisomer, a tautomer, a crystal form, or a pharmaceutically acceptable salt, a hydrate, a solvate or a prodrug thereof: 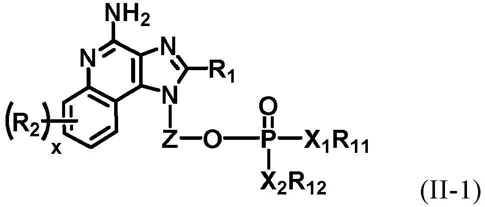 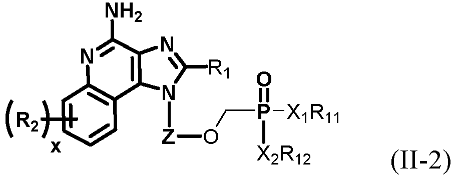 wherein:
R₁, R₂, x, Z, X₁, X₂, R₁₁ and R₁₂ are as described in general formula (I).

3. The imidazoquinoline-substituted phosphoric ester compound having a structure of general formula (I), or a stereoisomer, a tautomer, a crystal form, or a pharmaceutically acceptable salt, a hydrate, a solvate or a prodrug thereof according to claim 1, wherein, it is a compound of general formula (III-1) or (III-2), or a stereoisomer, a tautomer, a crystal form, or a pharmaceutically acceptable salt, a hydrate, a solvate or a prodrug thereof: 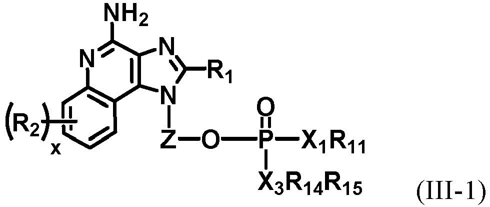 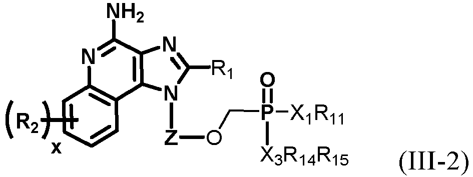 R₁, R₂, x, Z, X₁, X₃, R₁₁, R₁₄ and R₁₅ are as described in general formula (I).

4. The imidazoquinoline-substituted phosphoric ester compound having a structure of general formula (I), or a stereoisomer, a tautomer, a crystal form, or a pharmaceutically acceptable salt, a hydrate, a solvate or a prodrug thereof according to claim 1, wherein Z has a configuration of S or R.

5. The imidazoquinoline-substituted phosphoric ester compound having a structure of general formula (I), or a stereoisomer, a tautomer, a crystal form, or a pharmaceutically acceptable salt, a hydrate, a solvate or a prodrug thereof according to claim 2 or 3, wherein structure P has a configuration of S or R.

6. The imidazoquinoline-substituted phosphoric ester compound having a structure of general formula (I), or a stereoisomer, a tautomer, a crystal form, or a pharmaceutically acceptable salt, a hydrate, a solvate or a prodrug thereof according to claim 1, wherein the compound is selected from the group consisting of

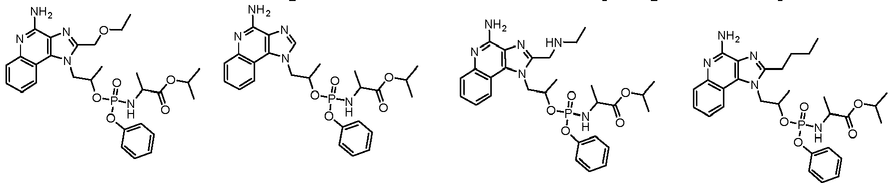

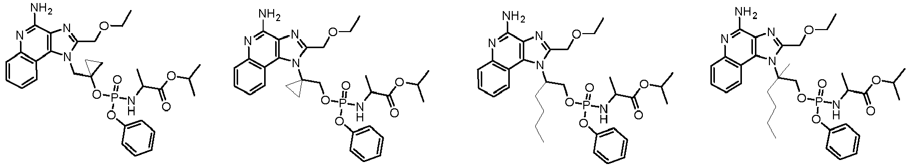

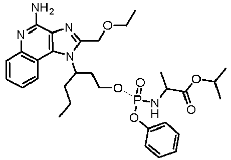

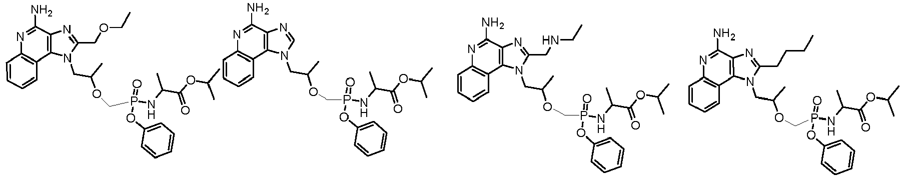

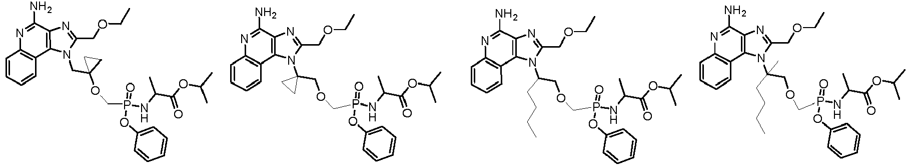

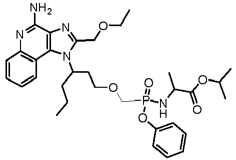

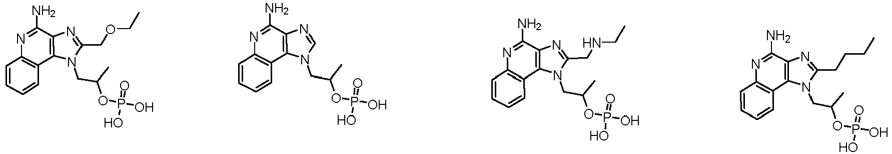

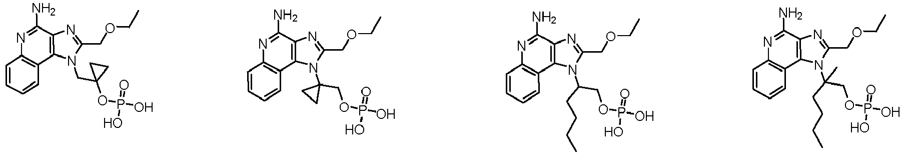

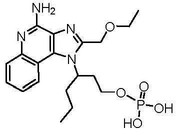

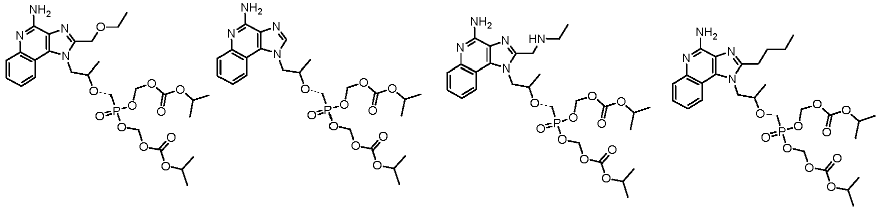

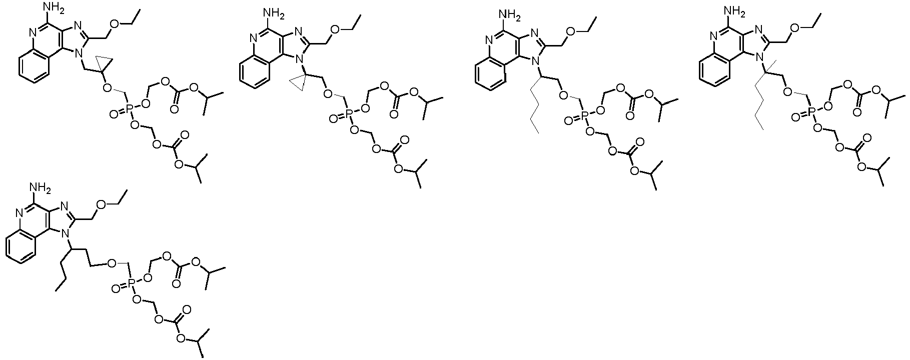

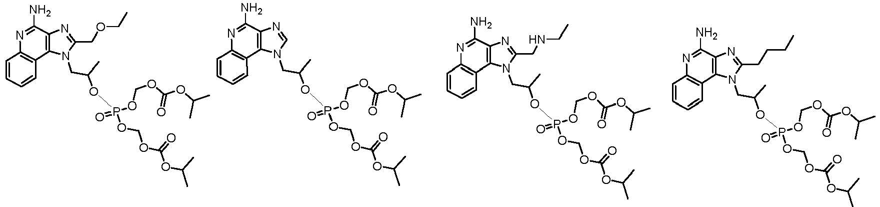

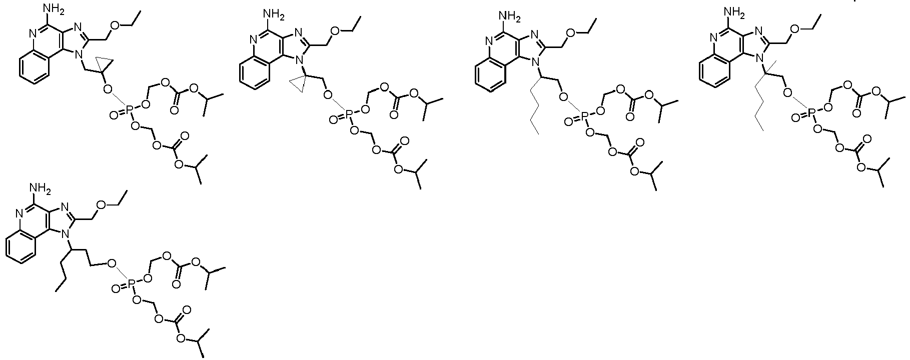

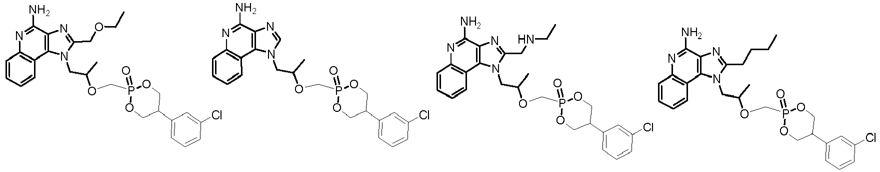

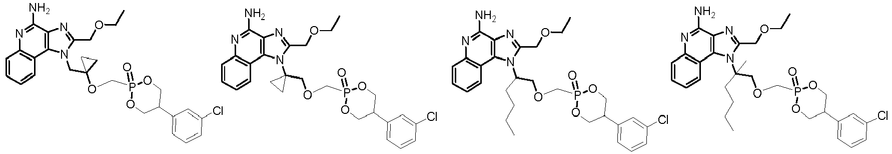

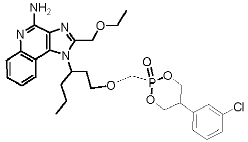

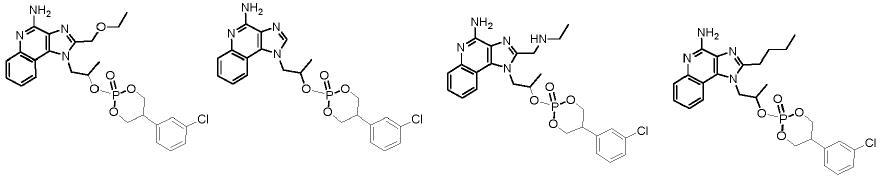

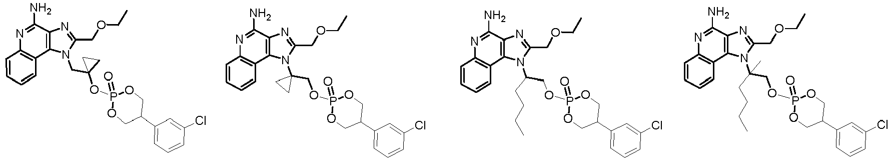

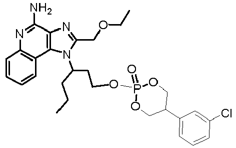

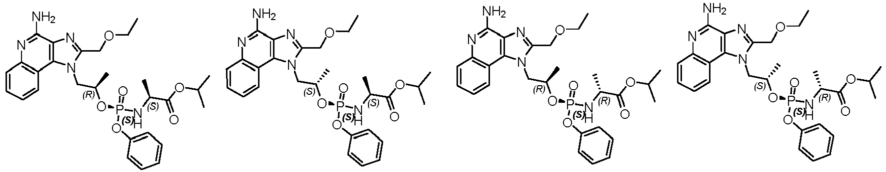

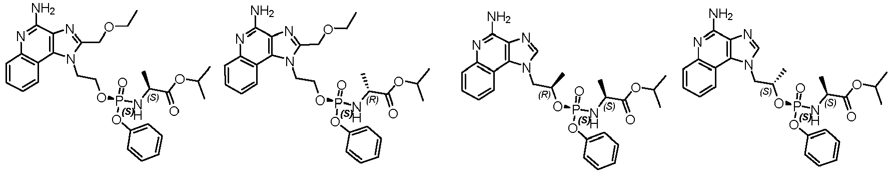

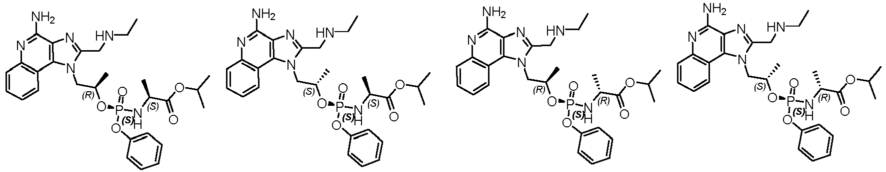

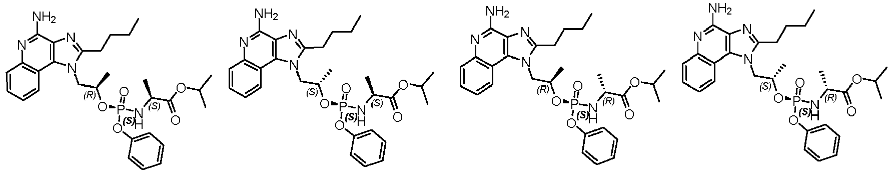

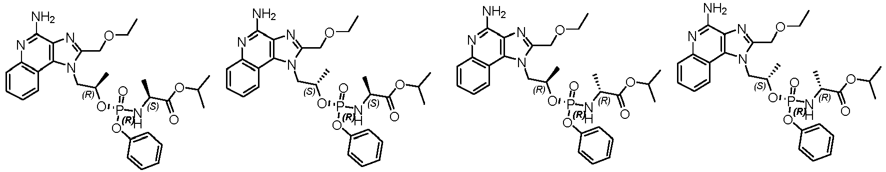

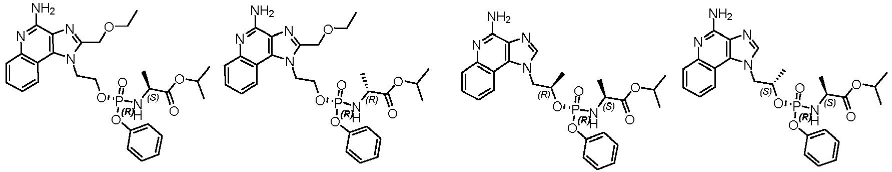

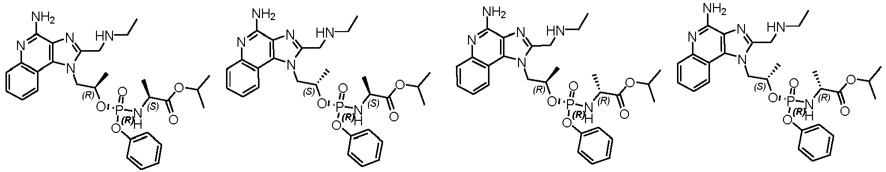

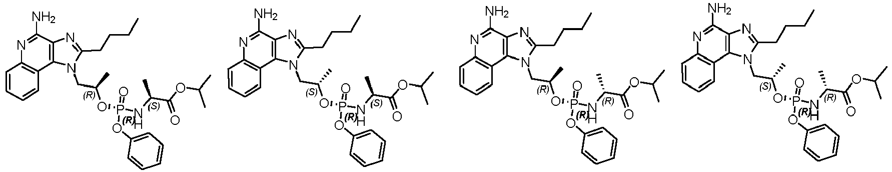

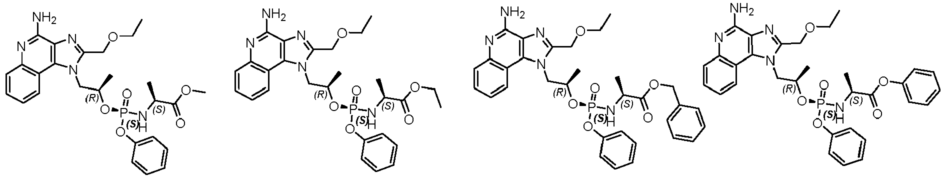

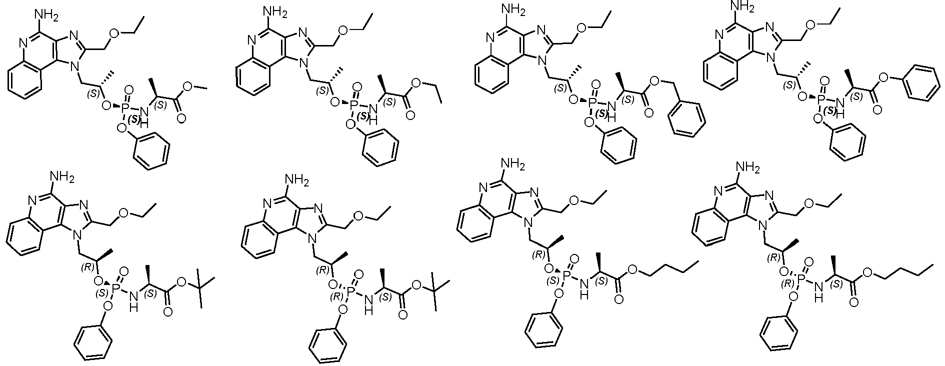

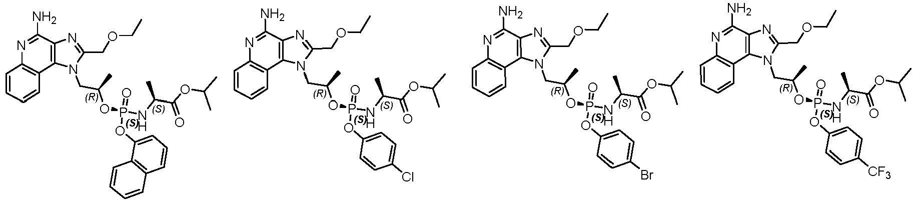

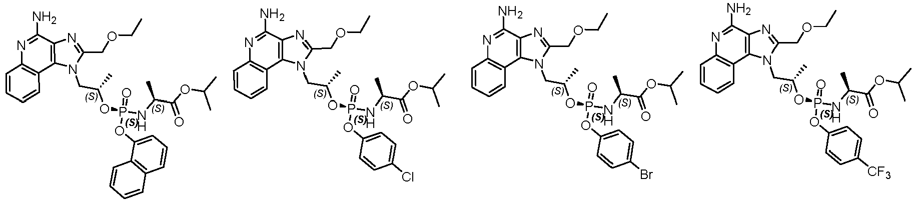

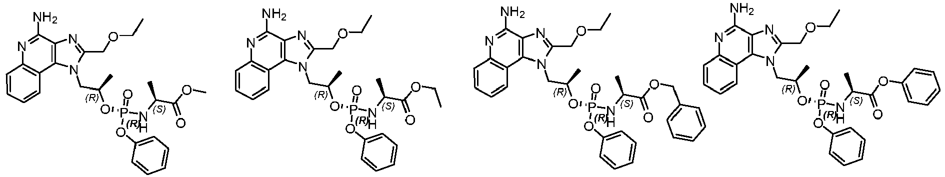

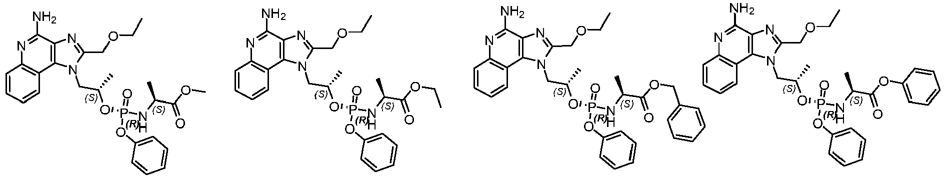

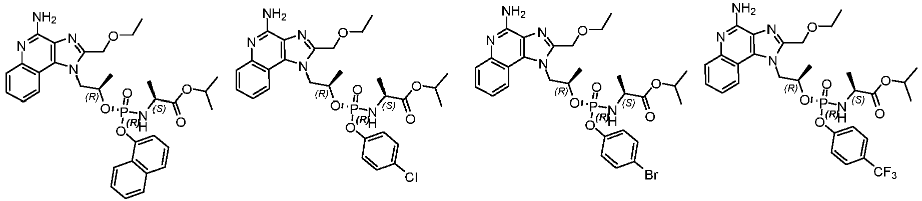

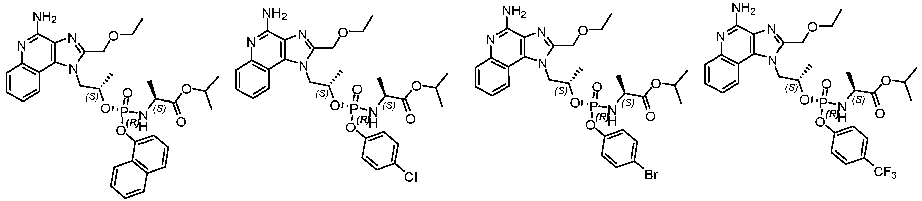

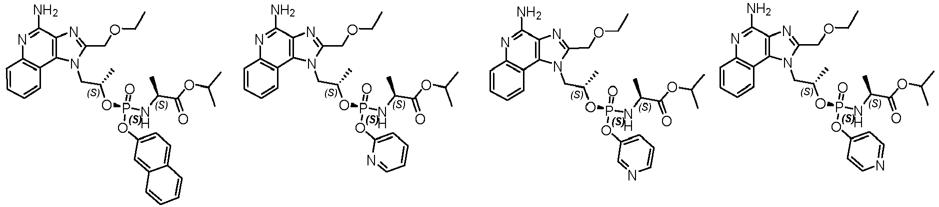

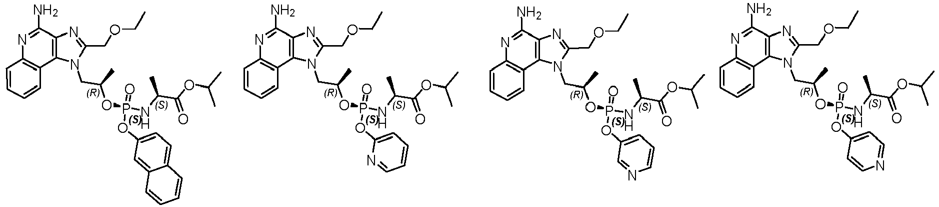

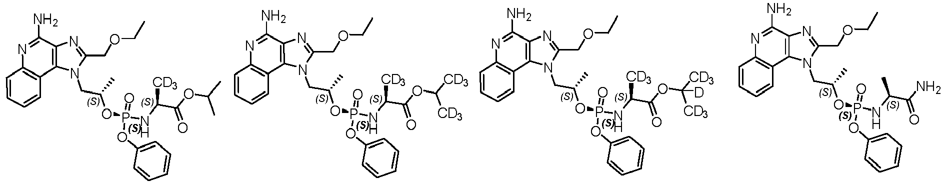

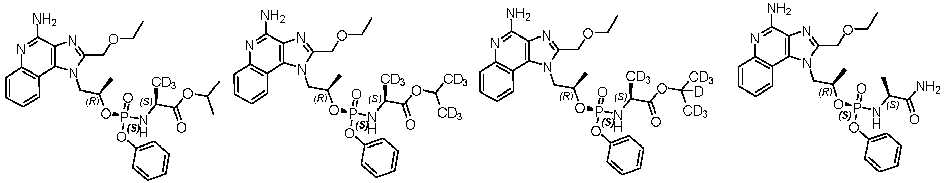

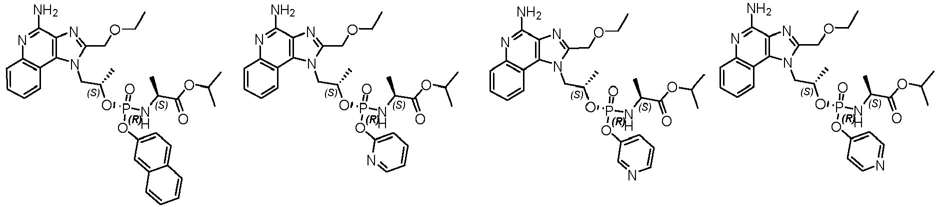

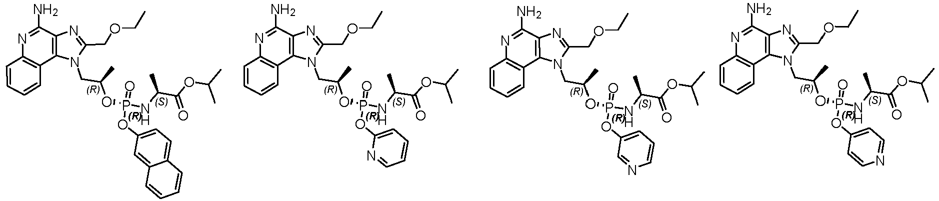

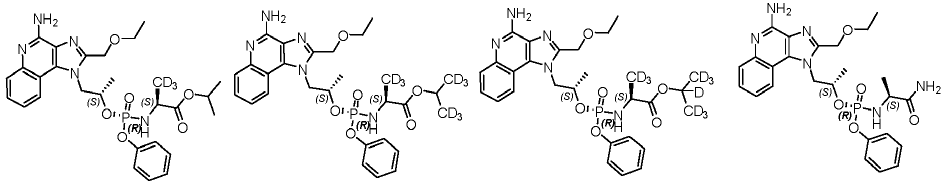

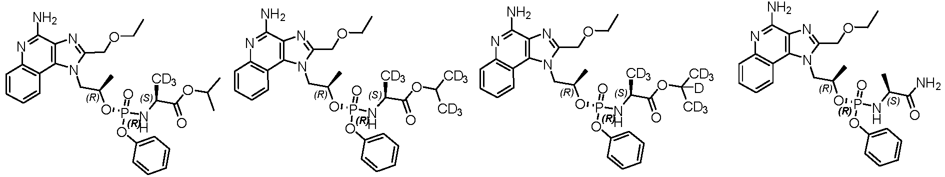

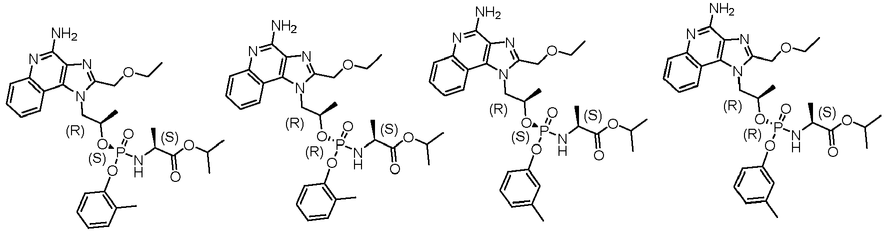

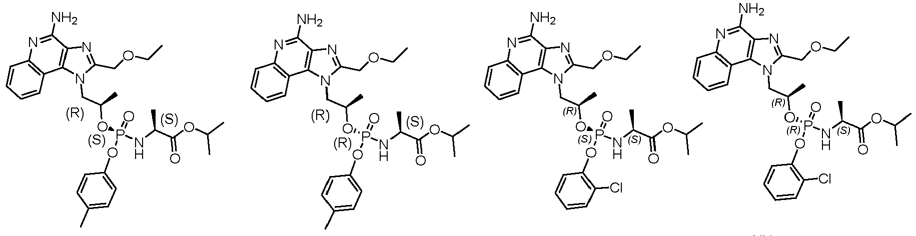

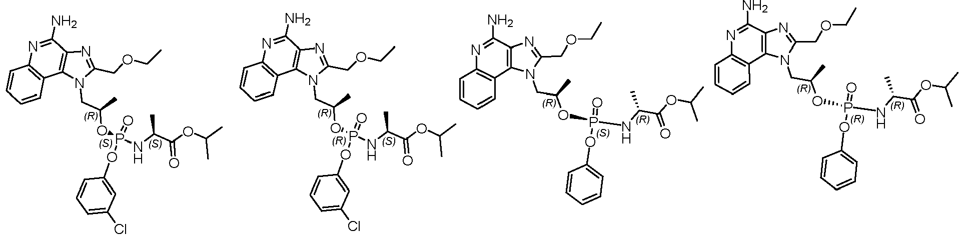

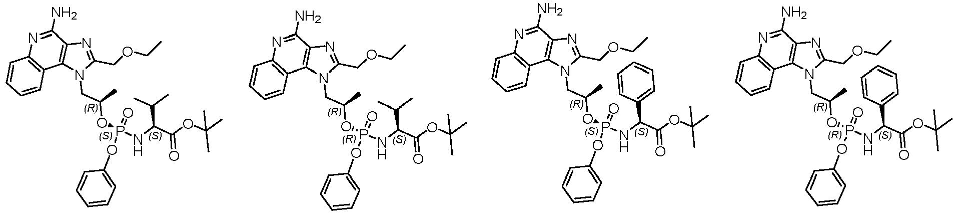

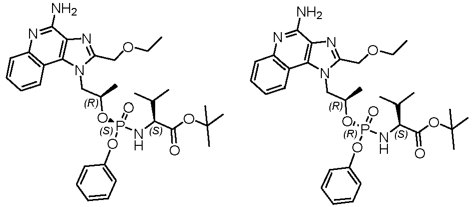

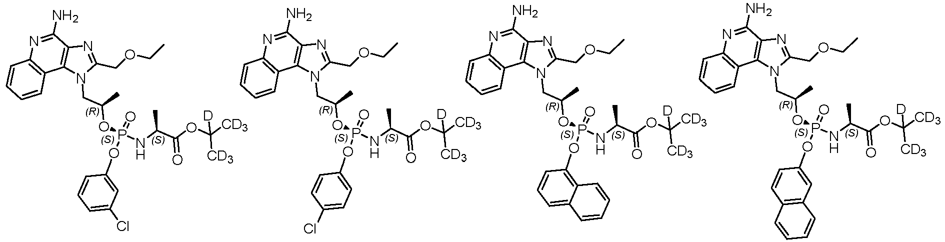

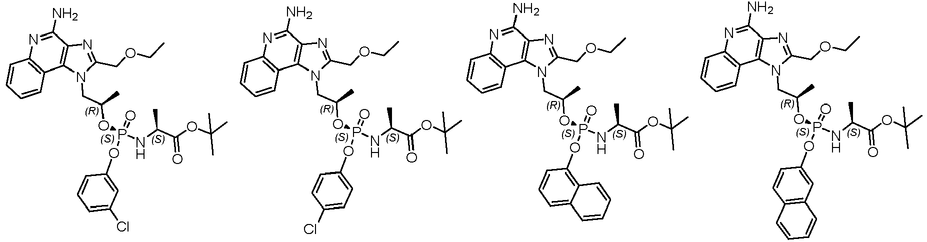

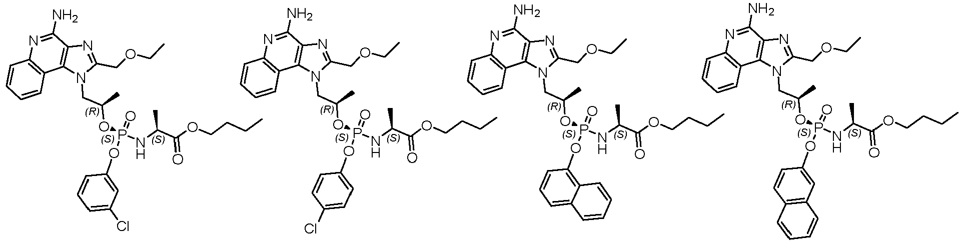

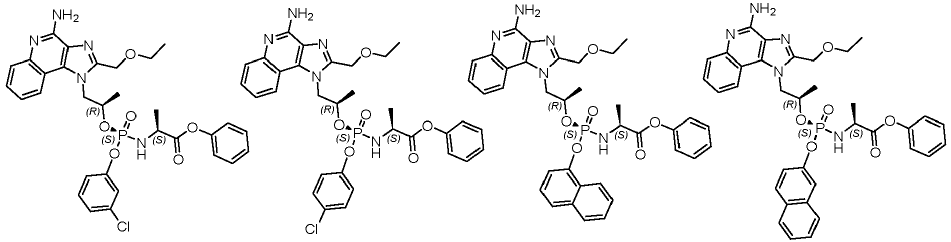

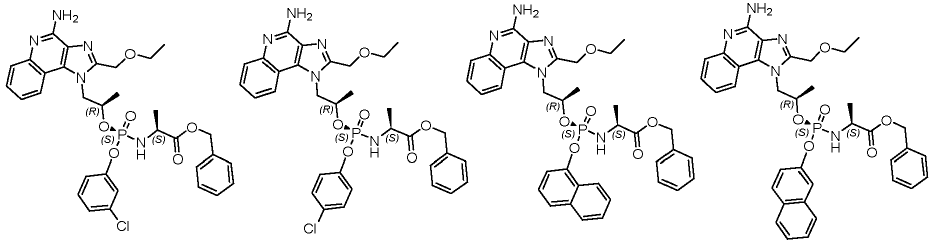

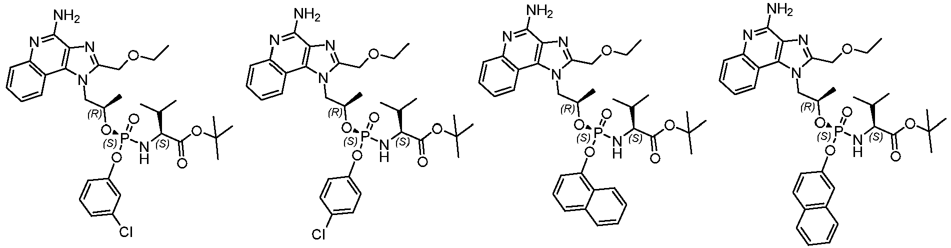

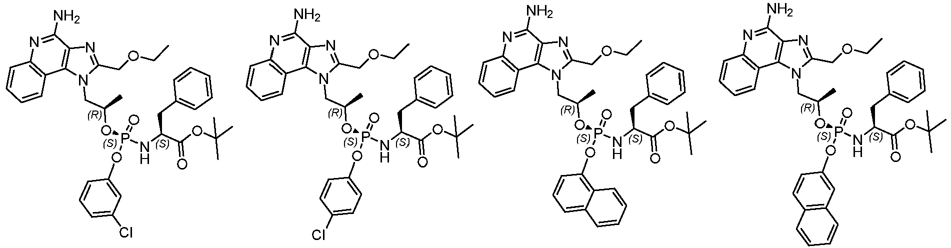

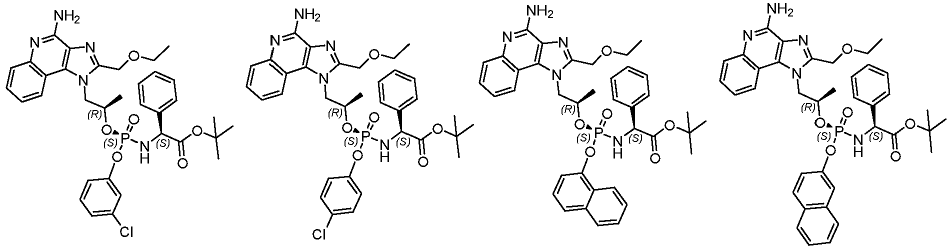

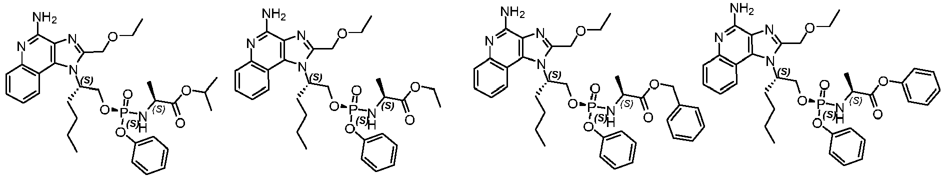

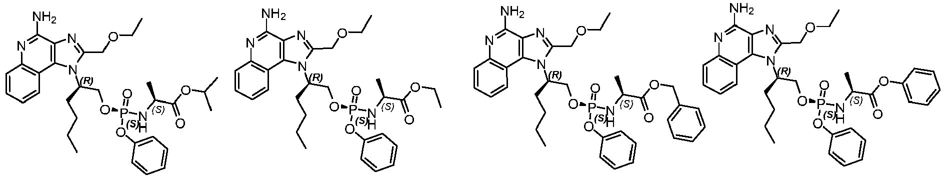

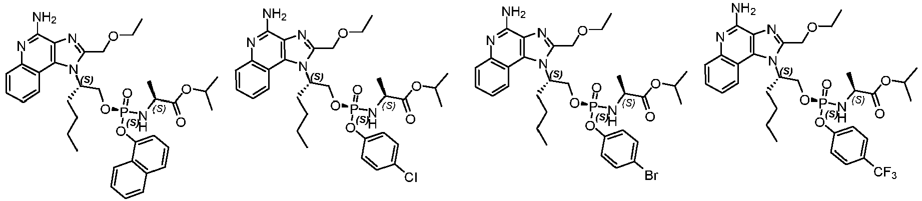

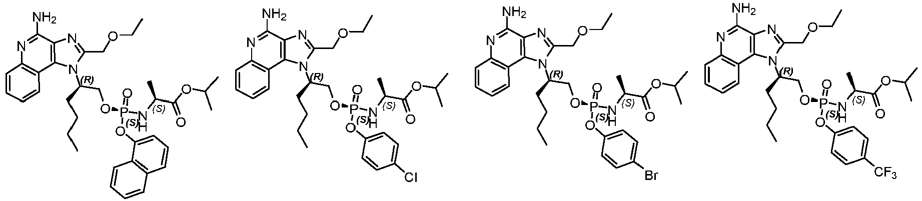

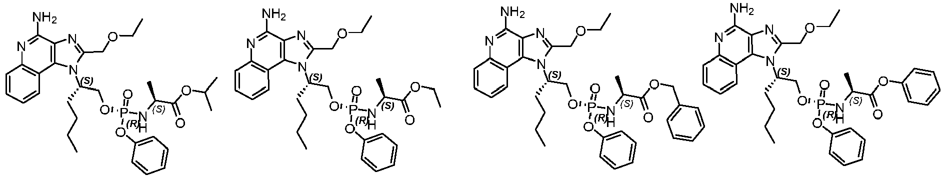

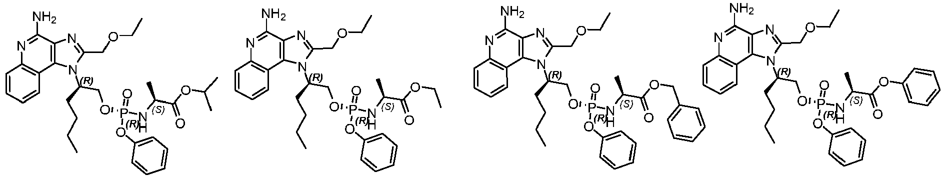

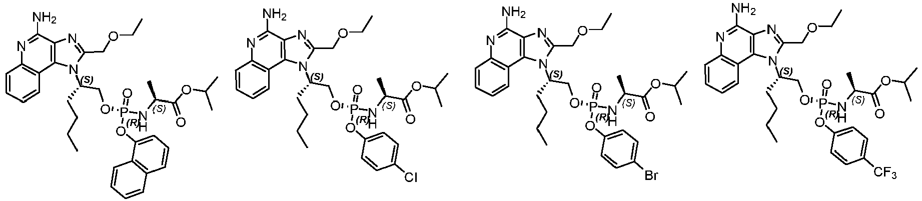

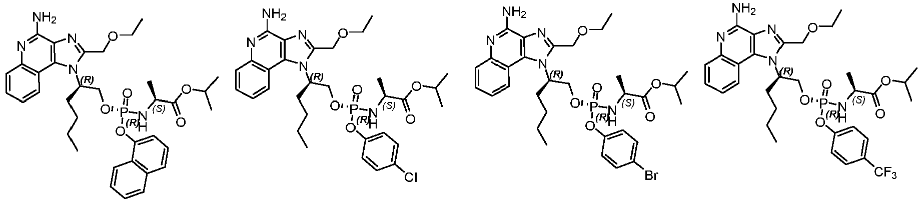

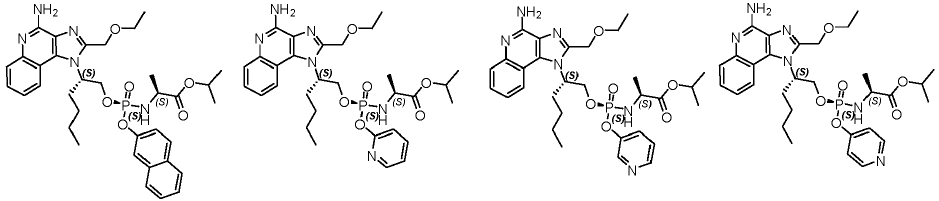

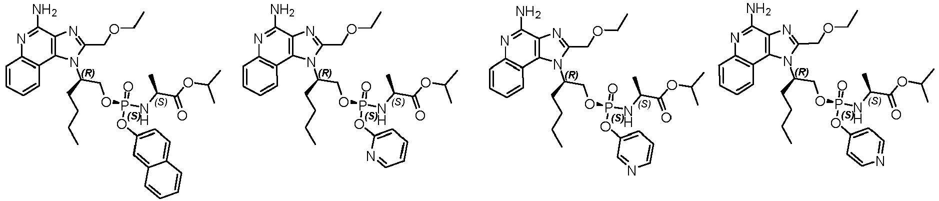

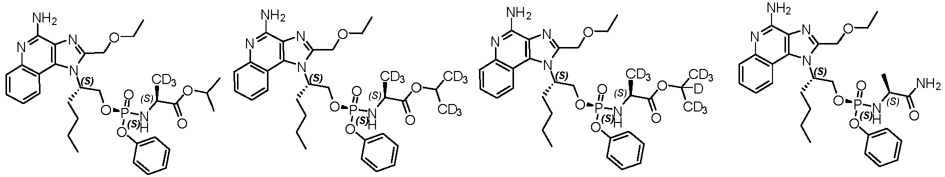

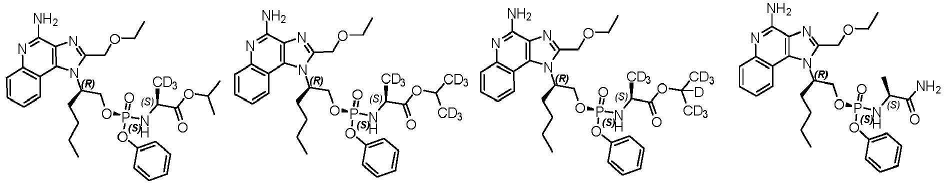

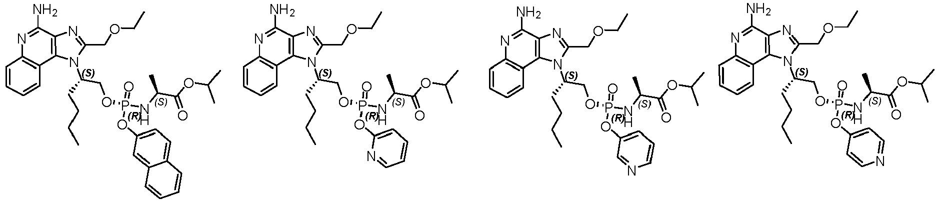

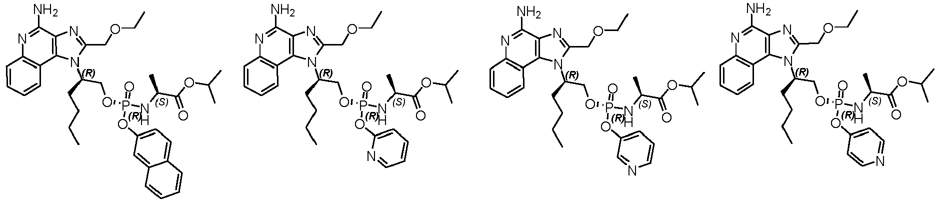

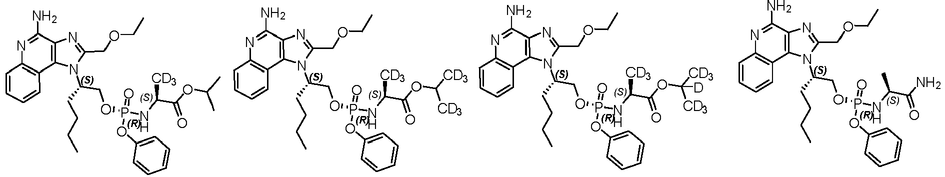

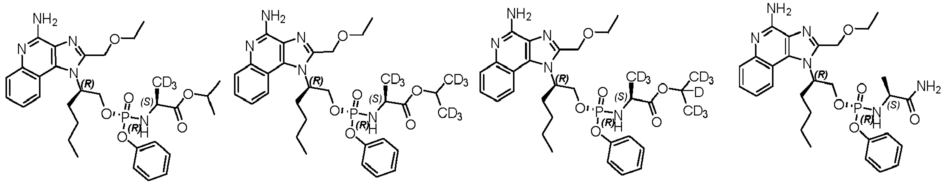

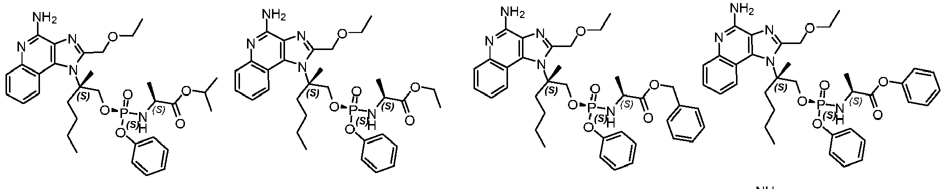

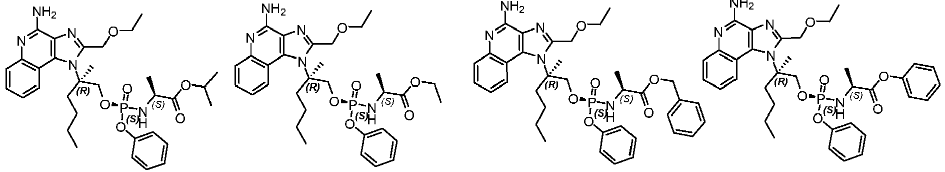

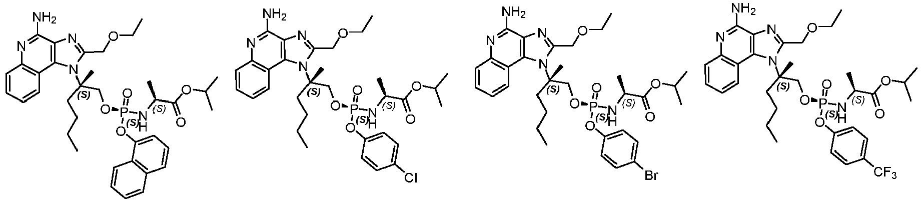

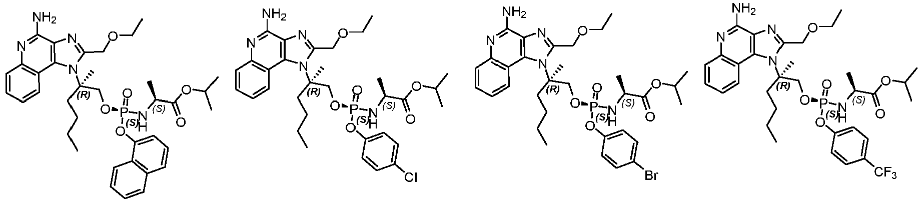

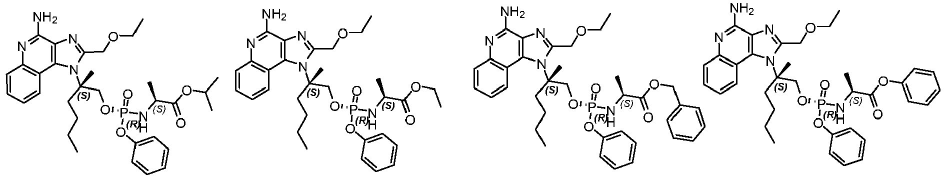

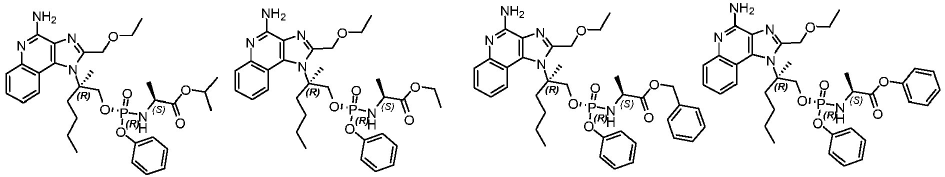

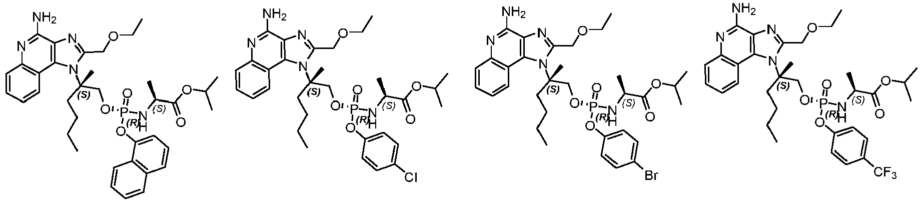

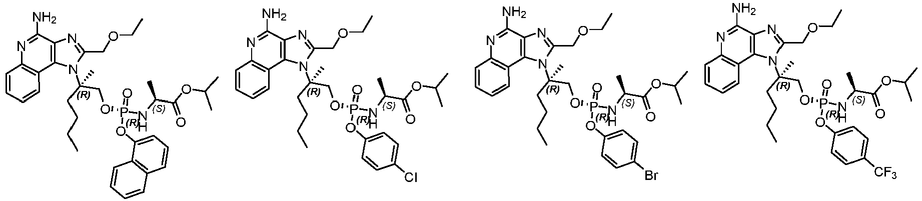

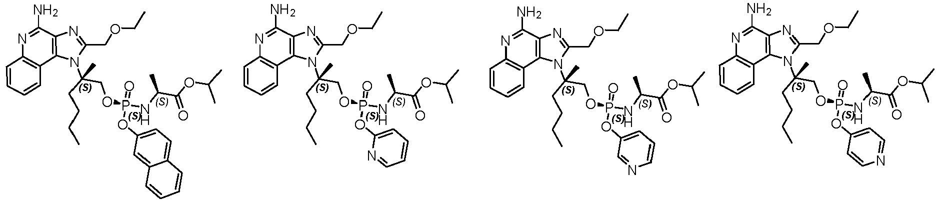

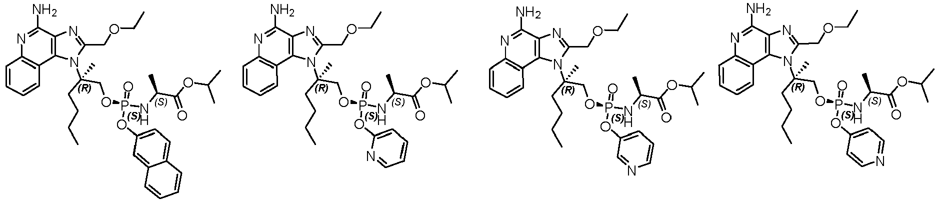

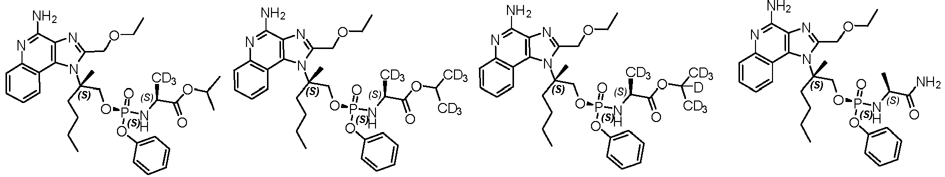

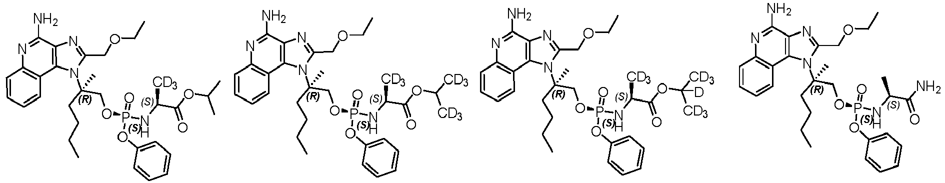

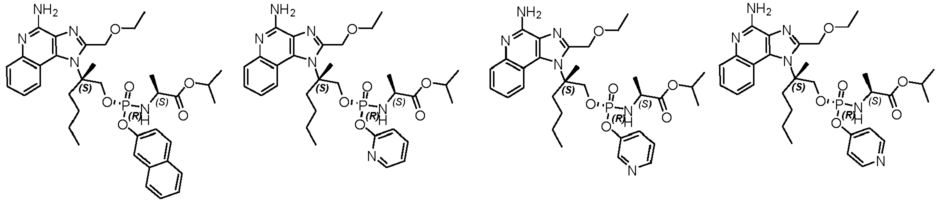

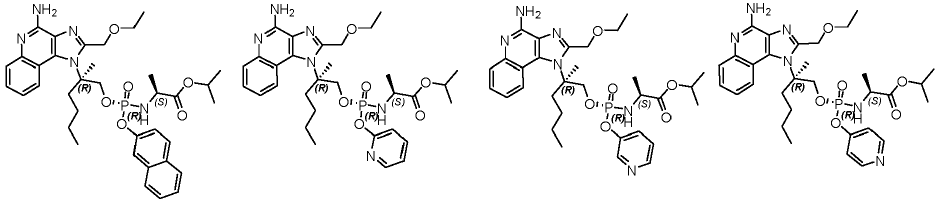

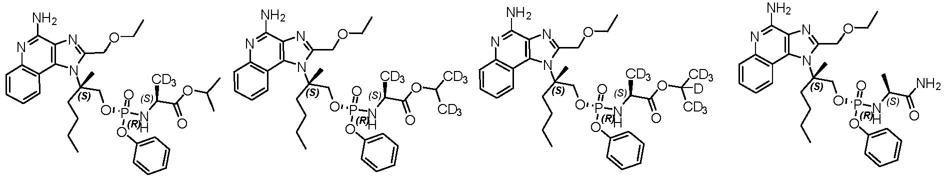

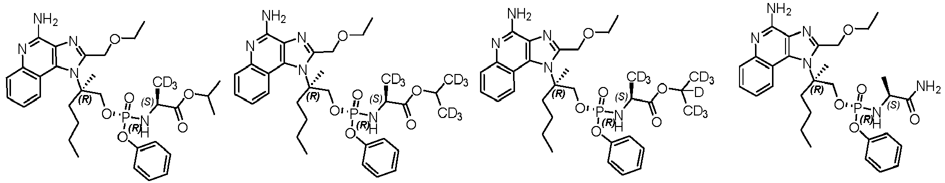

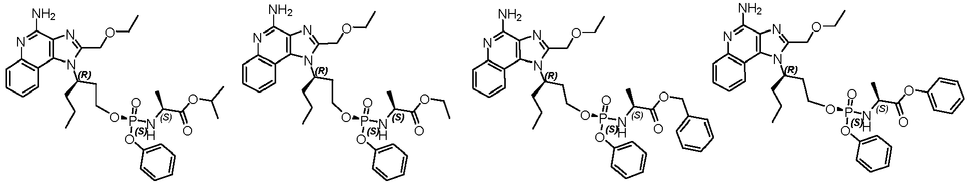

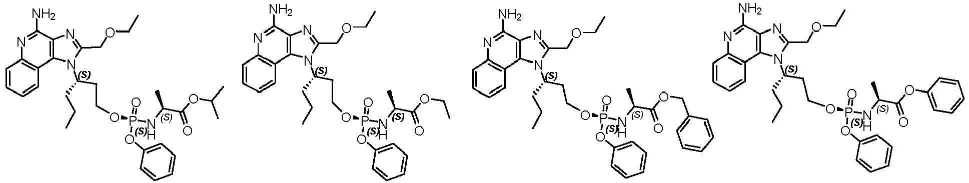

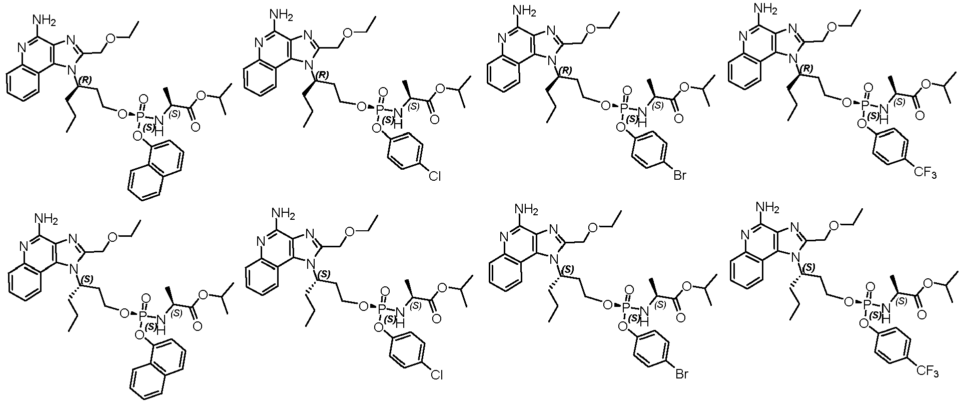

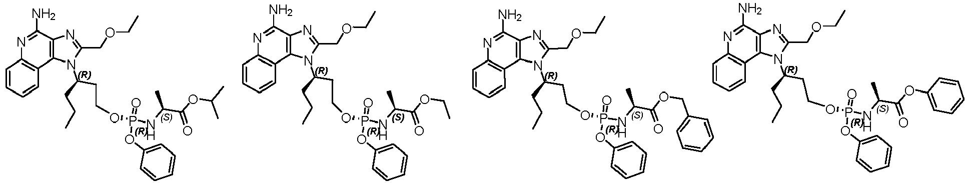

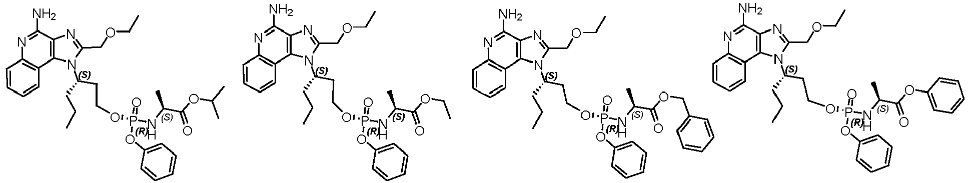

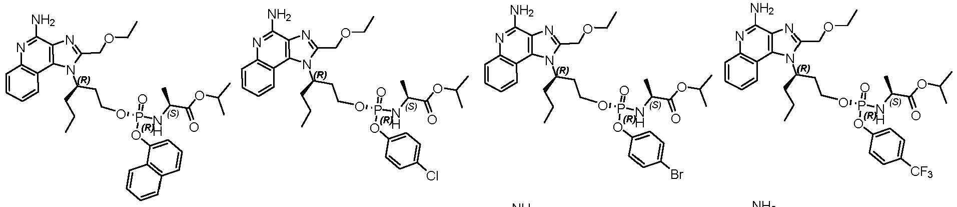

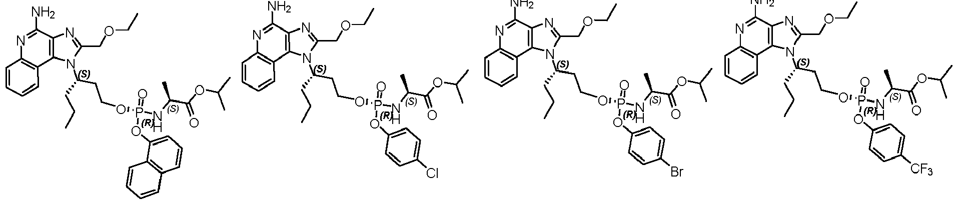

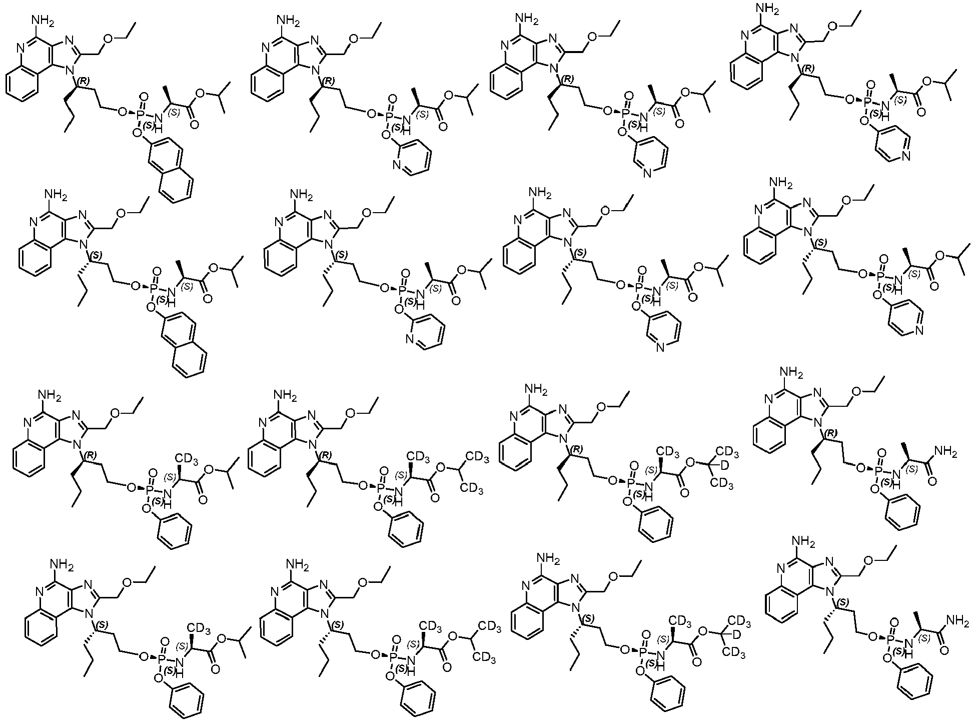

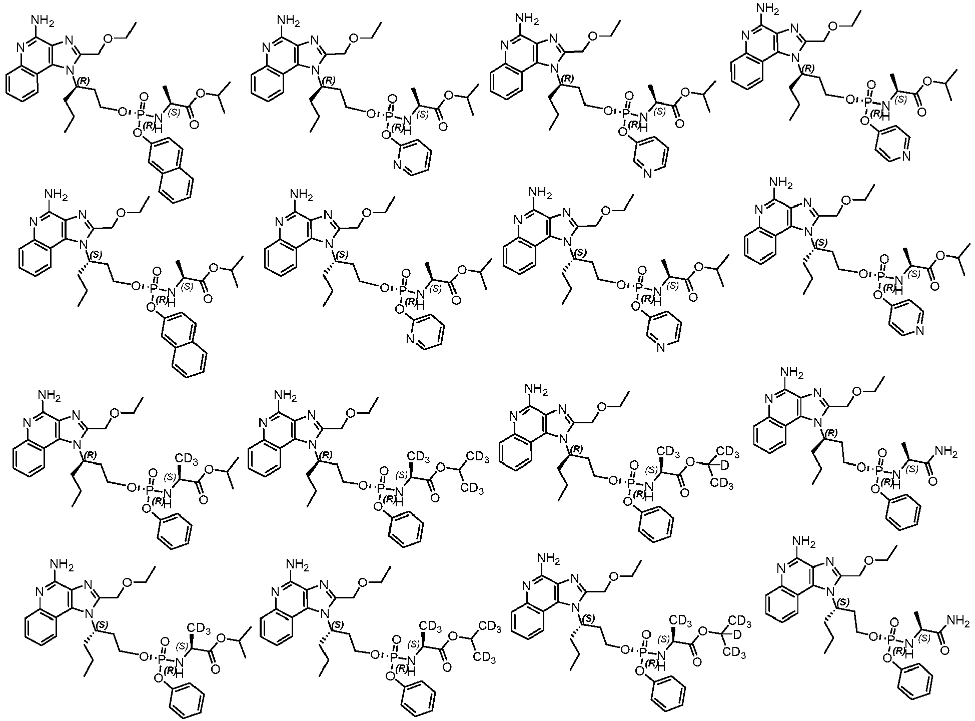

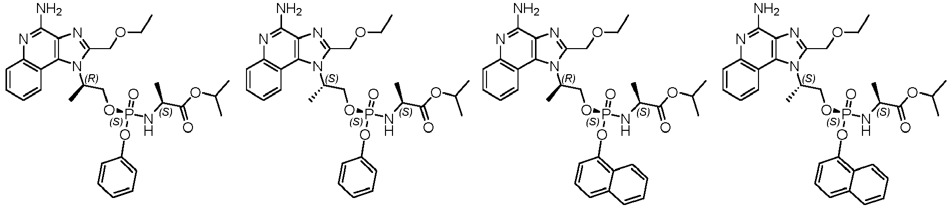

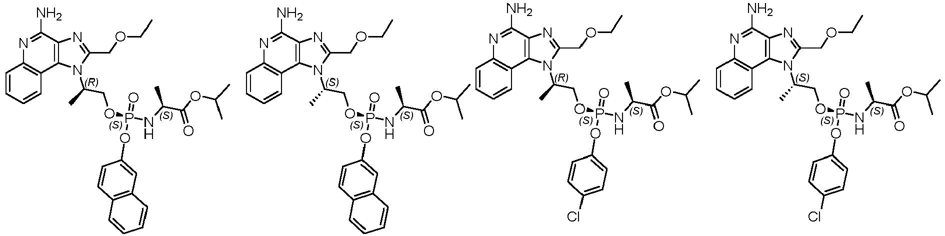

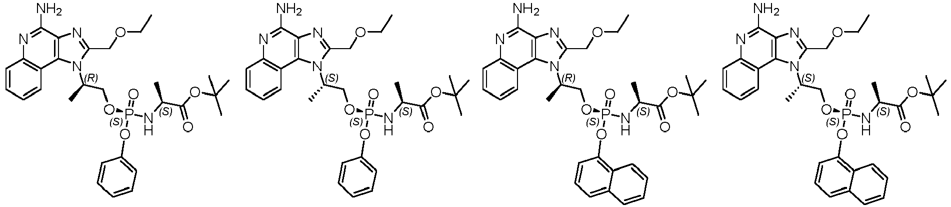

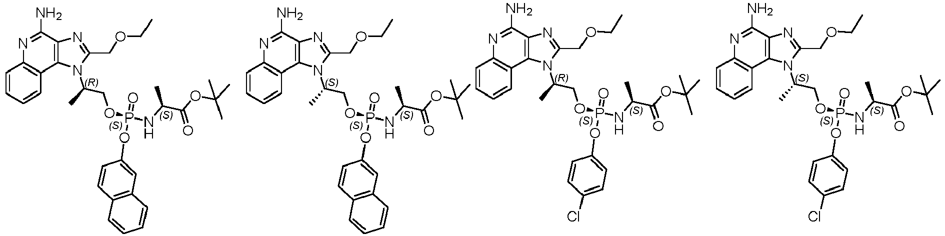

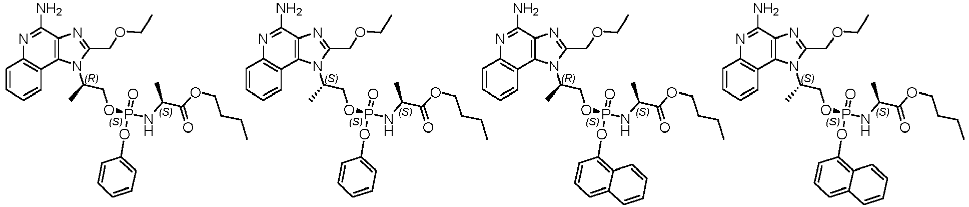

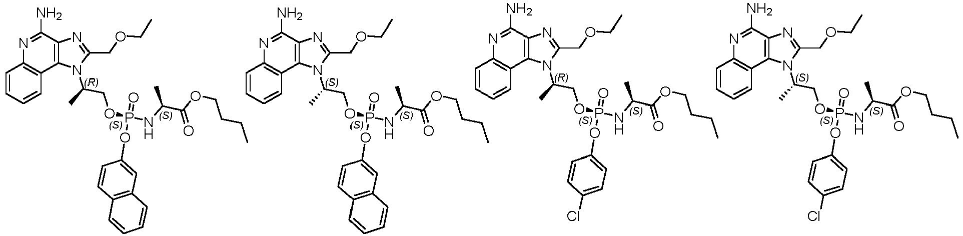

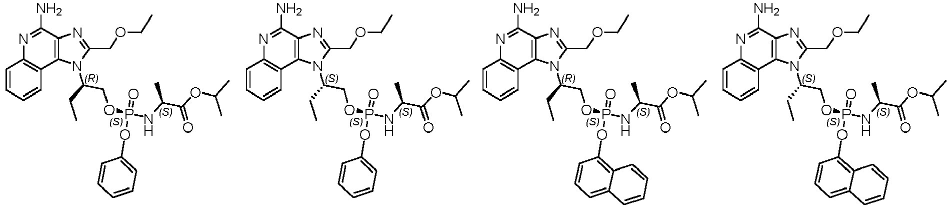

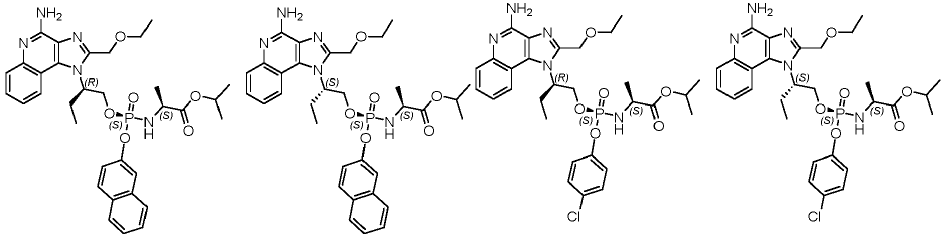

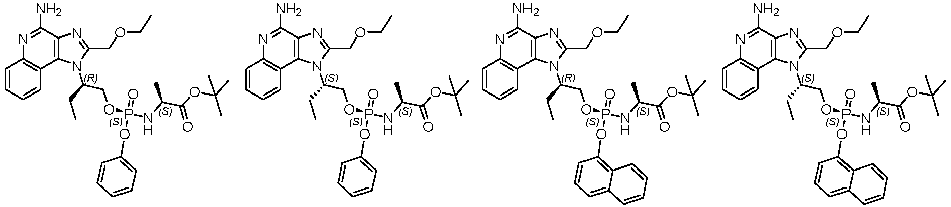

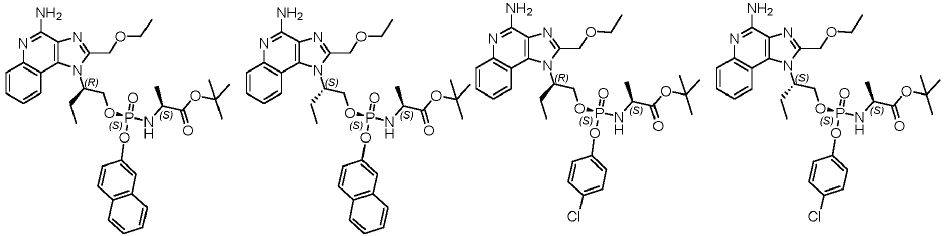

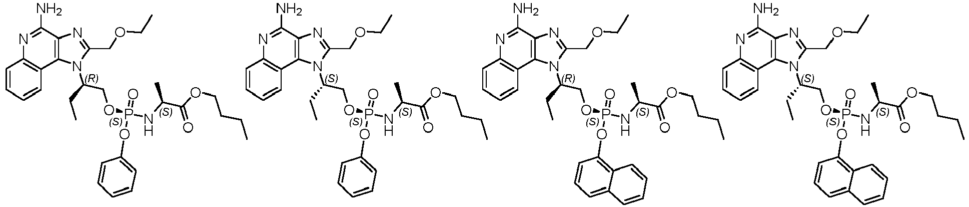

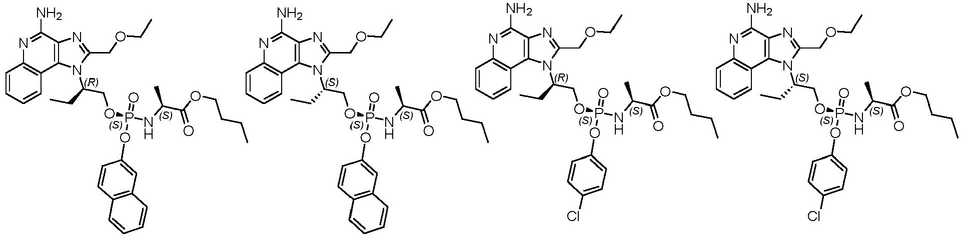

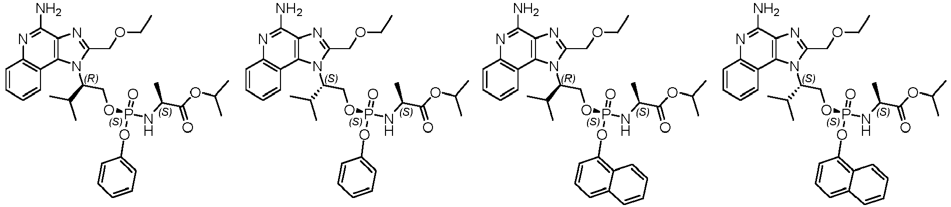

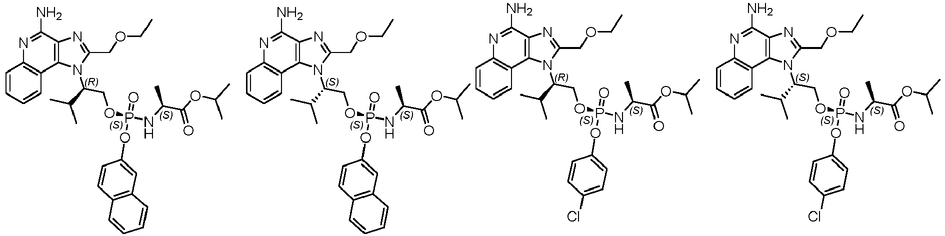

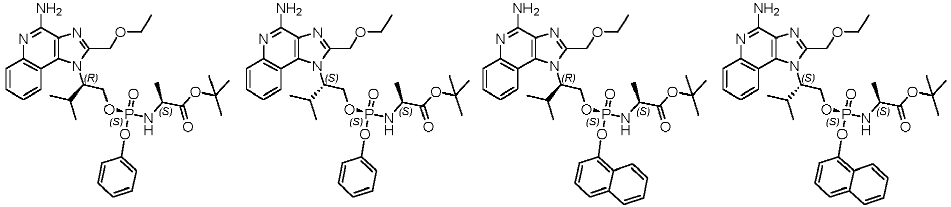

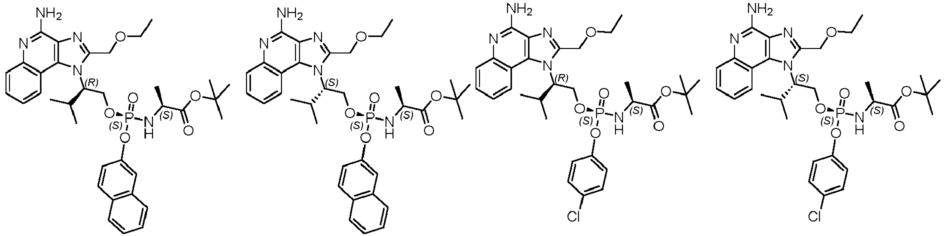

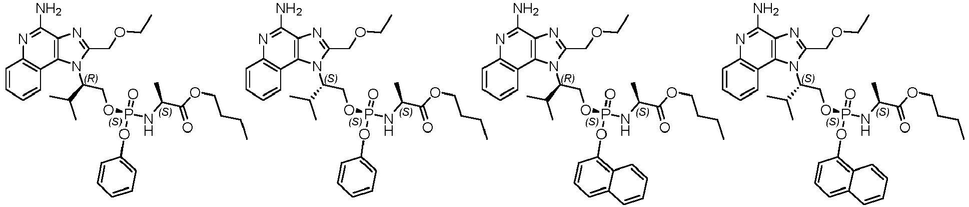

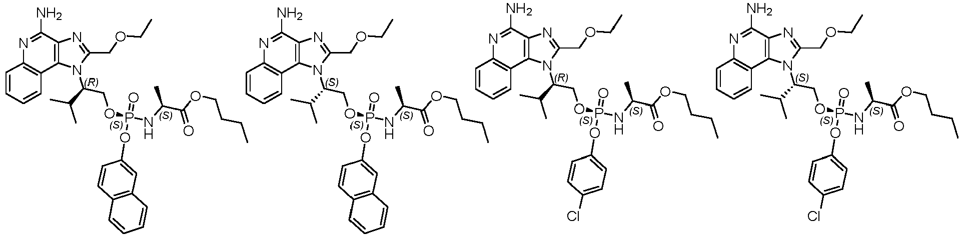

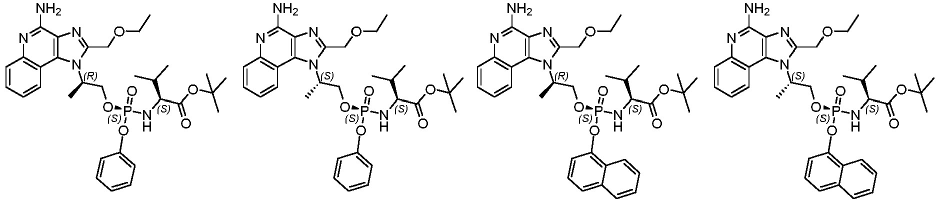

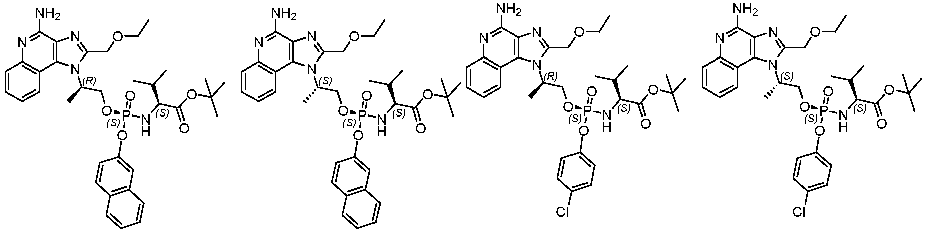

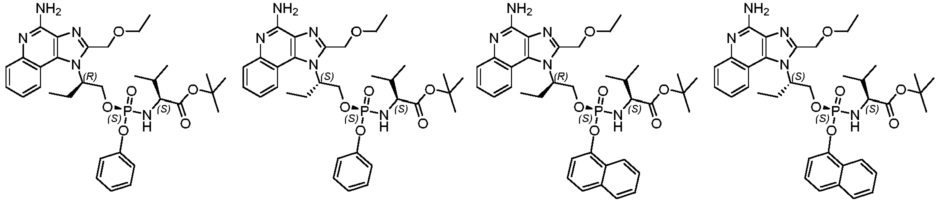

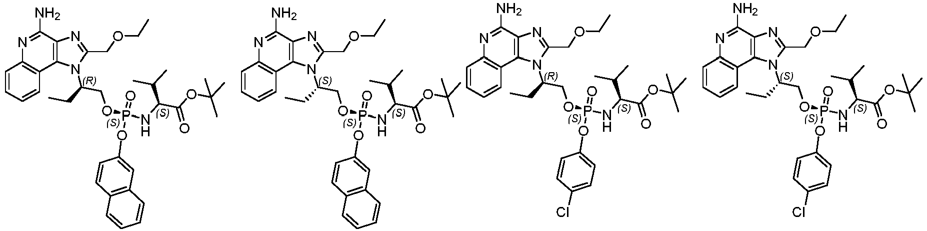

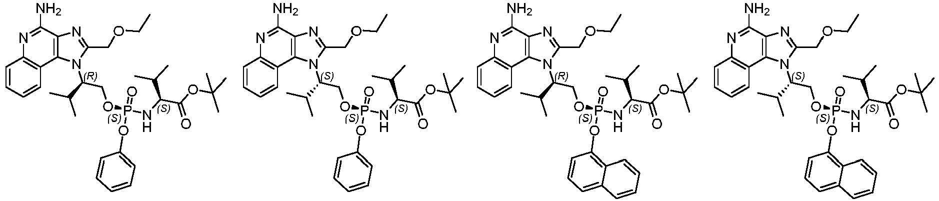

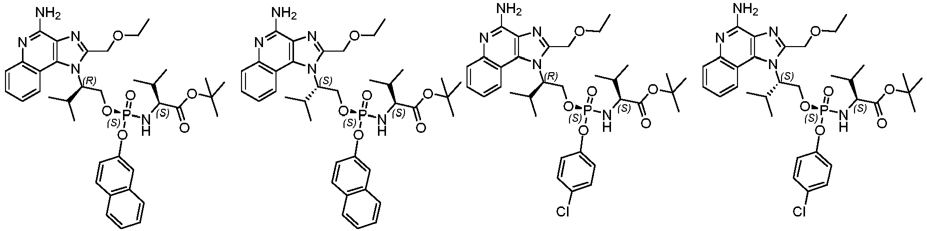

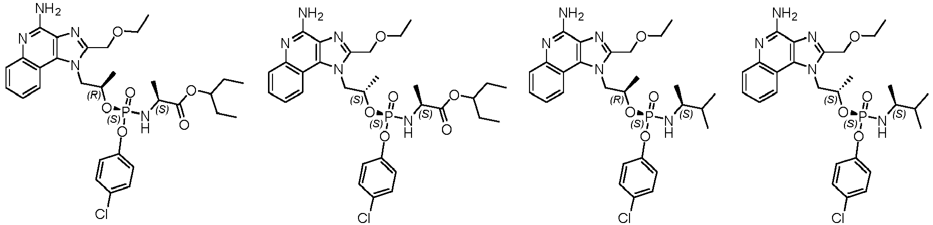

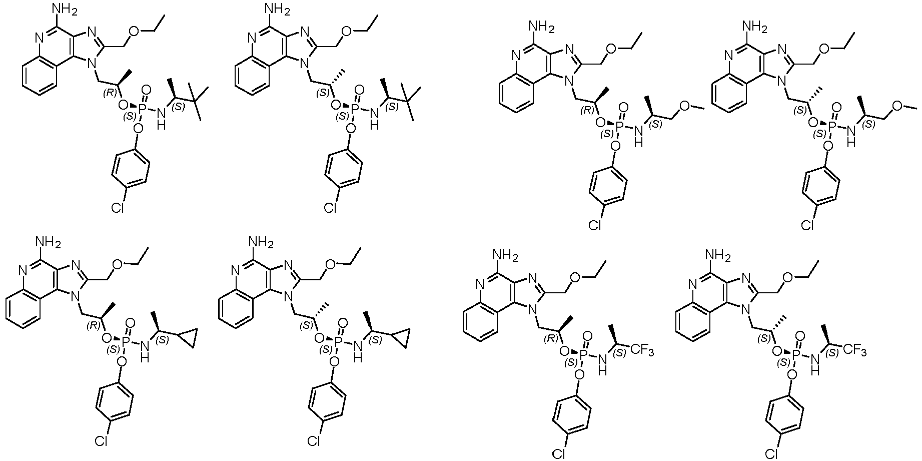

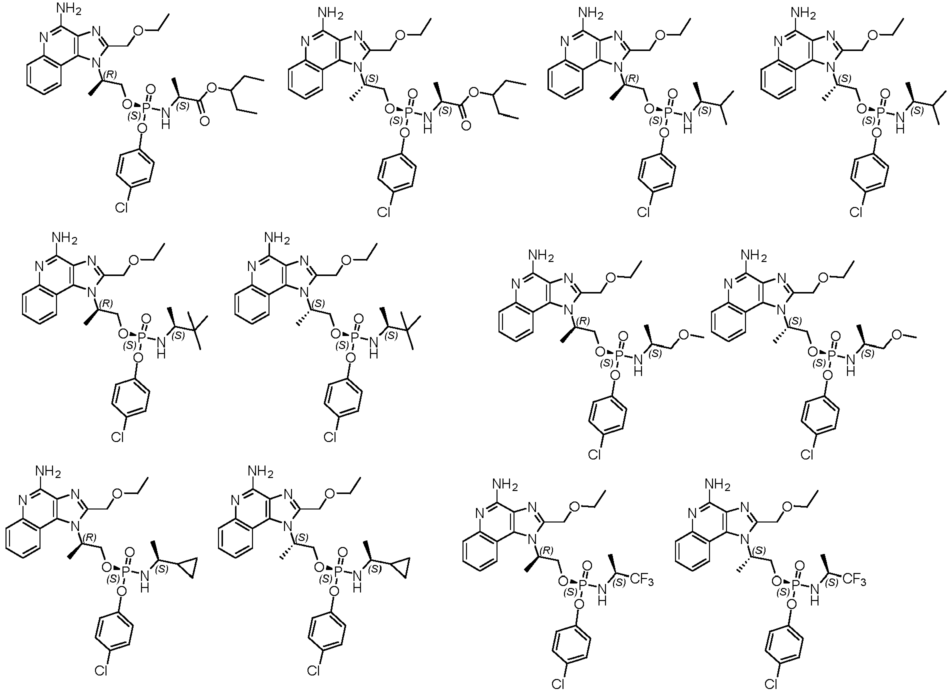

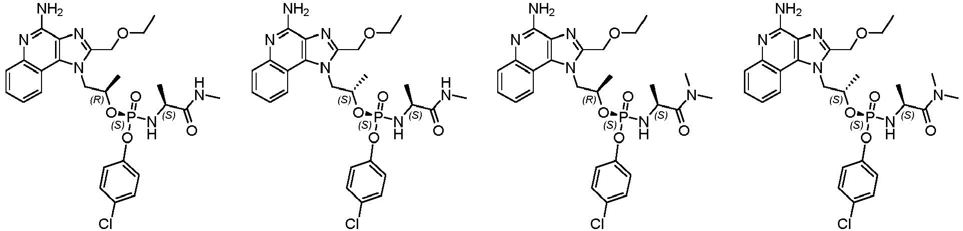

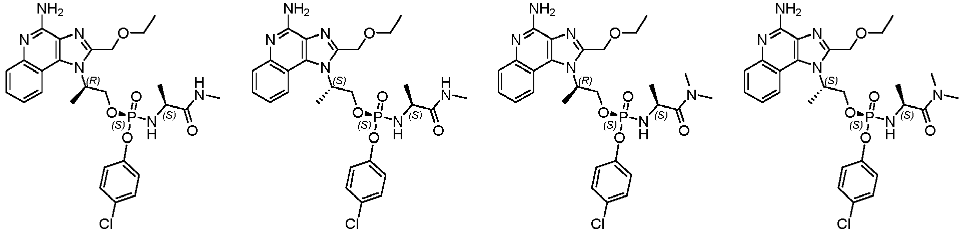

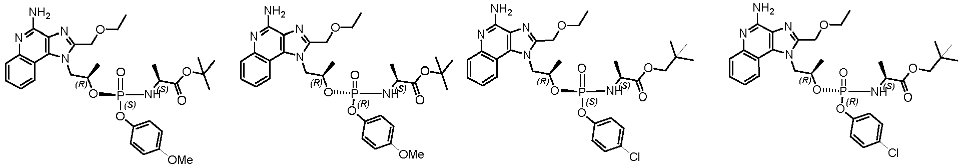

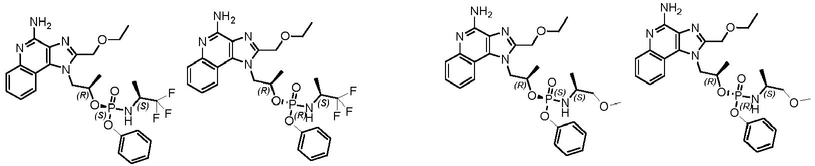

7. A method for preparing the imidazoquinoline-substituted phosphoric ester compound having a structure of general formula (I), or a stereoisomer, a tautomer, a crystal form, or a pharmaceutically acceptable salt, a hydrate, a solvate or a prodrug thereof according to claim 1 comprising the following steps:
1) protecting one of the H in -NH₂ of 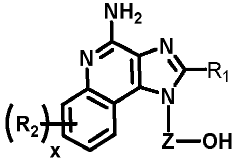 to obtain 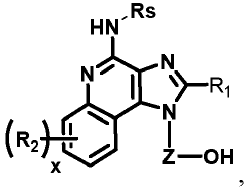 wherein Rs is a protecting group;
2) reacting 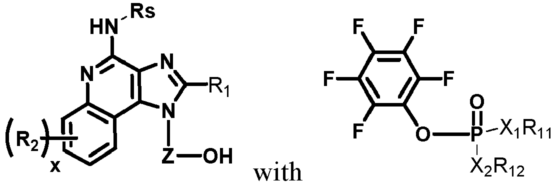 or 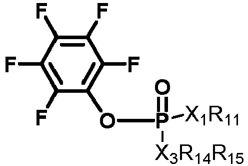 to obtain 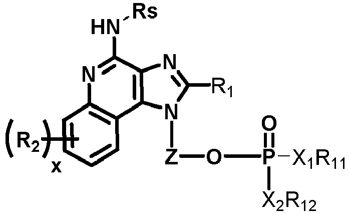 or 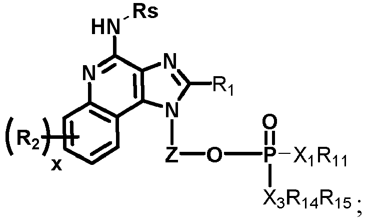
3) deprotecting 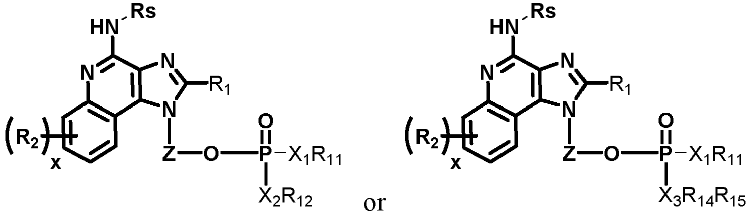 to obtain 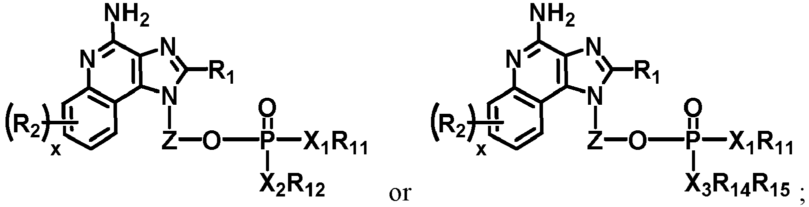 wherein, X is selected from the group consisting of -P(=O)(X₁R₁₁)(X₂R₁₂) and -P(=O)(X₁R₁₁)(X₃R₁₄R₁₅);
y is 1;
R₁, R₂, x, Z, X₁, X₂, X₃, R₁₁, R₁₂, R₁₄ and R₁₅ are as defined in claim 1.

8. A pharmaceutical composition comprising a pharmaceutically acceptable carrier and one or more imidazoquinoline-substituted phosphoric ester compounds having a structure of general formula (I), or a stereoisomer, a tautomer, a crystal form, or a pharmaceutically acceptable salt, a hydrate, a solvate or a prodrug thereof according to claim 1.

9. The pharmaceutical composition according to claim 8, further comprising other drugs for preventing and/or treating diseases selected from the group consisting of inflammation, cancer, cardiovascular disease, infection, immune disease, and metabolic disease.

10. Use of the imidazoquinoline-substituted phosphoric ester compound having a structure of general formula (I), or a stereoisomer, a tautomer, a crystal form, or a pharmaceutically acceptable salt, a hydrate, a solvate or a prodrug thereof according to claim 1 for a use selected from the group consisting of:
1) for the preparation of TLR7 agonists;
2) for the preparation of TLR8 agonists;
3) for the preparation of TLR7 and TLR8 dual agonists;
4) for the preparation of drugs for the prevention and/or treatment of viral infectious diseases;
5) for the preparation of drugs for the prevention and/or treatment of cancer.

11. The use according to claim 10, wherein the viral infectious disease is selected from the group consisting of dengue virus, yellow fever virus, West Nile virus, Japanese encephalitis virus, tick-borne encephalitis virus, Kunjun virus, Murray Valley encephalitis virus, St. Louis encephalitis virus, Omsk hemorrhagic fever virus, bovine viral diarrhea virus, Zika virus, viral hepatitis, and viral skin disease.

12. The use of claim 10, wherein the cancer is selected from the group consisting of lung cancer, breast cancer, prostate cancer, esophageal cancer, colorectal cancer, bone cancer, kidney cancer, gastric cancer, liver cancer, large intestine cancer, melanoma, lymphoma, blood cancer, brain tumor, myeloma, soft tissue sarcoma, pancreatic cancer, and skin cancer.
